# EUROPEAN PATENT APPLICATION

(11) **EP 1 632 494 A1**
(43) Date of publication of application: **08.03.2006**
(21) Application number: 04104062.7
(22) Date of filing: 24.08.2004
(51) Int. Cl.: C07D 487/04, C07D 267/14, C07D 265/36, C07D 215/08, C07D 498/14, C07D 513/14, A61K 31/5517, A61K 31/553

(54) **Vasopressin v1a antagonists**

(71) Applicant: Ferring B.V., 2132 JX Hoofddorp (NL)
(72) Inventor: Batt, Andrzej Roman, SO17 2LJ Southampton (GB); Stockley, Martin Lee, SO16 8FY Southampton (GB); Roe, Michael Bryan, SO14 6RB Southampton (GB); Heeney, Celine Marguerite Simone, SO14 6TQ Southampton (GB); Baxter, Andrew John, SO51 7HU, Hants (GB); Hudson, Peter, 2300 Copenhagen S (DK)
(74) Representative: Poulsen, Niels Jakob

(57) **Abstract**

The present invention concerns compounds according to general formula **1**. Compounds according to the invention are vasopressin V1a receptor antagonists. Pharmaceutical compositions of the compounds are useful as treatment of primary dysmenorrhoea.

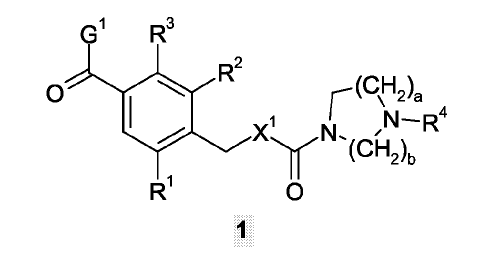

## Description

### Field of the Invention

The present invention relates to non-peptide vasopressin V1a antagonists and to pharmaceutical compositions comprising such compounds. The present invention also relates to the use of non-peptide vasopressin V1a antagonists for the treatment of certain physiological disorders, such as primary dysmenorrhoea and Raynaud's disease.

### Background

The neurophyseal hormones vasopressin (VP) and oxytocin (OT) are cyclic nonapeptides secreted by the posterior pituitary gland.

Only one OT receptor has so far been well characterised, while three VP receptors are known. These are designated the V₁ₐ, V_{1b} and V₂ receptors.

Vasopressin acts on the blood vessels, where it is a potent vasoconstrictor, and on the kidneys, where it promotes water reuptake leading to an antidiuretic effect.

The V₁ₐ, V_{1b}, and V₂, as well as the OT receptors, are members of the super-family of seven transmembrane receptors known as G-protein coupled receptors. The V₁ₐ receptor mediates phospholipase C activation and intracellular calcium mobilisation. Localisation of the receptors includes blood platelets, blood vessels, hepatocytes, brain and uterus-cervix. Thus a V₁ₐ antagonist may have effects on any or all of these tissues. For example, selective V₁ₐ antagonists have been cited as having clinical utility in primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

With respect to primary dysmenorrhoea it has been proposed that myometrial activity is markedly increased in women with primary dysmenorrhoea during menstruation. It is proposed that the myometrial ischemia caused by increased uterine contractility might explain the menstrual pain. Furthermore, on the first day of menstruation, higher plasma concentrations of vasopressin have been measured in dysmenorrhoeal women than in controls.

In healthy women without dysmenorrhoea, intravenous infusion of lysine-vasopressin resulted in decreased uterine blood flow, increased uterine contractility and slight to moderate dysmenorrhoea-like pain, these effects being inhibited by a selective human V₁ₐ receptor antagonist. (Bossmar, T.; Brouard, R.; Doberl, A.; Akerlund, M. Department of Obstetrics and Gynaecology, University Hospital of Lund, Sweden. BRITISH JOURNAL OF OBSTETRICS AND GYNAECOLOGY (1997 Apr), 104(4), 471-7.). Also, it is known that vasopressin contracts human uterine arteries in a dose-dependent and V₁ₐ-mediated fashion.

The above evidence suggests that a V₁ₐ antagonist would be an appropriate and effective treatment for primary dysmenorrhoea. Further evidence is taken from the clinical study carried out on the selective V₁ₐ antagonist SR49059 ("Effect of SR49059, an orally active V₁ₐ vasopressin receptor antagonist, in the prevention of dysmenorrhea". Brouard, R.; Bossmar, T.; Fournie-Lloret, D.; Chassard, D.; Akerlund, M. Sanofi Recherche, Clinical Development, Paris, Fr. BRITISH JOURNAL OF OBSTETRICS AND GYNAECOLOGY (2000), 107(5), 614-619.). It was found that there was a dose-related decrease in pain and a dose-related decrease in the amount of additional pain-killer taken compared to patients taking placebo.

The International Patent Application WO 03/01631 A1, published 27 February 2003, discloses a number of compounds which are claimed to be oxytocin agonists and to find use in the treatment of male erectile dysfunction. No V1a antagonist activity is reported.

There exists a need for treatments for conditions which are associated with the V1a receptors. There further continues to exist a need for alternative V1a antagonists.

### Disclosure of the Invention

According to an aspect, the present invention relates to novel compounds, preferably VP antagonists, and in particular specific antagonists of the V1a receptor, and pharmaceutically acceptable salts thereof.

According to another aspect, the present invention relates to pharmaceutical compositions comprising these novel compounds, which compositions are useful for the treatment of, inter alia, primary dysmenorrhoea.

According to another aspect, the present invention relates to the use of the compounds in the above mentioned series of potent and specific VP receptor antagonists for the manufacture of a pharmaceutical composition for treatment of dysmenorrhoea.

According to further aspects, the present invention relates to the use of the above mentioned compounds and compositions in therapy and to therapeutic methods wherein the above mentioned compounds and compositions are used.

### Detailed Description of the Invention

According to an aspect the invention concerns a compound according to general formula **1,** or a compound which is a tautomer or a pharmaceutically acceptable salt thereof, 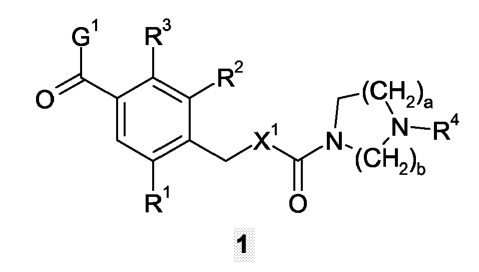
wherein:
G¹ is a bicyclic or tricyclic fused piperidine or azepine derivative selected among general formula **2, 3, 4** and **5** 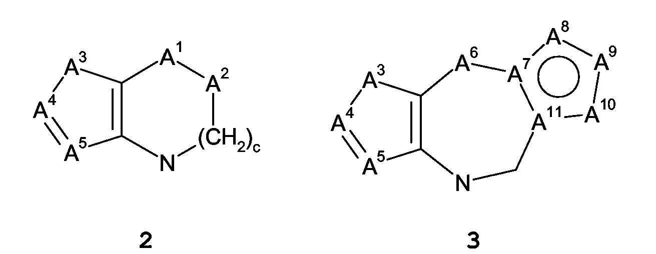 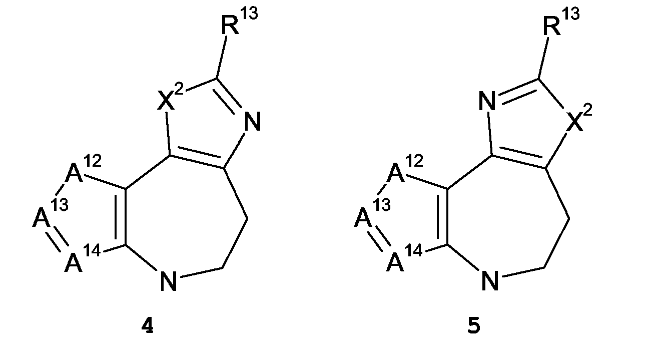
A¹ is selected among CH₂, CH(OH), NH, N-alkyl, O and S;
A² is selected among CH₂, CH (OH) , C(=O) and NH;
A³ is selected among S, NH, N-alkyl, -C(R⁷)=CH-,
-C(R⁷)=N-, -N=C(R⁷)- and -CH=C(R⁷)-;
A⁴ is selected among C(R⁸) and N;
A⁵ is selected among C(R⁹) and N;
A⁶ is selected among CH₂, NH, N-alkyl and O;
A⁷ and A¹¹ are independently selected among C and N;
A⁸ and A⁹ are independently selected among CH, N, NH,
N(CH₂)_{d}R⁵ and S;
A¹⁰ is selected among -CH=CH-, CH, N, NH, N-(CH₂)_{d}-R⁵ and S;
A¹² is selected among S, NH, N-alkyl, -C(R¹⁰)=CH-, -C(R¹⁰)=N-, -N=C(R¹⁰)- and -CH=C(R¹⁰)-;
A¹³ is selected among C(R¹¹) and N;
A¹⁴ is selected among C(R¹²) and N;
X¹ is selected among O and NH;
X² is selected among O, S, NH and N-alkyl;
R¹, R² and R³ are independently selected among H, alkyl, O-alkyl, F, Cl and Br;
R⁴ is selected among H, alkyl, optionally substituted phenyl, optionally substituted pyridyl, optionally substituted thienyl, optionally substituted furyl, -(CH₂)ₑ-R⁶ and Y-R¹⁵;
R¹³ and R¹⁵ are independently selected among H, alkyl, optionally substituted phenyl, optionally substituted pyridyl, optionally substituted thienyl, optionally substituted furyl and ―(CH₂)_{f}―R¹⁴;
R⁵, R⁶ and R¹⁴ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R⁷, R⁸ and R⁹ are independently selected among H, alkyl, alkoxy, F, Cl, Br, CN, NH₂, NO₂, NH(alkyl) and N(alkyl)₂; R¹⁰, R¹¹ and R¹² are independently selected among H, alkyl, alkoxy, F, Cl, Br, CN, NH₂, NO₂, NH(alkyl), and N(alkyl)₂; Y is selected among C=O and S(=O)_{g};
a is selected among 1 and 2;
b is selected among 1, 2 and 3;
c is selected among 1 and 2;
d is selected among 1, 2 and 3;
e is selected among 1, 2 and 3;
f is selected among 1, 2 and 3;
g is selected among 1 and 2; and
subject to the proviso that the ring containing A³, A⁴ and A⁵ according to general formulae **2** and **3** is substituted in at least one position by alkyl, alkoxy, F, Cl, Br, CN, NH₂, NO₂, NH(alkyl) or N(alkyl)₂;
and to the proviso that the ring constituted by A⁷, A⁸, A⁹, A¹⁰ and A¹¹ is aromatic.

Accordingly at least one of R⁷, R⁸ and R⁹ is not H and if A³ is S, NH or N-alkyl, then at least one of R⁸ and R⁹ is not H.
As the ring constituted by A⁷, A⁸, A⁹, A¹⁰ and A¹¹ is aromatic, the groups must satisfy certain requirements. When A¹⁰ is -CH=CH- the ring is a six memebred ring. As such, it can only comprise atoms of the type -C(R)= and -N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not -CH=CH- then one, and only one, of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions:
1) If A¹⁰ is not -CH=CH- then one of A⁸, A⁹ and A¹⁰ is NH, N-(CH)₂-R⁵ or S or one of A⁷ and A¹¹ is N.
2) Not more than one of A⁸, A⁹ and A¹⁰ may be NH, N-(CH)₂-R⁵ or S.
3) A⁷ and A¹¹ may not both simultaneously be N.
4) Neither A⁷ nor A¹¹ may be N if one of A⁸, A⁹ and A¹⁰ is NH, N-(CH)₂-R⁵ or S.

Certain compounds within the scope of the present invention may exist as tautomers. For example, where G¹ is general formula **4** and X² is NH the resulting imidazole can exist as its tautomer which is defined by G¹ as general formula **5** and X² as NH. All such tautomers are considered to be within the scope of the present invention.

As used herein, the term "alkyl" is intended to designate lower alkyl groups, i.e. saturated hydrocarbon groups of between one and six carbon atoms, including linear, branched and cyclic alkyl groups. Examples of "alkyl" include, but are not limited to: C1 - methyl, C2 - ethyl, C3 - propyl, isopropyl, cyclopropyl, C4 - n-butyl, sec-butyl, isobutyl, tert-butyl, cyclobutyl, cyclopropylmethyl, methylcyclopropyl, C5 - n-pentyl, neopentyl, cyclopropylethyl, dimethylcyclopropyl, and C6 - n-hexyl, cyclohexyl, bicyclo[3.1.0]hexyl, tert-butylethyl.

The term "acyl" denotes a group R-C(=O), where R is H, a saturated or unsaturated hydrocarbon moiety of up to seven carbon atoms or an optionally substituted phenyl, optionally substituted pyridyl or optionally substituted thienyl group. Examples of acyl groups include, but are not limited to: formyl, acetyl, pivaloyl, benzoyl and nicotinoyl.

Certain compounds of general formula **1** are capable of forming salts with acids or bases. For example, compounds containing one or more basic nitrogen atoms can form addition salts with mineral and organic acids such as hydrochloric acid, sulphuric acid, phosphoric acid, acetic acid, trifluoroacetic acid, methanesulphonic acid, citric acid and benzoic acid. Compounds containing acidic groups can form salts with bases. Examples of such salts include the sodium, potassium, calcium, triethylammonium and tetraethylammonium salts. Furthermore, compounds that have both acidic and basic groups can form internal salts (zwitterions). Insofar as these salts are pharmaceutically acceptable, they are included within the scope of the invention.

The compounds according to the present invention may have one or more stereogenic centres ("asymmetric carbon atoms") and so may exhibit optical isomerism. The scope of the present invention includes all epimers, enantiomers and diastereomers of compounds according to general formula **1,** including single isomers, mixtures and racemates.

According to aspects of the invention, preferred embodiments of the invention are as set out below. Additional aspects of the invention are indicated in the claims.

The preferred embodiments of G¹ are: 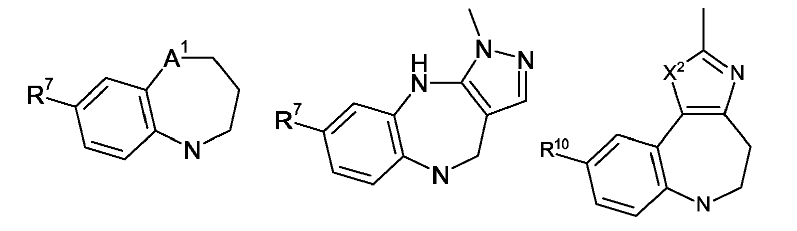
where A¹, R⁷, R¹⁰ and X² are as previously defined.

Of the above, the most preferred embodiments comprise: 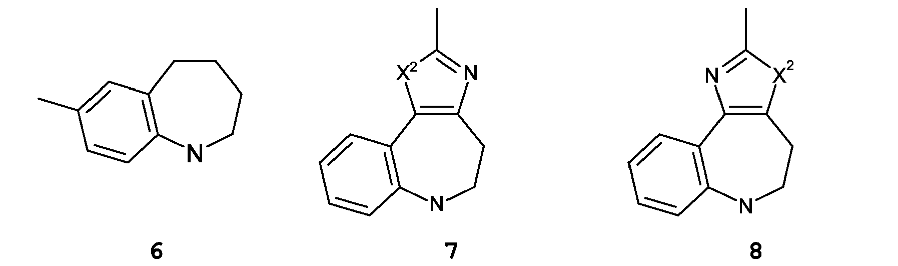 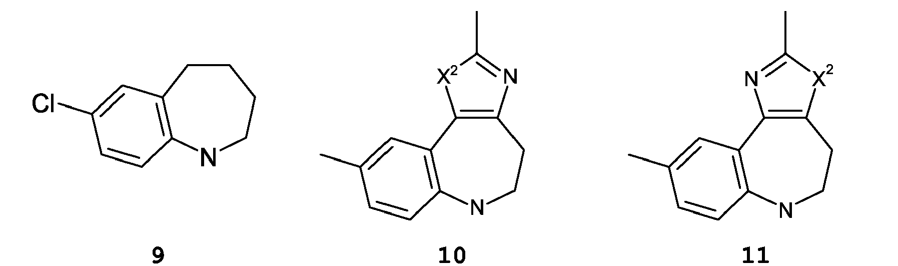 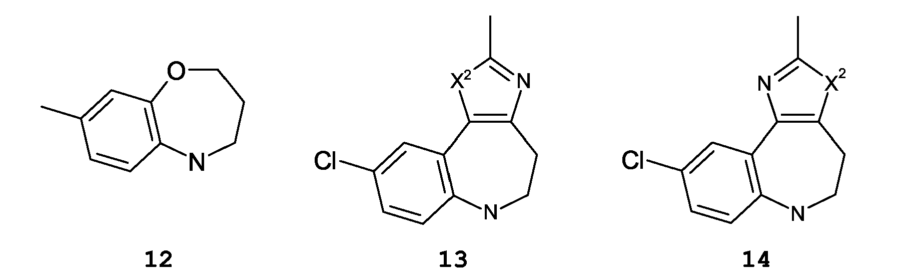 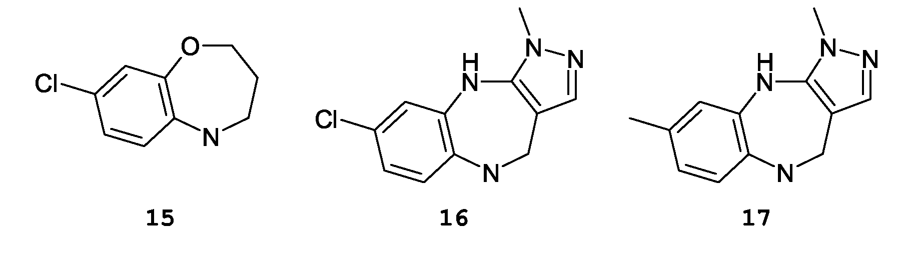
where X² is as previously defined.

It is preferred that at least one of R¹, R² and R³ is other than hydrogen. Most preferred is when one of R¹, R² and R³ is methyl, chlorine or fluorine and the others are hydrogen.

It is preferred that X¹ is NH.

It is preferred that a is 1 and b is 2 such that a piperazine is provided.

It is preferred that R⁴ is alkyl.

Particularly preferred embodiments within the present invention are those compounds that combine two or more of the preferred features described above. One such particularly preferred embodiment is a urea according to general formula **18,** which corresponds to a compound according to general formula **1** in which R² and R³ are both H, X¹ is NH, a is 1 and b is 2.

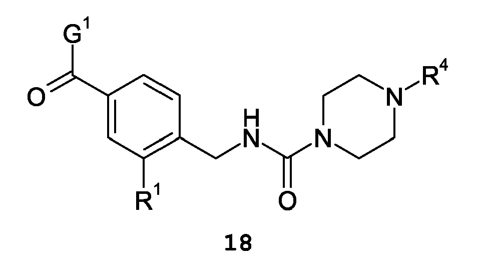

In general formula **18** R¹ is methyl, chlorine or fluorine, R⁴ is alkyl and G¹ is as previously defined.

Another particularly preferred embodiment is a urea according to general formula **19,** which corresponds to a compound according to general formula **1** in which R² and R³ are both H and X¹ is NH.

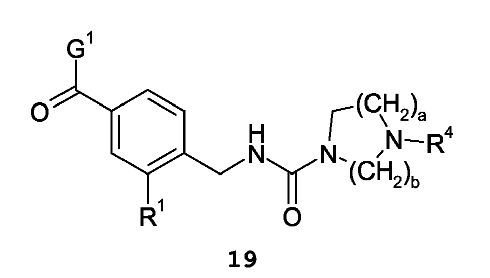

In general formula **19** G¹ is selected from one of general formulae **6** to **17,** R¹ is methyl, chlorine or fluorine and a, b and R⁴ are as previously defined.

Another particularly preferred embodiment is a compound according to general formula **20** which corresponds to a compound according to general formula **1** in which a is 1 and b is 2.

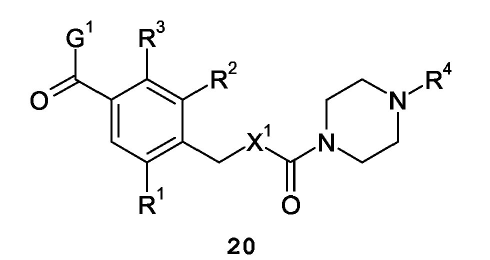

In general formula **20** G¹ is selected from one of general formulae **6** to **17,** R⁴ is alkyl and X¹, R¹, R² and R³ are as previously defined.

A yet more particularly preferred embodiment is a compound according to general formula **21** which corresponds to a compound according to general formula **1** in which R² and R³ are both H, X¹ is NH, a is 1 and b is 2.

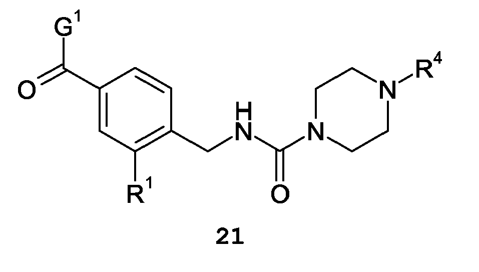

In general formula **21** R¹ is methyl, chlorine or fluorine, R⁴ is alkyl and G¹ is selected from one of general formulae **6** to **17**.

Particularly preferred compounds comprise:
4-(2,2-Dimethyl-propyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(2,2-Dimethyl-propyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 2-chloro-4-(3,6-dimethyl-4,10-dihydro-3H-2,3,4,9-tetraaza-benzo[f]azulene-9-carbonyl)-benzylamide;
4-(3,3-Dimethyl-butyl)-piperazine-1-carboxylic acid 2-chloro-4-(6-chloro-3-methyl-4,10-dihydro-3H-2,3,4,9-tetraaza-benzo[f]azulene-9-carbonyl)-benzylamide;
4-(3,3-Dimethyl-butyl)-piperazine-1-carboxylic acid 2-methyl-4-(2-methyl-4,5-dihydro-1H-1,3,6-triaza-benzo[e]azulene-6-carbonyl)-benzylamide;
4-(3,3-Dimethyl-butyl)-piperazine-1-carboxylic acid 2-methyl-4-(2-methyl-4,5-dihydro-1-oxa-3,6-diaza-benzo[e]azulene-6-carbonyl)-benzylamide;
4-(3,3-Dimethyl-butyl)-piperazine-1-carboxylic acid 2-methyl-4-(3-methyl-7,8-dihydro-6H-5-oxa-9-aza-benzocycloheptene-9-carbonyl)-benzylamide;
4-(3,3-Dimethyl-butyl)-piperazine-1-carboxylic acid 2-methyl-4-(8-methyl-3,4-dihydro-2H-quinoline-1-carbonyl)-benzylamide;
4-(3,3-Dimethyl-butyl)-piperazine-1-carboxylic acid 3-chloro-4-(3,6-dimethyl-4,10-dihydro-3H-2,3,4,9-tetraaza-benzo[f]azulene-9-carbonyl)-benzylamide;
4-(3,3-Dimethyl-butyl)-piperazine-1-carboxylic acid 3-chloro-4-(6-chloro-3-methyl-4,10-dihydro-3H-2,3,4,9-tetraaza-benzo[f]azulene-9-carbonyl)-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-3-fluoro-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-3-methyl-benzylamide;
4-(3,3-Dimethyl-butyl)-piperazine-1-carboxylic acid 4-(3-chloro-7,8-dihydro-6H-5-oxa-9-aza-benzocycloheptene-9-carbonyl)-2-methyl-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-2-fluoro-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-3-fluoro-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-3-methyl-benzylamide;
4-(3-Methyl-butyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(3-Methyl-butyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopentylmethyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Cyclopentylmethyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 2-chloro-4-(3,6-dimethyl-4,10-dihydro-3H-2,3,4,9-tetraaza-benzo[f]azulene-9-carbonyl)-benzylamide;
4-Cyclopropylmethyl-piperazine-1-carboxylic acid 2-chloro-4-(6-chloro-3-methyl-4,10-dihydro-3H-2,3,4,9-tetraaza-benzo[f]azulene-9-carbonyl)-benzylamide;
4-Cyclopropylmethyl-piperazine-1-carboxylic acid 3-chloro-4-(3,6-dimethyl-4,10-dihydro-3H-2,3,4,9-tetraaza-benzo[f]azulene-9-carbonyl)-benzylamide;
4-Cyclopropylmethyl-piperazine-1-carboxylic acid 3-chloro-4-(6-chloro-3-methyl-4,10-dihydro-3H-2,3,4,9-tetraaza-benzo[f]azulene-9-carbonyl)-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-2-fluoro-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-3-fluoro-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-3-methyl-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-2-fluoro-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-3-fluoro-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-3-methyl-benzylamide;
4-Isobutyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Isobutyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Propyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Propyl-piperazine-1-carboxylic acid 4-(6-chloro-3-methyl-4,10-dihydro-3H-2,3,4,9-tetraaza-benzo[f]azulene-9-carbonyl)-2-methyl-benzylamide; and
4-(3,3-Dimethyl-butyl)-piperazine-1-carboxylic acid 2-methyl-4- (2-methyl-4, 5-dihydro-3-thia-1, 6-diaza-benzo[e]azulene-6-carbonyl)-benzylamide.

The particularly preferred compounds are V1a antagonists of high activity.

It appears that the compounds of formula **1b** of claim 14 are subject to following constraints. The ring constituted by A⁷, A⁸, A⁹, A¹⁰ and A¹¹ is aromatic, and accordingly the groups must satisfy certain requirements. When A¹⁰ is -CH=CH- the ring is a six membered ring. As such, it can only comprise atoms of the type -C(R)= and-N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not -CH=CH- then one (and only one) of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{c-}R⁶ or S or one of A⁷ and A¹¹ is N. Not more than one of A⁸, A⁹ and A¹⁰ may be NH, N-(CH₂)_{c}-R⁶ or S. A⁷ and A¹¹ may not both simultaneously be N. Neither A⁷ nor A¹¹ may be N if one of A⁸, A⁹ and A¹⁰ is NH, N-(CH₂)_{c}-R⁶ or S.

It appears that the compounds of formula **1c** of claim 29 are subject to the following constraints. The ring constituted by A⁷, A⁸, A⁹, A¹⁰ and A¹¹ is aromatic, and accordingly the groups must satisfy certain requirements. When A¹⁰ is -CH=CH- the ring is a six membered ring. As such, it can only comprise atoms of the type -C(R)= and-N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not -CH=CH- then one (and only one) of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{c-}R¹¹ or S or one of A⁷ and A¹¹ is N. Not more than one of A⁸, A⁹ and A¹⁰ may be NH, N-(CH₂)_{c}-R¹¹ or S. A⁷ and A¹¹ may not both simultaneously be N. Neither A⁷ nor A¹¹ may be N if one of A⁸, A⁹ and A¹⁰ is NH, N-(CH₂)_{c}-R¹¹ or S.

It appears that the compounds of formula **1d** of claim 53 are subject to the following constraints. Where R4 is H, alkyl, optionally substituted phenyl, optionally substituted pyridyl, optionally substituted thienyl, optionally substituted furyl or -(CH₂)_{c}-R¹² then at least one of R⁸, R⁹ and R¹⁰ is not H and if A³ is S, NH or N-alkyl then at least one of R⁹ and R¹⁰ is not H. The ring constituted by A⁷, A⁸, A⁹, A¹⁰ and A¹¹ is aromatic, and accordingly the groups must satisfy certain requirements. When A¹⁰ is -CH=CH- the ring is a six membered ring. As such, it can only comprise atoms of the type -C(R)= and-N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not -CH=CH- then one (and only one) of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{b-}R¹¹ or S or one of A⁷ and A¹¹ is N. Not more than one of A⁸, A⁹ and A¹⁰ may be NH, N-(CH₂)_{b}-R¹¹ or S. A⁷ and A¹¹ may not both simultaneously be N. Neither A⁷ nor A¹¹ may be N if one of A⁸, A⁹ and A¹⁰ is NH, N-(CH₂)_{b}-R¹¹ or S.

According to an aspect, the invention concerns a pharmaceutical composition comprising a compound according to the invention as an active agent.

The invention is further related to pharmaceutical compositions incorporating a compound according to the invention used as a V1a antagonist, which compositions are particularly useful for medical indications such as the treatment of primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

Any excipients used will depend on the intended nature of the formulation, which will, in turn, depend on the intended route of administration. Administration may be oral, transmucosal (such as sublingual, buccal, intranasal, vaginal and rectal), transdermal or by injection (such as subcutaneous, intramuscular and intravenous). Oral administration is generally preferred. For oral administration, the formulation may be a tablet, a capsule or a sachet. Other formulations include dry powders, solutions, suspensions, suppositories and the like.

According to an aspect, the invention concerns the use of a compound of the invention for the manufacture of a medicament for the treatment of a disease selected among primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

The compounds according to the present invention are useful for treatment of several diseases, disorders or conditions. The term "treatment" used herein relates to both treatment in order to cure or alleviate a disease, disorder or a condition, and to treatment in order to prevent the development of a disease, disorder or a condition. The treatment may either be performed in an acute or in a chronic way. The human or animal to be treated, i.e. the patient, may be any human or non-human mammal in need of treatment according to the invention.

The administration of the compositions of the present invention will generally be under the control of a physician. The physician will determine the amount of composition to be administered and the dosing schedule, taking into account the patient's physical condition and the therapeutic goals.

Further aspects of the invention relates to methods of treatment of the above mentioned diseases, disorders or conditions. According to a method according to the invention a therapeutically effective amount of the compound, or of the pharmaceutical composition described above, is administered to a patient in need of this treatment. According to different aspects of the invention, it concerns a method of treatment of a disorder selected among primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

The term "therapeutically effective amount" relates to an amount that will lead to the desired therapeutical effect. The therapeutically effective amount will be determined by the attending physician taking into consideration all appropriate factors. Generally a single dose will comprise between 0.1 mg and 1000 mg, preferably between 1 mg and 250 mg, of the active compound according to the invention. The dose may be given on a single occasion or repeatedly. When given repeatedly, it may be given at regular intervals, such as once, twice or three times daily, or on demand, according to the condition being treated.

The pharmaceutical composition according to the present invention may be presented in any form that is known in the art. For example, the formulation may be presented as a tablet, capsule, powder, suppository, cream, solution or suspension, or in a more complex form such as an adhesive patch. The formulation will generally include one or more excipients, such as diluents, bulking agents, binding agents, dispersants, solvents, preservatives, flavouring agents and the like. The formulation may also include one or more additional phar-macologically active species. Preferably the formulation includes no such additional active agents.

When used as therapeutic agents, the compositions of the present invention may be administered by any appropriate route that is known in the art. For example, they may be administered by the oral, buccal, sublingual, rectal, intra-vaginal, nasal, pulmonary or transdermal routes. Alternatively, they may be given by injection, including intravenous, subcutaneous and intramuscular injection.

The compounds of the present invention can be prepared using standard chemical manipulations. These are described in detail in WO 03/01631 A1, pages 12-17. (Hudson, P. J.; Pitt, G. R. W.; Rooker, D. P.; Batt, A. R. Laurent, C. M. S.; Roe, M. B. "Diazacycloalkanes as Oxytocin Agonists").

The following examples are to be considered as enabling and not limiting for the invention.

### Examples

The following abbreviations are used:
- AIBN: 2,2'-azobisisobutyronitrile
- boc: carboxylic acid tert-butyl ester or tert-butoxycarbonylamino
- Bu: butyl - alkyl residues may be further denoted as n (normal, i.e. unbranched), i (iso) and t (tertiary)
- DIEA: *N,N*-diisopropylethylamine
- DMAP: 4-(dimethylamino)pyridine
- DMF: dimethylformamide
- Et: ethyl
- EtOAc: ethyl acetate
- HOBt: 1-hydroxybenzotriazole
- HPLC: High performance liquid chromatography
- h: hour(s)
- Me: methyl
- min: minute(s)
- MS: mass spectrum
- NMR: nuclear magnetic resonance spectrum - NMR spectra were recorded in CDCl₃ at a frequency of 270MHz unless otherwise indicated
- OVA: ornithine vasotocin analogue
- pet.: petroleum ether boiling in the range 60-80°C
- ether Ph: phenyl
- Pn: pentyl
- Pr: propyl
- THF: tetrahydrofuran
- Tos: toluene-4-sulphonyl
- WSCD: water-soluble carbodiimide (*N*-ethyl-*N*'-(3-dimethylaminopropyl)carbodiimide hydrochloride

Examples 1 - 14 describe the synthesis of intermediates. Compounds according to the present invention are described in Examples 15 - 61. Further enabling disclosure is provided in Examples 62 - 72. Example A describes how compounds can be assayed based on their ability to inhibit the cellular consequences of AVP stimulation on intact cells. Example B describes tablets for oral administration comprising a compound according to the invention.

### Example 1

### 6-Chloro-3-methyl-3,4,9,10-tetrahydro-2,3,4,9-tetraaza-benzo[f]azulene

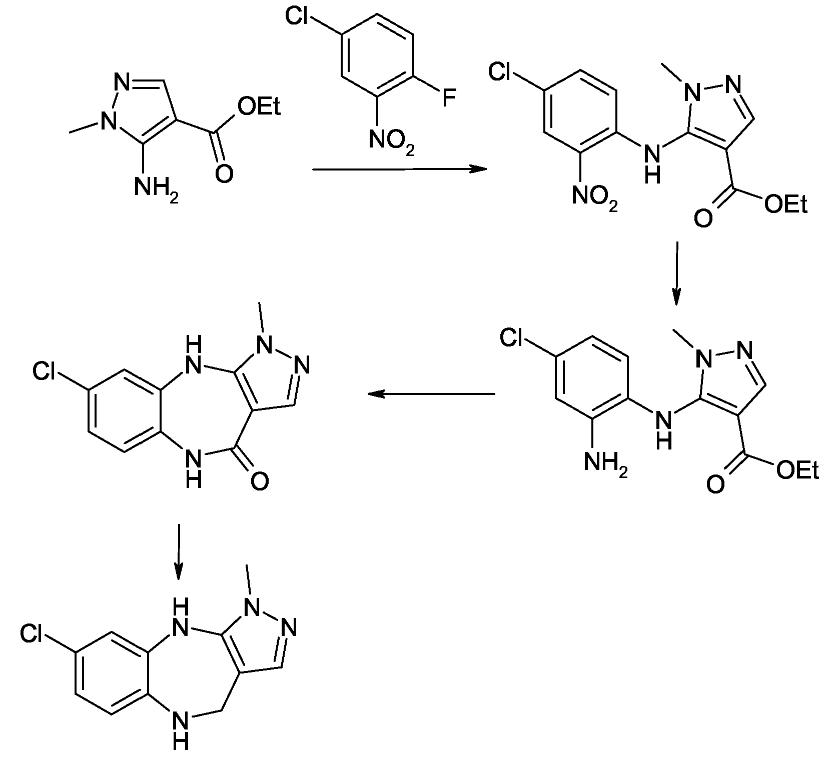

### Example 1.1

### 5-(4-Chloro-2-nitro-phenylamino)-1-methyl-1H-pyrazole-4-carboxylic acid ethyl ester.

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h. A solution of 4-chloro-2-fluoronitrobenzene (22.6g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x 2), and the combined organic extracts were washed with brine and concentrated *in vacuo.* The residue was purified by flash chromatography on silica gel (eluant; 60% pet ether:40% EtOAc) to yield the title compound (27.5g, 62%).

### Example 1.2

### 5-(2-Amino-4-chloro-phenylamino)-1-methyl-1H-pyrazole-4-carboxylic acid ethyl ester.

Zinc powder (26.17g, 400mmol) was added to a suspension of 5-(4-chloro-2-nitro-phenylamino)-1-methyl-1H-pyrazole-4-carboxylic acid ethyl ester from Example 1.1 (26.0g, 80mmol) in methanol/acetic acid (10:1, 330ml) at room temperature. After the exothermic reaction which followed, the resulting suspension was stirred at room temperature for 18h before being filtered through Celite® filter agent. The filtrate was concentrated *in vacuo.* The residue was dissolved in EtOAc, and the solution was washed with saturated NaHCO₃ and brine, and concentrated *in vacuo.* The residue was purified by flash chromatography on silica gel (eluant; 65% EtOAc:35% pet ether to 80% EtOAc:20% pet ether) to yield the title compound (18.41g, 78.0%).

### Example 1.3

### 6-Chloro-3-methyl-4,9-dihydro-3H-2,3,4,9-tetraaza-benzo[f]azulen-10-one

Sodium (methylsulphinyl)methanide (53.4mmol) was prepared from sodium hydride (60% dispersion in oil, 2.14g, 53.4mmol) and anhydrous dimethyl sulphoxide (35ml) by heating at 65°C until a solution was observed. To this was added a solution of 5-(2-amino-4-chloro-phenylamino)-1-methyl-1H-pyrazole-4-carboxylic acid ethyl ester from Example 1.2 (9.02g, 30.5mmol) in anhydrous dimethyl sulphoxide (20ml), and stirring continued at 65°C for 30min. The mixture was poured into ice (200ml), and the resulting solid collected and purified by recrystallisation from methanol/EtOAc to yield the title compound (5.56g, 73.3%).

### Example 1.4

### 6-Chloro-3-methyl-3,4,9,10-tetrahydro-2,3,4,9-tetraaza-benzo[f]azulene

To a suspension of 6-chloro-3-methyl-4,9-dihydro-3H-2,3,4,9-tetraaza-benzo[f]azulen-10-one from Example 1.3 (5.56g, 22.4 mmol) in anhydrous THF (200ml) at 0°C was added lithium aluminium hydride (4.24g, 112mmol), and the resulting suspension was heated at reflux for 18h, then allowed to cool to room temperature. A further portion of lithium aluminium hydride (4.24g, 112mmol) was added, and the mixture was heated at reflux for 18h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated *in vacuo.* The residue was purified by flash chromatography on silica gel (eluant; 0.5% 35% ammonia:5% methanol:dichloromethane) to yield the title compound (3.88g, 74%).

### Example 2

### 3,6-Dimethyl-3,4,9,10-tetrahydro-2,3,4,9-tetraaza-benzo[f]azulene

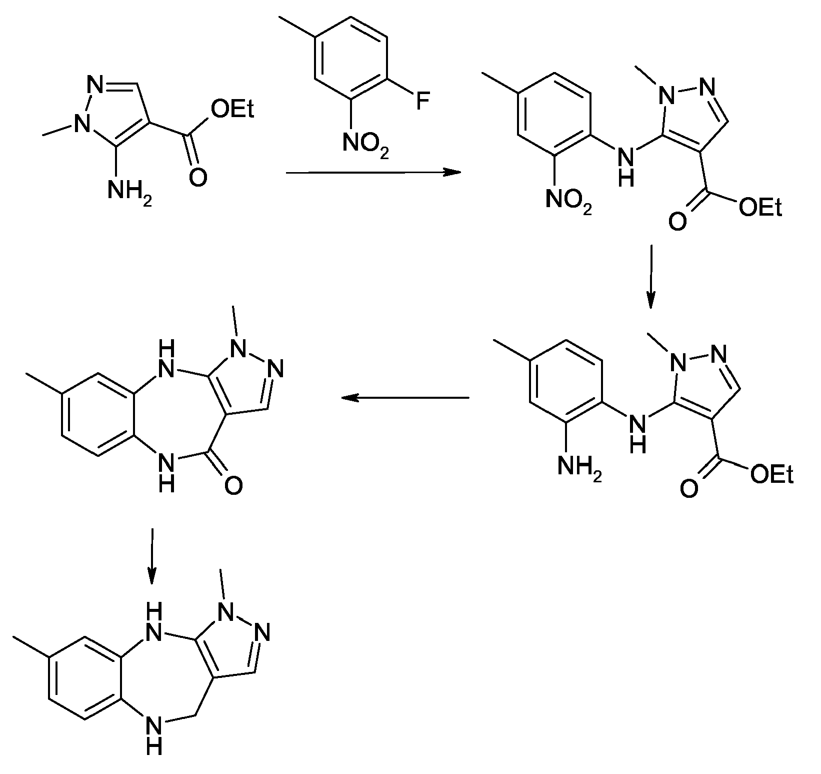

### Example 2.1

### 1-Methyl-5-(4-methyl-2-nitro-phenylamino)-1H-pyrazole-4-carboxylic acid ethyl ester.

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h then a solution of 3-fluoro-4-nitrotoluene (20g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x2), and the combined organic extracts were washed with brine and concentrated *in vacuo.* The residue was purified by flash chromatography on silica gel (eluant; 60% pet ether:40% EtOAc) to yield the title compound (28.00g, 61%).

### Example 2.2

### 5-(2-Amino-4-methyl-phenylamino)-1-methyl-1H-pyrazole-4-carboxylic acid ethyl ester

Tin (II) chloride (86.65g, 457.0mmol) was added to a solution of 1-methyl-5-(4-methyl-2-nitro-phenylamino)-1H-pyrazole-4-carboxylic acid ethyl ester from Example 2.1 (28.00g, 91.4mmol) in methanol and the mixture heated at reflux for 3 days. The solvent was removed *in vacuo* and the residue taken up in EtOAc (400ml) and cooled to 0°C. 35% Ammonia solution was added to pH 14, and the mixture was stirred for 15min before being filtered through Celite® filter agent. The filtrate was washed with 2M NH₃ and brine and concentrated *in vacuo.* The residue was purified by flash chromatography on silica gel (eluant; 6% methanol:94% dichloromethane rising to 10% methanol:90% dichloromethane) to yield the title compound (12.8g, 52%).

### Example 2.3

### 3,6-Dimethyl-4,9-dihydro-3H-2,3,4,9-tetraaza-benzo[f]azulen-10-one

Sodium (methylsulphinyl)methanide (22.7mmol) was prepared from sodium hydride (60% dispersion in oil, 912mg, 22.7mmol) and anhydrous dimethyl sulphoxide (5.5ml) by heating at 65°C until a solution was observed. To this was added a solution of 5-(2-amino-4-methyl-phenylamino)-1-methyl-1H-pyrazole-4-carboxylic acid ethyl ester from Example 2.2 (3.56g, 13.0mmol) in anhydrous dimethyl sulphoxide (10ml), and stirring continued at 65°C for 30 min. The mixture was then poured into ice (200ml), the resulting solid collected and purified by flash chromatography on silica gel (eluant; 10%methanol:90% dichloromethane) to yield the title compound (1.12g, 38%).

### Example 2.4

### 3,6-Dimethyl-3,4,9,10-tetrahydro-2,3,4,9-tetraaza-benzo[f]azulene

To a suspension of 3,6-dimethyl-4,9-dihydro-3H-2,3,4,9-tetraaza-benzo[f]azulen-10-one from Example 2.3 (2.35g, 10.3mmol) in anhydrous THF (100ml) at 0°C was added lithium aluminium hydride (1.56g, 41.2mmol), and the resulting suspension was heated at reflux for 18h then allowed to cool to room temperature. A further portion of lithium aluminium hydride (781 mg, 20.6mmol) was added, and the mixture was heated at reflux for 3h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated *in vacuo.* The residue was purified by flash chromatography on silica gel (eluant; 10% methanol:90% dichloromethane) to yield the title compound (1.60g, 72%).

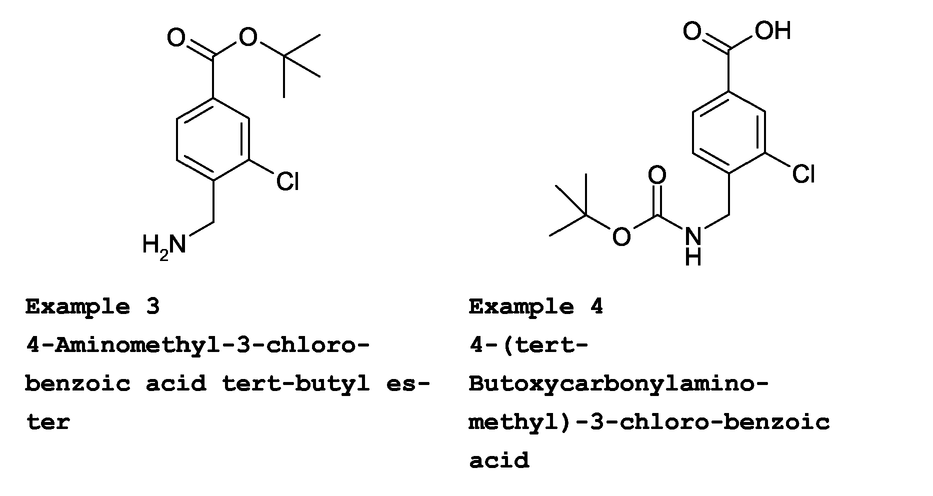

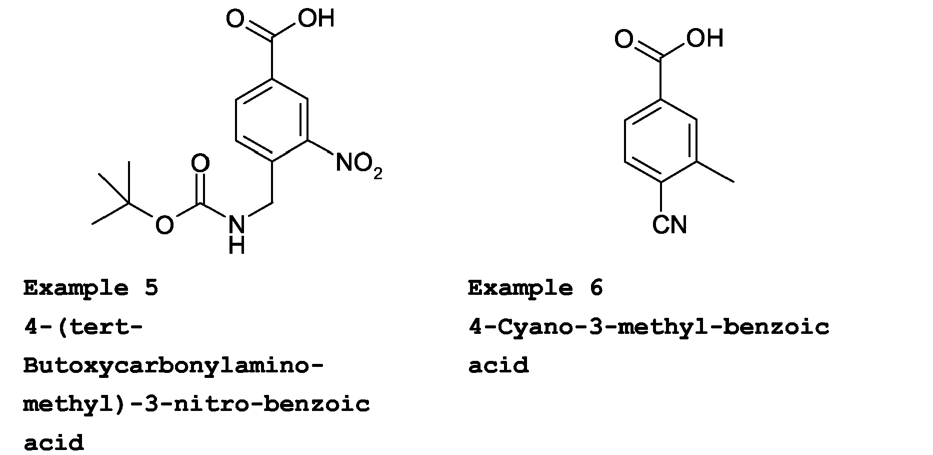

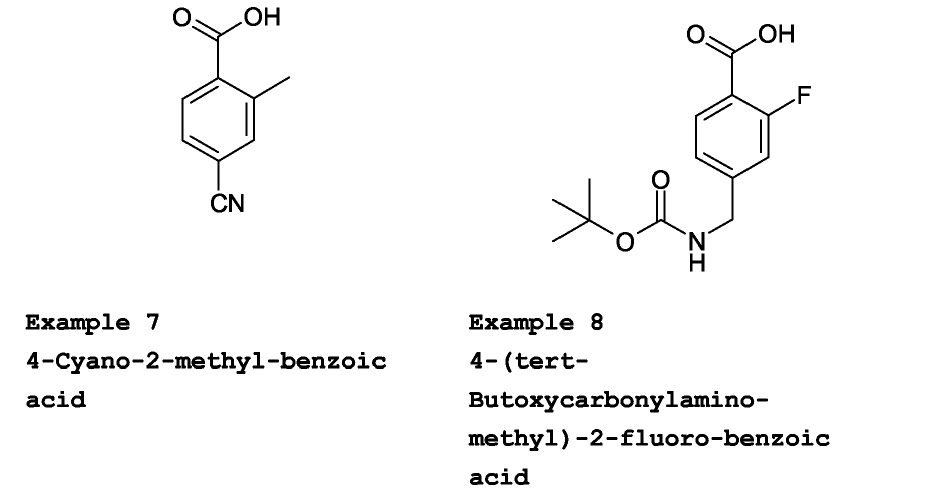

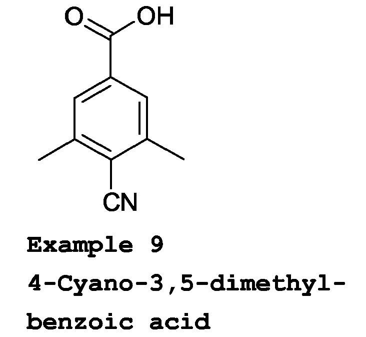

Examples 3 - 9 are described in WO 03/01631 A1, (Hudson, P. J.; Pitt, G. R. W.; Rooker, D. P.; Batt, A. R.

Laurent, C. M. S.; Roe, M. B. "Diazacycloalkanes as Oxytocin Agonists"), pages 26-31.

### Example 10

### 4-(tert-Butoxycarbonylamino-methyl)-3-fluoro-benzoic acid

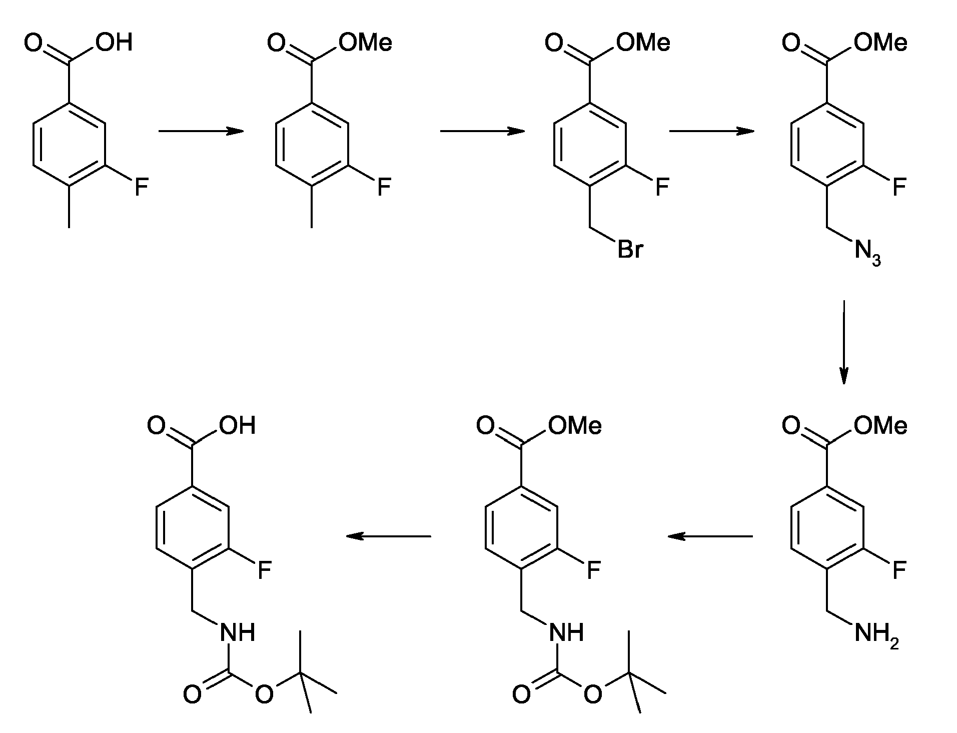

### Example 10.1

### 3-Fluoro-4-methylbenzoic acid methyl ester

Thionyl chloride (9.62g, 81mmol) was added to a solution of 3-fluoro-4-methylbenzoic acid (5.0 g, 32.4mmol) in toluene (50ml). The mixture was stirred at room temperature for 1.5h and heated at reflux for 3h. The solvent was removed *in vacuo* and the residue was taken up in methanol (30ml) and CH₂Cl₂ (30ml) and stirred for 18h. The mixture was evaporated *in vacuo* and the residue was taken up in EtOAc (50ml), washed with saturated NaHCO₃ solution (3x75ml), dried and evaporated *in vacuo* to yield the title compound (4.5g, 83%).

### Example 10.2

### 4-Bromomethyl-3-fluorobenzoic acid methyl ester

3-Fluoro-4-methylbenzoic acid methyl ester from Example 10.1 (4.5g, 26.6mmol) was dissolved in carbon tetrachloride (150ml). AIBN (457mg, 2.7mmol) and *N-*bromosuccinimide (5.2g, 29.3mmol) were added and the mixture was heated at reflux for 18h. The mixture was allowed to cool and further portions of AIBN (457mg, 2.7mmol) and *N*-bromosuccinimide (5.2g, 29.3mmol) were added. The mixture was heated at reflux for 56h. The mixture was allowed to cool and evaporated *in vacuo.* The residue was purified by flash chromatography on silica gel (eluant; 10% EtOAc:90% pet ether) to yield the title compound (2.7g, 41%).

### Example 10.3

### 4-Azidomethyl-3-fluorobenzoic acid methyl ester

Sodium azide (609mg) was added to a solution of 4-bromomethyl-3-fluorobenzoic acid methyl ester from Example 10.2 (2.1g, 8.5mmol) in DMF (30ml). The mixture was stirred for 18h, diluted with EtOAc, washed with water and brine and concentrated *in vacuo* to give a colourless oil identified as the title compound (1.8 g, 100%).

### Example 10.4

### 4-Aminomethyl-3-fluorobenzoic acid methyl ester

Hydrogen was passed through a degassed solution of 4-azidomethyl-3-fluorobenzoic acid methyl ester from Example 10.3 (2.11g, 10mmol) in methanol containing 10% palladium on carbon for 2h. The reaction mixture was filtered through Celite® filter agent and the filtrate was concentrated *in vacuo* to give a colourless oil identified as the title compound (1.51 g, 83%).

### Example 10.5

### 4-(tert-Butoxycarbonylamino-methyl)-3-fluorobenzoic acid methyl ester

To a solution of 4-aminomethyl-3-fluorobenzoic acid methyl ester from Example 10.4 (1.5g, 8.2mmol) in dichloromethane (20ml) were added di-tert-butyl dicarbonate (2.3g, 11mmol) and triethylamine (1.4ml, 10mmol). The mixture was stirred for 18h, washed with 0.3M KHSO₄ and brine and concentrated *in vacuo.* The residue was purified by flash chromatography on silica gel (eluant; 10% EtOAc:90% pet. ether) to give a white solid identified as the title compound (1.4 g, 60%) .

### Example 10.6

### 4-(tert-Butoxycarbonylamino-methyl)-3-fluorobenzoic acid

To a solution of 4-(tert-butoxycarbonylamino-methyl)-3-fluorobenzoic acid methyl ester from Example 10.5 (640mg, 2.25mmol) in dioxan (40ml) was added 1N NaOH (4.5ml, 4.5mmol). The mixture was stirred for 18h, diluted with EtOAc, washed with 1N KHSO₄, water and brine and concentrated *in vacuo* to give a white solid identified as the title compound (608 mg, 100%).

### Example 11

### 1-(3,3-Dimethyl-butyl)-piperazine hydrochloride

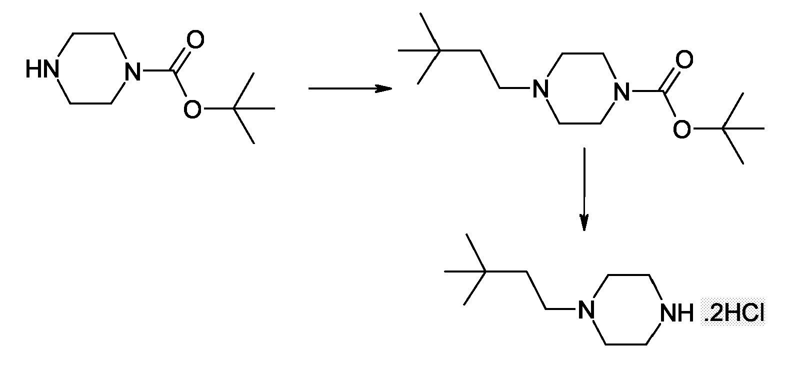

### Example 11.1

### 4-(3,3-Dimethyl-butyl)-piperazine-1-carboxylic acid tert-butyl ester

To a solution of 1-boc-piperazine (8.19g, 43.9mmol) in methanol/acetic acid (99:1, v/v, 100ml) was added 3,3-dimethylbutyraldehyde (4.0g, 40.0mmol) and the resulting mixture was stirred at room temperature for 1h. Sodium cyanoborohydride (3.27g, 51.9mmol) was added, and the resulting mixture was stirred at room temperature for 18h then concentrated in vacuo. The residue was dissolved in EtOAc and the resulting solution was washed with saturated NaHCO₃, water and brine, dried and concentrated *in vacuo.* The residue was purified by flash chromatography on silica gel (eluant; 5% methanol: 95% EtOAc) to yield the title compound (9.1g, 84%).

### Example 11.2

### 1-(3,3-Dimethyl-butyl)-piperazine

A solution of 4-(3,3-dimethyl-butyl)-piperazine-1-carboxylic acid tert-butyl ester from Example 11.1 (9.1g, 31.8mmol) in methanol (100ml) was treated with 4N HCl/dioxan (100ml) and the mixture was stirred at room temperature for 30min then concentrated *in vacuo.* The residue was triturated with diethyl ether and the resulting solid recrystallised from methanol/diethyl ether to yield 1-(3,3-dimethyl-butyl)-piperazine hydrochloride (7.4g, 90%).

### Example 12

### 2-Methyl-1,4,5,6-tetrahydro-1,3,6-triaza-benzo[e]azulene

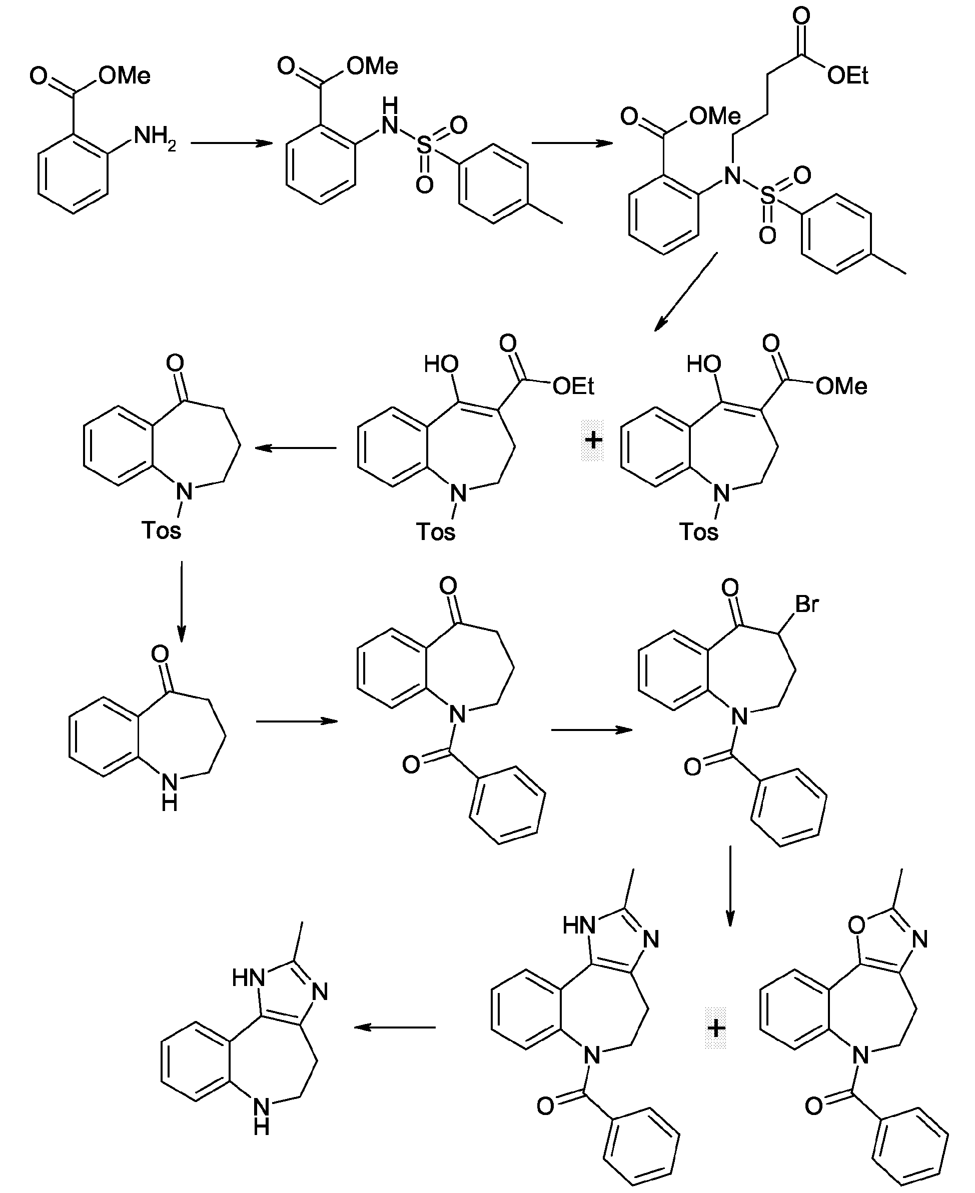

### Example 12.1

### 2-(Toluene-4-sulfonylamino)-benzoic acid methyl ester

Methyl anthranilate (110g, 0.73mol) was dissolved in dichloromethane (1 litre) at 0°C and triethylamine (115ml, 0.8mol) was added. Tosyl chloride (133g, 0.745mol) was added portionwise at 0°C. The reaction mixture was stirred for 30min at 0°C and 64h at room temperature. The mixture was reduced *in vacuo.* The residue was dissolved in EtOAc and the organic layer was washed with 5% KHCO₃(aq) solution, 1N HCl solution and brine, dried, filtered and concentrated *in vacuo.* The residue was crystallised from EtOAc/hexane to yield the title compound (181g, 81%).

### Example 12.2

### 2-[(3-Ethoxycarbonyl-propyl)-(toluene-4-sulfonyl)-amino]-benzoic acid methyl ester

2-(Toluene-4-sulfonylamino)-benzoic acid methyl ester from Example 12.1 (100g) was dissolved in DMF (250ml). Potassium carbonate (125g) and ethyl 4-bromobutanoate (60g) were added and the mixture was heated at 80°C for 18h. The mixture was cooled to room temperature, filtered and reduced *in vacuo.* The residue was partitioned between chloroform and 1M HCl solution. The aqueous layer was extracted with chloroform. The organic layers were combined, washed with brine, dried, filtered and concentrated *in vacuo.* The material was crystallised from EtOAc/hexane and dried in a vacuum oven at 60°C for 3 hours to yield the title compound (98.6g, 72%).

### Example 12.3

### 5-Hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester

2-[3-Ethoxycarbonyl-propyl)-(toluene-4-sulfonyl)-amino]-benzoic acid methyl ester from Example 12.2 (98.6g, 0.235mol) was taken up in warm toluene (600ml) and was added dropwise to a mixture of potassium tert-butoxide (40g) in toluene (1litre) refluxing under Dean-Stark conditions. The mixture was heated at reflux under Dean-Stark condidtions for 1h further and cooled to room temperature. It was diluted with EtOAc (500ml) and washed with 1M HCl solution, saturated NaHCO₃(aq) and brine. The organic layer was dried, filtered and reduced *in vacuo.* The residue was precipitated from EtOAc/hexane and dried in a vacuum oven to yield a mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester (53.5g).

### Example 12.4

### 1-(Toluene-4-sulfonyl)-1,2,3,4-tetrahydro-benzo[b]azepin-5-one

A mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester from Example 12.3 were heated at reflux in a mixture of ethanol (100ml), acetic acid (300ml), concentrated HCl (100ml) and water (50ml) for 18h. The mixture was cooled to room temperature, diluted with water (800ml) and extracted with chloroform. The combined organic extracts were dried, filtered and reduced *in vacuo.* The residue was crystallised twice from methanol to yield the title compound (44g, 60% over two steps).

### Example 12.5

### 1,2,3,4-Tetrahydro-benzo[b]azepin-5-one

Polyphosphoric acid (25g) was heated at 100°C under nitrogen until it could be stirred. 1-(Toluene-4-sulfonyl)-1,2,3,4-tetrahydro-benzo[b]azepin-5-one from Example 12.4 (2.6g, 8.26mmol) was added portionwise and the reaction mixture was heated at 100°C for 1.5h. It was poured into ice and basified with 2M NaOH(aq). The aqueous layer was extracted twice with dichloromethane. The organic extracts were combined, washed with brine, dried and reduced *in vacuo.* The residue was purified by flash chromatography on silica gel (eluant; 40% EtOAc: 60% hexane) to yield the title compound (1.05g, 79%).

### Example 12.6

### 1-Benzoyl-1,2,3,4-tetrahydro-benzo[b]azepin-5-one

1,2,3,4-Tetrahydro-benzo[b]azepin-5-one from Example 12.5 (480mg, 2.98mmol) was dissolved in a mixture of dichloromethane (30ml) and triethylamine (1.3ml). Benzoyl chloride (0.46g, 3.28mmol) was added and the reaction mixture was heated for at reflux for 2h. The mixture was cooled and reduced *in vacuo.* The residue was dissolved in EtOAc and washed with 1M KHSO₄(aq), water and brine. The organic layer was dried, filtered and reduced *in vacuo.* The residue was purified by flash chromatography on silica gel (eluant; 50% EtOAc: 50% hexane) to yield the title compound (440mg, 56%).

### Example 12.7

### 1-Benzoyl-4-bromo-1,2,3,4-tetrahydro-benzo[b]azepin-5-one

1-Benzoyl-1,2,3,4-tetrahydro-benzo[b]azepin-5-one from Example 12.6 (54.2g, 205mmol), *N*-bromosuccinimide (3.6g, 20.4mmol) and bromine (11.1ml, 214.7mmol) were dissolved in dichloromethane (1.0litre). Triethylamine (30ml, 215mmol) was added dropwise over 30 minutes then the reaction mixture was heated at reflux for 4h. Additional bromine (1.1ml, 21.5mmol) and triethylamine (3.0ml, 21.5mmol) were added and the reaction mixture was heated at reflux for a further 4h. Additional bromine (1.1ml, 21.5mmol) and triethylamine (3.0ml, 21.5mmol) were added again and the reaction mixture was heated at reflux for a further 4h. On cooling to room temperature, the reaction solution was washed with 5% aqueous sodium metabisulfate solution (150ml) and the aqueous phase was diluted with water (600ml). The organic phase was separated, washed with saturated NaHCO₃(aq), dried over sodium sulphate and filtered. The filtrate was diluted with EtOAc (100ml), filtered through a silica pad (eluant; CH₂Cl₂) and reduced *in vacuo* to yield the title compound (73.6g) which was used without further purification.

### Example 12.8

### 6-Benzoyl-2-methyl-1,4,5,6-tetrahydro-1,3,6-triaza-benzo[e]azulene

1-Benzoyl-4-bromo-1,2,3,4-tetrahydro-benzo[b]azepin-5-one (67.5g, 200mmol) from Example 12.7, acetamidine hydrochloride (92.7g, 980mmol) and potassium carbonate (136.0g, 980mmol) were suspended in acetonitrile (2.0litres) and heated at reflux for 17h under nitrogen. Additional acetamidine hydrochloride (18.5g, 200mmol) and potassium carbonate (27.7g, 200mmol) were added and the reaction mixture was heated at reflux for a further 6h. Additional acetamidine hydrochloride (18.5g, 200mmol) and potassium carbonate (27.7g, 200mmol) were added again and the reaction mixture was heated at reflux for a further 6h. On cooling to room temperature, the reaction mixture was filtered and the filtrate was concentrated *in vacuo.* The residue was taken up in dichloromethane (1.41itres), washed with water (500ml), dried over sodium sulfate, filtered and reduced *in vacuo.* The crude products were purified by flash chromatography on silica gel (eluant; 45% EtOAc: 45% acetonitrile: 10% methanol) to yield the title compound (26.7g, 34%) and 6-benzoyl-2-methyl-5,6-dihydro-4H-1-oxa-3,6-diaza-benzo[e]azulene (3.3g, 4%).

### Example 12.9

### 2-Methyl-1,4,5,6-tetrahydro-1,3,6-triaza-benzo[e]azulene

6-Benzoyl-2-methyl-1,4,5,6-tetrahydro-1,3,6-triaza-benzo[e]azulene from Example 12.8 (160mg, 0.53mmol) was dissolved in a 6M HCl/dioxane solution (50ml) and heated at reflux for 18h. The reaction mixture was cooled to room temperature and reduced *in vacuo.* The residue was partitioned between EtOAc and saturated NaHCO₃ (aq) and the layers were separated. The organic layer was washed with brine, dried, filtered and reduced *in vacuo* to yield the title compound (69mg, 66%).

### Example 13

### 2-Methyl-5,6-dihydro-4H-1-oxa-3,6-diaza-benzo[e]azulene

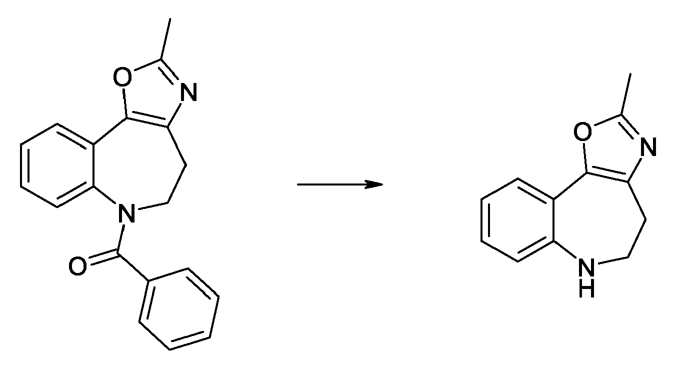

6-Benzoyl-2-methyl-5,6-dihydro-4H-1-oxa-3,6-diaza-benzo[e]azulene from Example 12.8 (1.0g, 3.25mmol) was dissolved in a 6M HCl/dioxane solution (100ml) and heated at reflux for 18h. The reaction mixture was cooled to room temperature and reduced *in vacuo.* The residue was partitioned between EtOAc and saturated NaHCO₃(aq) and the layers were separated. The organic layer was washed with brine, dried, filtered and reduced *in vacuo* to yield the title compound (540mg, 82%).

### Example 14

### 2-Methyl-5,6-dihydro-4H-3-thia-1,6-diaza-benzo[e]azulene

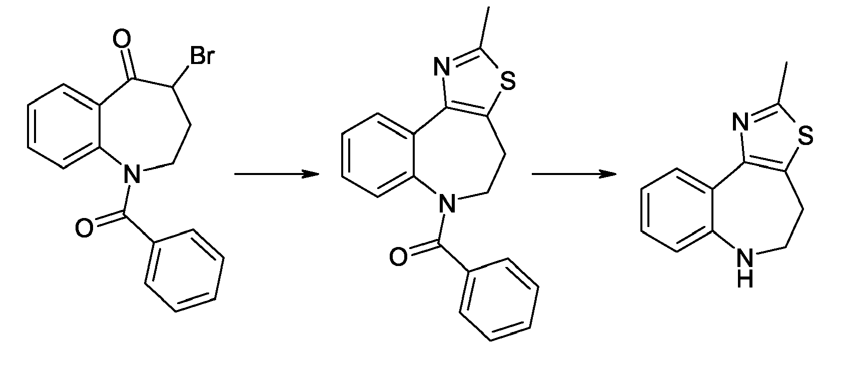

### Example 14.1

### (2-Methyl-4,5-dihydro-3-thia-1,6-diaza-benzo[e]azulen-6-yl)-phenyl-methanone

To a solution of 1-benzoyl-4-bromo-1,2,3,4-tetrahydrobenzo[b]azepin-5-one (1.0g, 2.9mmol) from Example 12.7 in ethanol (50 ml) was added thioacetamide (0.75g, 10mmol). The solution was stirred for 16h. The resultant suspension was reduced in volume by evaporation and cooled. The precipitate was collected by filtration and the solid was washed with cold ethanol and dried to yield the title compound as a white solid (0.65g, 70%).

### Example 14.2

### 2-Methyl-5,6-dihydro-4H-3-thia-1,6-diaza-benzo[e]azulene

A suspension of (2-methyl-4,5-dihydro-3-thia-1,6-diaza-benzo[e]azulen-6-yl)-phenyl-methanone from Example 14.1 (0.42g, 1.3mmol) in 6M hydrochloric acid (45ml) was heated at reflux for 16h. The solution was cooled and treated with saturated NaHCO₃(aq) (100ml). Additional solid NaHCO₃ was added until the solution was basic. The mixture was extracted with dichloromethane and the organic extracts were dried and reduced *in vacuo* to yield the title compound as a yellow oil (0.21g, 76%).

### Example 15

### 4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide

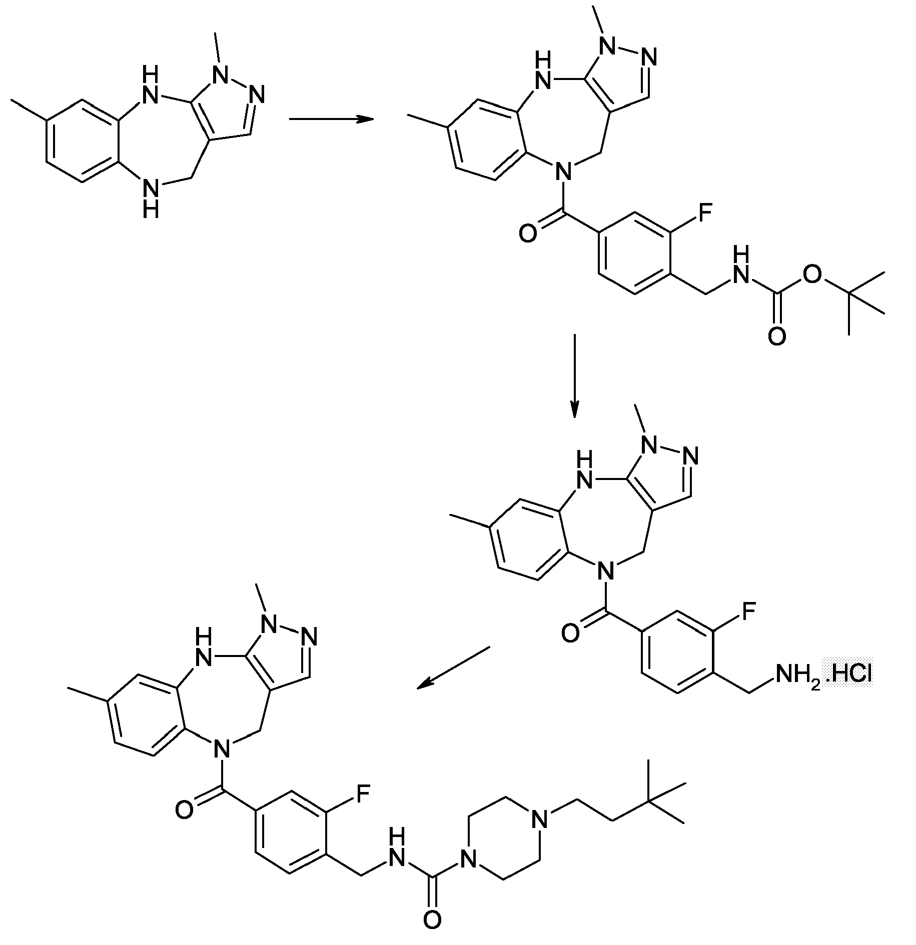

### Example 15.1

### [4-(3,6-Dimethyl-4,10-dihydro-3H-2,3,4,9-tetraaza-benzo[f]azulene-9-carbonyl)-2-fluoro-benzyl]-carbamic acid tert-butyl ester

A solution of 4-(tert-butoxycarbonylamino-methyl)-3-fluorobenzoic acid from Example 10.1 (538mg, 2.0mmol) and DMAP (220mg, 1.8mmol) in dichloromethane (20ml) at room temperature was treated with DIEA (0.93ml, 5.4mmol) and WSCD (460mg, 2.4mmol), and the resulting solution was stirred at room temperature for 1h. 3,6-Dimethyl-3,4,9,10-tetrahydro-2,3,4,9-tetraaza-benzo[f]azulene from Example 2 (385mg, 1.8mmol) was added and the resulting solution was heated at reflux for 20h and allowed to cool to room temperature. The solution was diluted with dichloromethane, washed with saturated NaHCO₃ and brine and concentrated *in vacuo.* The residue was purified by preparative HPLC (eluant 10% methanol:90% dichloromethane) to yield the title compound (265mg, 32%).

### Example 15.2

### (4-Aminomethyl-3-fluoro-phenyl)-(3,6-dimethyl-4,10-dihydro-3H-2,3,4,9-tetraaza-benzo[f]azulen-9-yl)-methanone hydrochloride

A solution of [4-(3,6-dimethyl-4,10-dihydro-3H-2,3,4,9-tetraaza-benzo[f]azulene-9-carbonyl)-2-fluoro-benzyl]-carbamic acid tert-butyl ester (265 mg, 32%) from Example 15.1 (237mg, 0.51mmol) in methanol (3.0ml) at 0°C was treated with 4N HCl/dioxan (8.0ml) and the solution was allowed to warm to room temperature and stirred at room temperature for 30min and concentrated *in vacuo.* The residue was azeotroped several times with diethyl ether to yield the title compound (205mg, 100%).

### Example 15.3

### 4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide

DIEA (0.10ml, 0.60mmol) and 1,1'-carbonyldiimidazole (28mg, 0.17mmol) were added to a solution of (4-aminomethyl-3-fluoro-phenyl)-(3,6-dimethyl-4,10-dihydro-3H-2,3,4,9-tetraaza-benzo[f]azulen-9-yl)-methanone hydrochloride from Example 15.2 (57mg, 0.14mmol) in DMF (5.0ml) and the mixture was stirred at room temperature for 4h. 1-(3,3-Dimethyl-butyl)-piperazine hydrochloride from Example 11 (38mg, 0.16mmol) was added and the mixture was stirred at room temperature for 24h and concentrated *in vacuo.* The residue was dissolved in EtOAc and the resulting solution was washed with brine and concentrated *in vacuo.* The residue was purified by preparative HPLC (eluant; 10% methanol:90% dichloromethane) to give a white solid identified as the title compound (45mg, 56%).
¹H NMR: δ 0.86 (9H, s), 1.33-1.39 (2H, m), 2.12 (3H, s), 2.27-2.38 (6H, m), 3.33-3.35 (4H, m), 3.68 (3H, s), 3.92, (1H, d, J=14.3Hz), 4.25 (2H, d, J=5.4Hz), 5.45 (1H, t, J=5.4Hz), 5.79 (1H, d, J=14.3Hz), 6.43 (1H, d, J=8.0Hz), 6.53 (1H, d, J=8.0Hz), 6.79-7.00 (4H, m), 7.17 (1H, s), 7.31 (1H, s) ppm.
MS: *m*/*z* = 562.4 [M+H]⁺

### Examples 16 - 61

The following compounds were prepared using analogous methods to those described above.

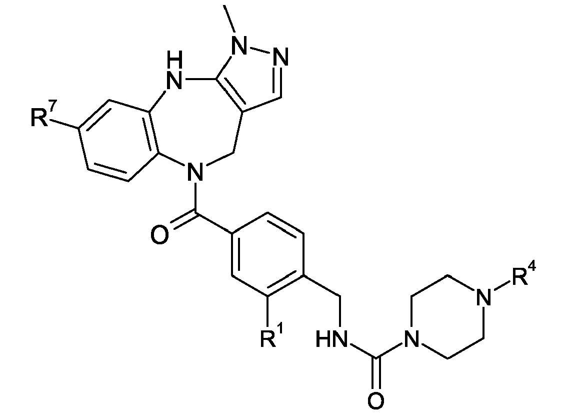

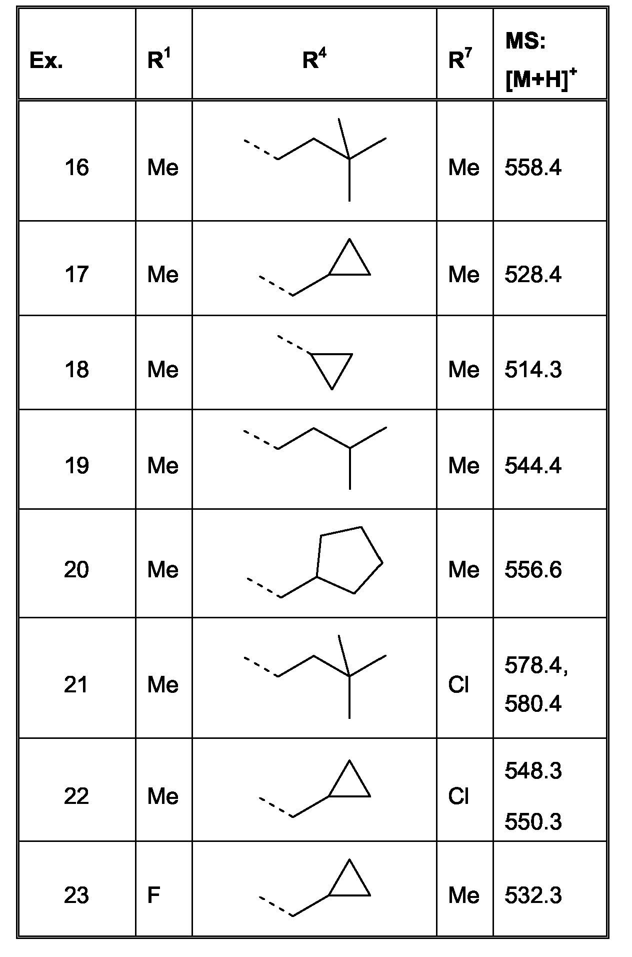

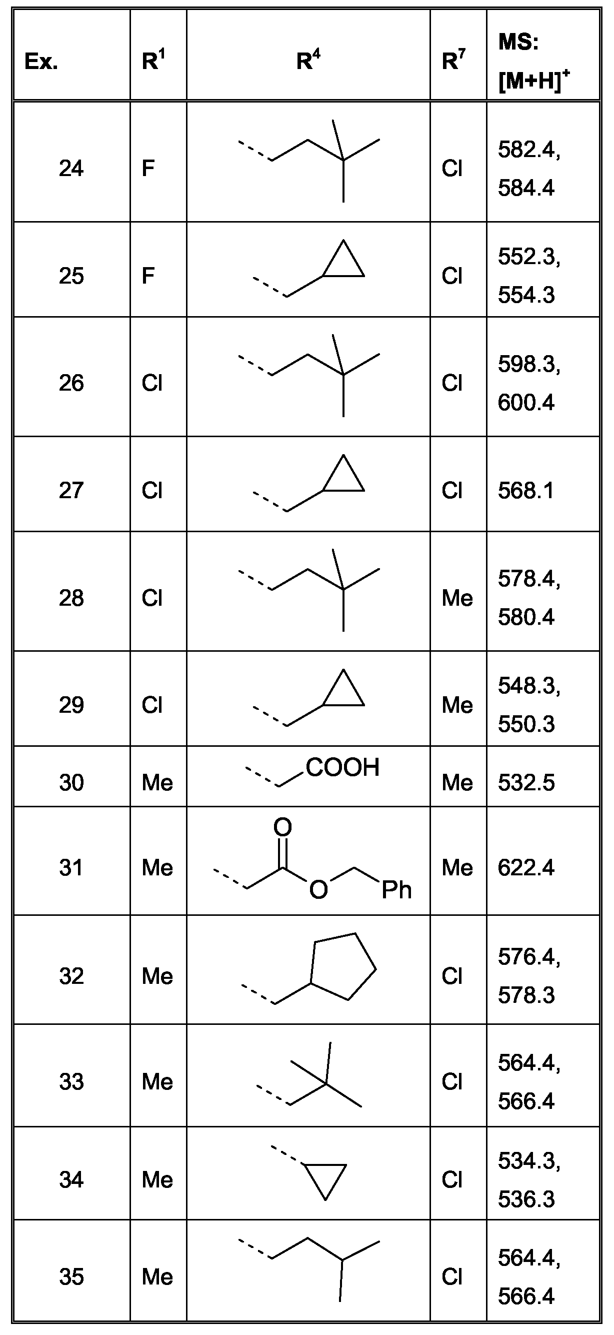

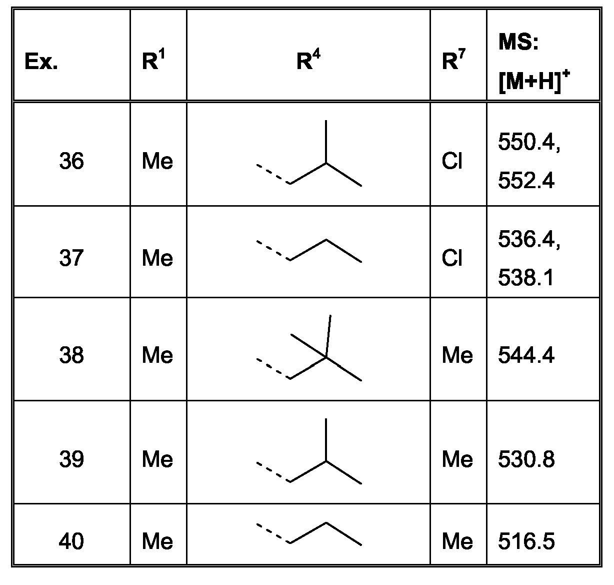

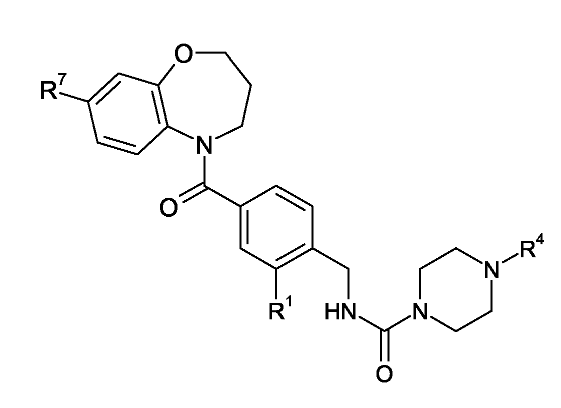

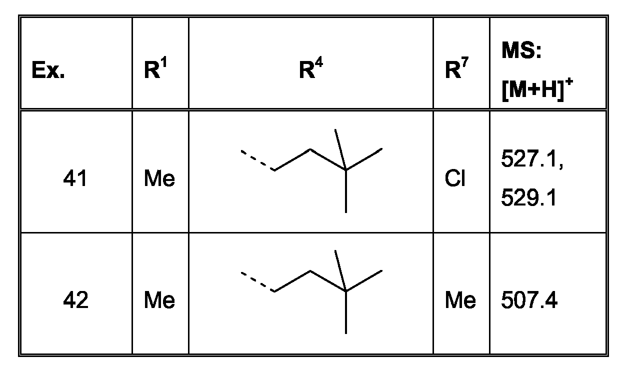

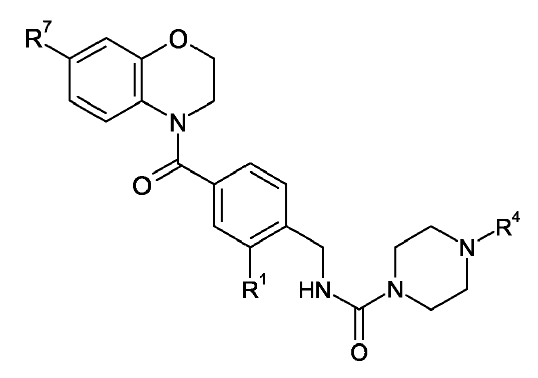

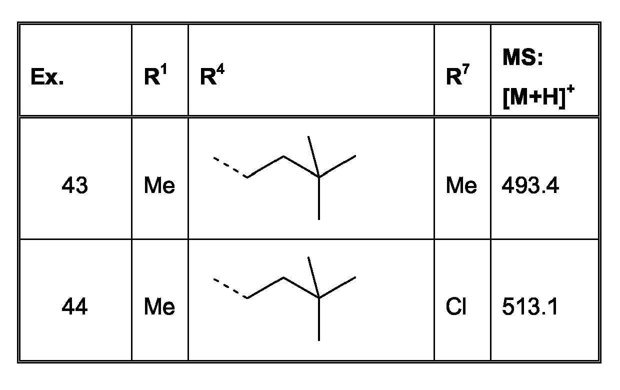

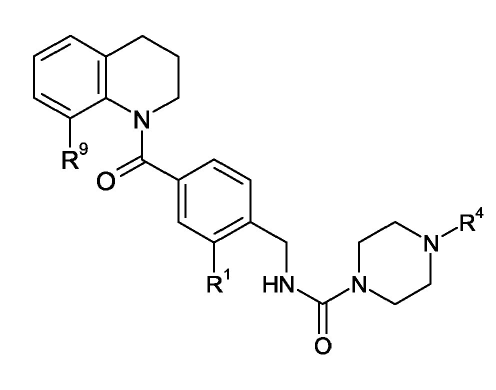

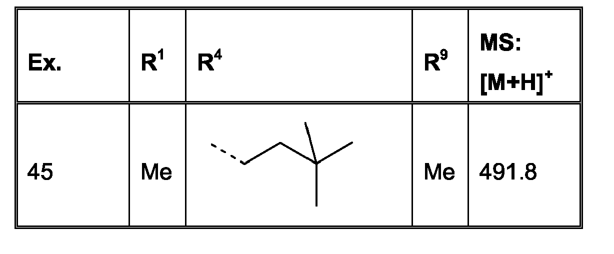

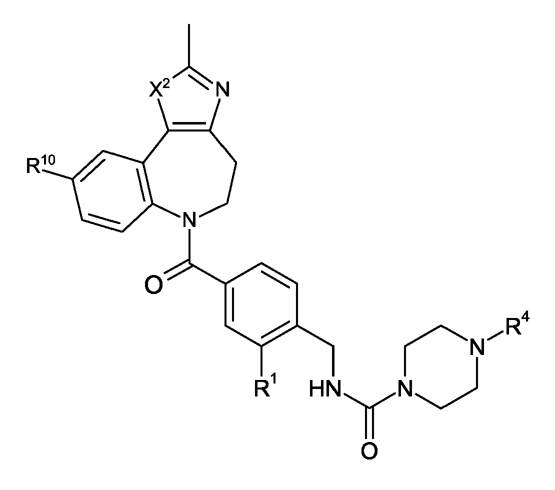

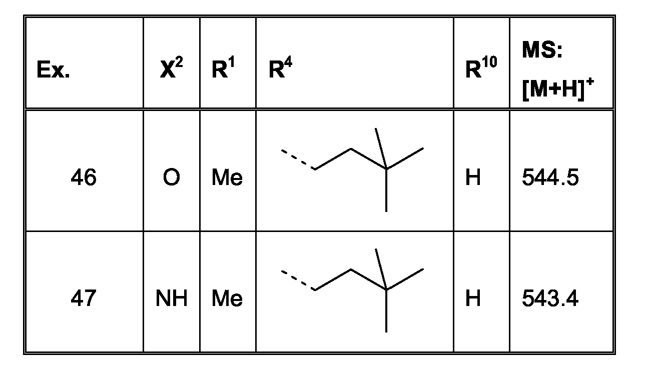

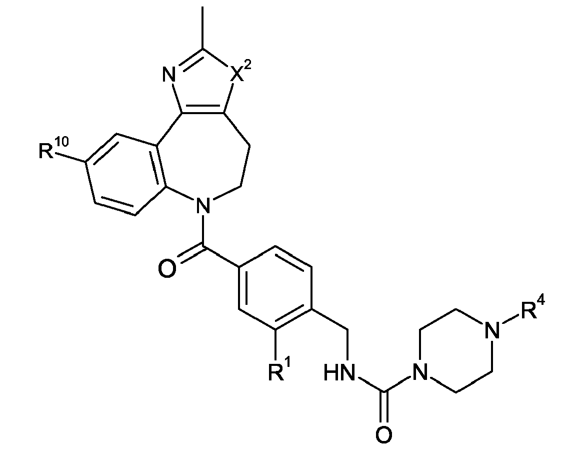

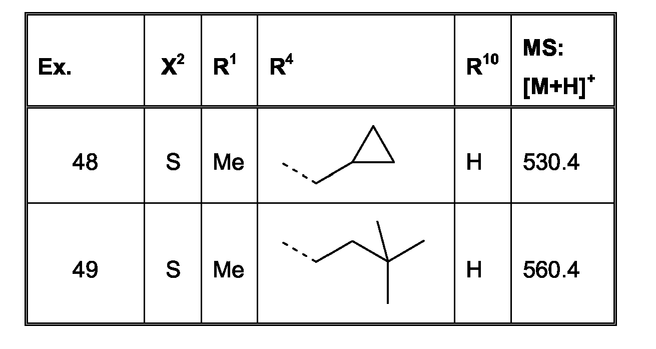

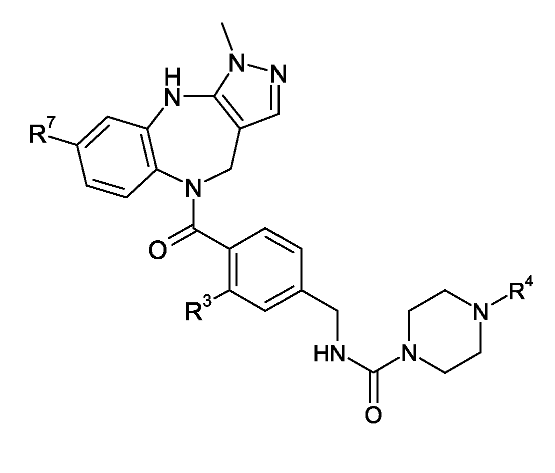

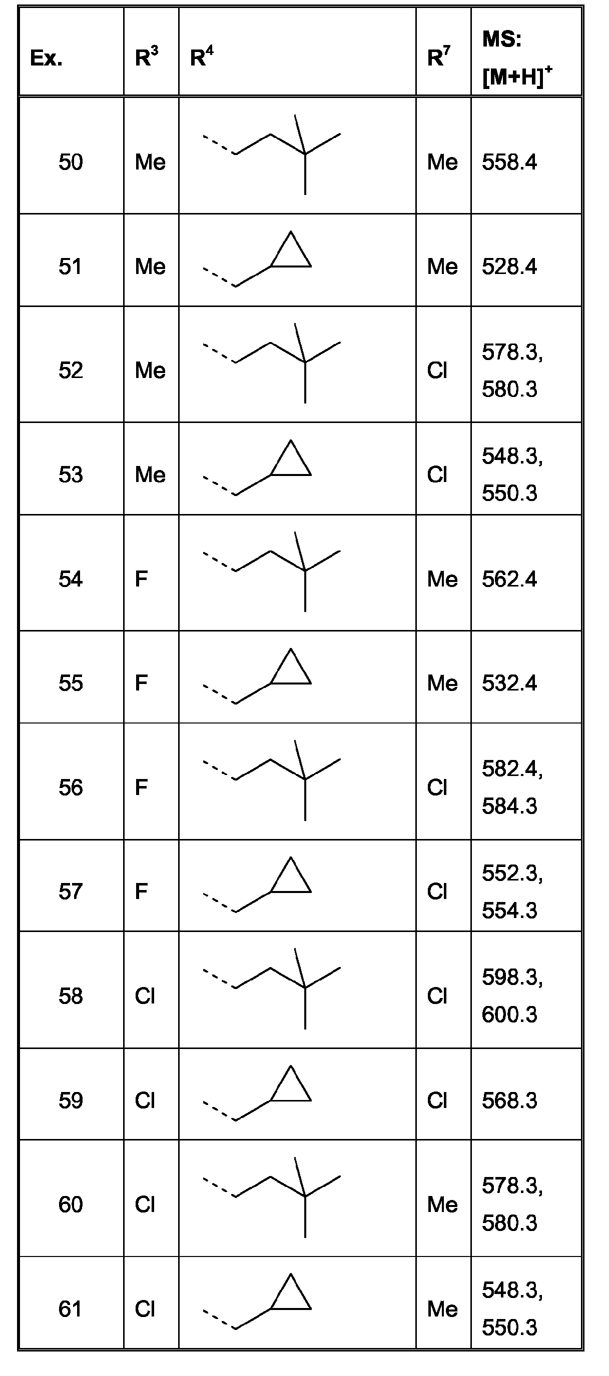

| **Ex.** | ^{**1**}**H NMR: δ(ppm)** |
|---|---|
| 16 | 0.88 (9H, s), 1.34-1.41 (2H, m), 2.09 (3H, s), 2.18 (3H, s), 2.29-2.36 (2H, m), 2.40 (4H, t, J=4.7Hz), 3.37 (4H, t, J=4.7Hz), 3.75 (3H, s), 3.94 (1H, d, J=14.4Hz), 4.24 (2H, d, J=5.2Hz), 4.95 (1H, t), 5.85 (1H, d, J=14.4Hz), 6.53 (1H, q, J=7.9Hz), 6.76 (1H, d, J=5.7Hz), 6.90 (1H, s), 7.02-7.11 (3H, m), 7.58 (1H, s) |
| 17 | 0.05-0.09 (2H, m), 0.47-0.54 (2H, m), 0.81-0.86 (1H, m), 2.08 (3H, s), 2.16 (3H, s), 2.24 (2H, d, J=6.4Hz), 2.47-2.49 (4H, m), 3.38-3.44 (4H, m), 3.67 (3H, s), 3.92 (1H, d, J=14.3Hz), 4.22 (2H, m), 4.92 (1H, m), 5.85 (1H, d, J=14.3Hz), 6.43-6.56 (2H, m), 6.66-6.85 (3H, m), 7.07-7.24 (3H, m) |
| 18 | 0.45-0.47 (4H, m), 1.64 (1H, m), 2.05 (3H, s), 2.13 (3H, s), 2.56 (4H, m), 3.32 (4H, m), 3.65 (3H, s), 3.90 (1H, d, J=14.6Hz), 4.18 (2H, m), 5.21 (1H, m), 5.82 (1H, d, J=14.6Hz), 6.43 (1H, d, J=8.0Hz), 6.53 (1H, d, J=8.0Hz), 6.80-6.85 (3H, m), 6.93 (1H, s), 7.04 (1H, s), 7.16 (1H, s) |
| 19 | 0.84 (3H, s), 0.86 (3H, s), 1.28-1.37 (2H, m), 1.48-1.60 (1H, m), 2.01 (3H, s), 2.10 (3H, s), 2.22-2.35 (2H, m), 2.32 (4H, br t), 3.34 (4H, br t), 3.59 (3H, s), 3.90 (1H, d, J=14.6Hz), 4.16 (2H, d, J=4.9Hz), 5.15 (1H, t, J=5.2Hz), 5.81 (1H, d, J=14.6Hz), 6.40 (1H, d, J=7.9Hz), 6.51 (1H, d, J=7.9Hz), 6.69-6.83 (3H, m), 7.01 (1H, s), 7.11 (1H, s), 7.14 (1H, s) |
| 20 | 1.06-1.23 (2H, m), 1.38-1.59 (4H, m), 1.63-1.78 (2H, m), 1.98-2.05 (1H, m), 2.07 (3H, s), 2.15 (3H, s), 2.22 (2H, d, J=7.4Hz), 2.35 (4H, t, J=4.7Hz), 3.33 (4H, t, J=4.7Hz), 3.67 (3H, s), 3.91 (1H, d, J=14.6Hz), 4.22 (2H, d, J=5.2Hz), 4.84 (1H, br t), 5.84 (1H, d, J=14.6Hz), 6.43 (1H, d, J=8.2Hz), 6.54 (1H, d, J=8.2Hz), 6.67 (1H, s), 6.76 (1H, s), 6.77-6.88 (2H, m), 7.07(1Hs), 7.18(1H, s) |
| 21 | 0.88 (9H, s), 1.34-1.41 (2H, m), 2.09 (3H, s), 2.25-2.36 (2H, m), 2.36-2.41 (4H, m), 3.35-3.42 (4H, m), 3.72 (3H, s), 3.93 (1H, d, J=14.6Hz), 4.24 (2H, d, J=4.45Hz), 5.02 (1H, br t), 5.83 (1H, d, J=14.6Hz), 6.59 (1H, s), 6.88 (1H, s), 7.01-7.09 (3H, m), 7.56-7.63 (2H, m) |
| 22 | 0.11 (2H, q, J=4.7Hz), 0.52 (2H, q, J=4.7Hz), 0.76-0.93 (1H, m), 2.10 (3H, s), 2.31 (2H, d, J=6.4Hz), 2.45-2.62 (4H, m), 3.39-3.53 (4H, m), 3.79 (3H, s), 3.93 (1H, d, J=14.6Hz), 4.23-4.25 (2H, m), 5.27 (1H, s), 5.81 (1H, d, J=14.6Hz), 6.58 (2H, s), 6.80-6.90 (2H, m), 7.07 (3H, s), 7.19 (1H, s) |
| 23 | 0.05-0.11 (2H, m), 0.47-0.55 (2H, m), 0.75-0.89 (1H, m), 2.18 (3H, s), 2.24 (2H, d, J=6.7Hz), 2.47 (4H, m), 3.38 (4H, m), 3.75 (3H, s), 3.93 (1H, d, J=14.6Hz), 4.31 (2H, d, J=5.7Hz), 5.13 (1H, t, J=5.4Hz), 5.82 (1H, d, J=14.6Hz), 6.47-6.58 (2H, m), 6.72-6.80 (2H, m), 6.87-6.95 (2H, m), 7.00-7.04 (1H, m), 7.08-7.17 (1H, m) |
| 24 | 0.86 (9H, s), 1.33-1.39 (2H, m), 2.28-2.38 (6H, m), 3.37 (4H, m), 3.66 (3H, m), 3.94 (1H, d, J=14.6Hz), 4.28 (2H, d, J=5.5Hz), 5.41 (1H, t, J=5.5Hz), 5.78 (1H, d, J=14.6Hz), 6.56 (2H, s), 6.84-6.88 (2H, m), 7.00-7.06 (2H, m), 7.18 (1H, s), 7.98 (1 H, s) |
| 25 | 0.27-0.29 (2H, m), 0.65-0.67 (2H, m), 0.94-1.10 (1H, m), 1.36-1.56 (2H, m), 2.59 (1H, d, J=6.4Hz), 2.76-2.89 (4H, m), 3.54-3.70 (4H, m), 3.83 (3H, s), 3.96 (1H, d, J=14.6), 4.31-4.35 (2H, m), 5.81 (1H, d, J=14.6Hz), 6.63 (1H, s), 6.83-7.00 (1H, m), 7.11 (4H, s), 7.23-7.24 (1H, m), 7.70 (1H, s) |
| 26 | 0.87 (9H, s), 1.33-1.39 (2H, m), 2.28-2.38 (6H, m), 3.37 (4H, m), 3.66 (3H, m), 3.94 (1H, d, J=14.6Hz), 4.31 (2H, d, J=5.5Hz), 5.45 (1H, t, J=5.5Hz), 5.78 (1H, d, J=14.6Hz), 6.91 (1H, m), 7.03 (2H, m), 7.19 (2H, m), 7.84 (1H, s) |
| 27 | (CD30D): 0.14-0.18 (2H, m), 0.52-0.59 (2H, m), 0.89 (1H, m), 2.28-2.30 (2H, m), 2.52-2.56 (4H, m), 3.43-3.47 (4H, m), 3.80 (3H, s), 3.97 (1H, d, J=14.6Hz), 4.34 (2H, d, J=4.5Hz), 5.73 (1H, d, J=14.6Hz), 6.64-6.77 (2H, m), 7.08-7.17 (2H, m), 7.23-7.28 (3H, m) |
| 28 | 0.87 (9H, s), 1.36 (2H, m), 2.12 (3H, s), 2.32 (6H, m), 3.39 (4H, m), 3.66 (3H, s), 3.92 (1H, d, J=14.3Hz), 4.28 (2H, m), 5.43 (1H, m), 5.78 (1H, d, J=14.3Hz), 6.48 (1H, d, J=7.9Hz), 6.52 (1H, d, J=7.9Hz), 6.78 (1H, s), 6.88-6.98 (2H, m), 7.05 (1H, s), 7.17 (1H, m) |
| 29 | 0.06-0.08 (2H, m), 0.48-0.51 (2H, m), 0.76-0.89 (1H, m), 2.15 (3H, s), 2.21-2.29 (2H, m), 2.44-2.48 (4H, m), 3.36-3.38 (4H, m), 3.68 (3H, s), 3.93 (1H, d, J=14.6Hz), 4.30 (2H, d, J=6.0Hz), 5.33 (1H, m), 5.80 (1H, d, J=14.6Hz), 6.48-6.55 (2H, m), 6.77-7.00 (4H, m), 7.19-7.21 (2H, m) |
| 30 | CD30D: 2.18 (3H, s), 2.22 (3H, s), 3.05-3.09 (4H, m), 3.47 (2H, s), 3.58-3.62 (4H, m), 3.79 (3H, s), 3.93 (1H, d, J=14.6Hz), 4.25 (2H, s), 5.75 (1H, d, J=14.6Hz), 6.47 (1H, d, J=8.0Hz), 6.59 (1H, d, J=8.0Hz), 6.97-7.06 (4H, m), 7.21 (1H, s) |
| 31 | 2.02 (3H, s), 2.11 (3H, s), 2.39-2.50 (4H, m), 3.22 (2H, s), 3.32-3.43 (4H, m), 3.60 (3H, s), 3.90 (1H, d, J=14.6Hz), 4.16 (2H, d, J=5.0Hz), 5.12 (2H, s), 5.25 (1H, t, J=5.0Hz), 5.80 (1H, d, J=14.6Hz), 6.41 (1H, d, J=7.9Hz), 6.51 (1H, d, J=7.9Hz), 6.76-6.83 (3H, m), 7.02 (1H, s), 7.12-7.14 (2H, m), 7.28-7.31 (5H, m) |
| 32 | 1.11-1.18 (2H, m), 1.45-1.56 (4H, m), 1.67-1.72 (2H, m), 1.98-2.01, (1H, m), 2.04 (3H, s), 2.22 (2H, d, J=7.2Hz), 2.29-2.50 (4H, m), 3.30-3.41 (4H, m), 3.62 (3H, s), 3.91 (1H, d, J=14.6Hz), 4.19-4.21 (2H, m), 5.04-5.06 (1H, m), 5.80 (1H, d, J=14.6Hz), 6.53-6.54 (2H, m), 6.80-6.84 (2H, m), 7.01-7.03 (2H, m), 7.16 (1H, s), 7.72 (1H, s) |
| 33 | 0.84 (9H, s), 2.05 (2H, s), 2.12 (3H, s), 2.41-2.54 (4H, m), 3.27-3.39 (4H, m), 3.72 (3H, s), 3.92 (1H, d, J=14.6Hz), 4.26-4.27 (2H, m), 4.66-4.68 (1H, m), 5.85 (1H, d, J=14.6Hz), 6.58-6.59 (2H, m), 6.88-6.92 (2H, m), 6.98-7.00 (2H, m), 7.09 (1H, s), 7.21 (1H, s) |
| 34 | 0.38-0.44 (4H, m), 1.57-1.58 (1H, m), 2.04 (3H, s), 2.53 (4H, m), 3.31 (4H, m), 3.62 (3H, s), 3.91 (1H, d, J=14.6Hz), 4.20 (2H, m), 5.08 (1H, m), 5.80 (1H, d, J=14.6Hz), 6.54-6.58 (2H, m), 6.80-6.86 (2H, m), 7.01-7.03 (2H, m), 7.16 (1H, s), 7.71 (1H, s) |
| 35 | 0.85 (6H, d, J=6.4Hz), 1.23-1.36 (2H, m), 1.49-1.56 (1H, m), 2.01 (3H, s), 2.23-2.35 (6H, m), 3.36 (4H, m), 3.62 (3H, s), 3.91 (1H, d, J=14.6Hz), 4.18 (2H, m), 5.29 (1H, m), 5.78 (1H, d, J=14.6Hz), 6.48-6.56 (2H, m), 6.80-6.86 (2H, m), 6.98-7.02 (2H, m), 7.15 (1H, s), 8.12 (1H, s) |
| 36 | 0.85 (6H, d, J=6.4Hz) 1.73 (1H, m), 2.05 (5H, m), 2.26-2.31 (4H, m), 3.34 (4H, m), 3.62 (3H, s), 3.91 (1H, d, J=14.6Hz), 4.21 (2H, m), 5.02 (1H, m), 5.80 (1H, d, J=14.6Hz), 6.54 (2H, m), 6.84-6.87 (2H, m), 7.02 (2H, m), 7.16 (1H, s), 7.72 (1H, s) |
| 37 | 0.85 (3H, t, J=7.2Hz), 1.40-1.49 (2H, m), 2.01 (3H, s), 2.22-2.28 (2H, m), 2.34 (4H, m) 3.35 (4H, m), 3.58 (3H, s), 3.90 (1H, d, J=14.6Hz), 4.18 (2H, m), 5.21 (1H, m), 5.77 (1H, d, J=14.6Hz), 6.51-6.55 (2H, m), 6.81-6.85 (2H, m), 7.00 (2H, m), 7.13 (1H, s), 7.88 (1H, s) |
| 38 | 0.84 (9H, s), 2.04 (2H, s), 2.11 (3H, s), 2.18 (3H, s), 2.45 (4H, t, J=4.7Hz), 3.30 (4H, t, J=4.7Hz), 3.71 (3H, s), 3.92 (1 H, d, J=14.6Hz), 4.24 (2H, d, J=5.2Hz), 4.66 (1 H, br t), 5.86 (1 H, d, J=14.6Hz), 6.41 (1 H, s), 6.46 (1 H, d, J=7.9Hz), 6.56 (1 H, d, J=7.9Hz), 6.75 (1 H, s), 6.88 (2H, s), 7.10 (1H, s), 7.20 (1 H, s) |
| 39 | 0.86 (6H, d, J=6.7Hz), 1.68-1.81 (1 H, m), 2.05 (2H, d, J=7.4Hz), 2.09 (3H, s), 2.17 (3H, s), 2.32 (4H, t, J=4.7Hz), 3.33 (4H, t, J=4.7Hz), 3.69 (3H, s), 3.92 (1 H, d, J=14.6Hz), 4.23 (2H, d, J=4.9Hz), 4.78 (1H, t, J=4.9Hz), 5.85 (1 H, d, J=14.6Hz), 6.46 (1 H, d, J=7.9Hz), 6.55 (1 H, d, J=7.9Hz), 6.57 (1 H, s), 6.76 (1 H, s), 6.86 (2H, s), 7.08 (1 H, s), 7.19 (1 H, s) |
| 40 | 0.88 (3H, t, J=7.4Hz), 1.43-1.54 (2H, m), 2.12 (3H, s), 2.19 (3H, s), 2.28 (2H, t, J=7.4Hz), 2.38 (4H, t, J=4.9Hz), 3.35 (4H, t, J=4.9Hz), 3.73 (3H, s), 3.93 (1 H, d, J=14.6Hz), 4.25 (2H, d, J=5.2Hz), 4.66 (1 H, br t), 5.87 (1 H, d, J=14.6Hz), 6.29 (1 H, s), 6.47 (1 H, d, J=7.7Hz), 6.56 (1 H, d, J=7.7Hz), 6.74 (1 H, s), 6.89 (2H, s), 7.11 (1H, s), 7.21 (1 H, s) |
| 41 | 0.88 (9H, s), 1.35-1.41 (2H, m), 2.00-2.18 (2H, m), 2.27-2.35 (2H, m), 2.36-2.43 (4H, m), 3.34-3.41 (4H, m), 3.44 (2H, s), 4.29 (2H, d, J=5.2Hz), 4.58-4.63 (1 H, m), 6.48-6.61 (1 H, m), 6.62-6.75 (1 H, m), 6.88-7.03 (2H, m), 7.07-7.11 (2H, m) |
| 42 | 0.87 (9H, s), 1.35-1.41 (2H, m), 2.16 (3H, s), 2.22 (3H, s), 2.23-2.36 (2H, m), 2.40 (4H, t, J=4.7Hz), 3.35 (4H, t, J=4.7Hz), 4.28-4.30 (2H, m), 4.50 (1 H, br s), 6.52 (2H, br s), 6.88 (1 H, s), 6.95 (2H, s), 7.11 (1 H, s) |
| 43 | 0.89 (9H, s), 1.36-1.43 (2H, m), 2.23 (3H, s), 2.31 (3H, s), 2.31-2.41 (2H, m), 2.43 (4H, t, J= 4.9Hz), 3.40 (4H, t, J=4.9Hz), 3.95 (2H, t, J=4.7Hz), 4.31 (2H, t, J=4.7Hz), 4.41-4.44 (2H, m), 4.55-4.60 (1H, m), 6.47-6.51 (1 H, m), 6.70 (1 H, s), 6.81-7.00 (1 H, m), 7.21-7.27 (2H, m), 7.34 (1 H, s) |
| 44 | 0.89 (9H, s), 1.36-1.43 (2H, m), 2.31 (3H, s), 2.32-2.39 (2H, m), 2.44 (4H, t, J=4.9Hz), 3.40 (4H, t, J=4.9Hz), 3.94 (2H, t, J=4.4Hz), 4.31 (2H, t, J=4.4Hz), 4.42 (2H, d, J=5.4Hz), 4.62 (1 H, t, J=5.2Hz), 6.64-6.73 (1 H, m), 6.91 (1 H, d, J=2.2Hz), 7.00-7.15 (1 H, m), 7.24 (3H, d, J=3.0Hz) |
| 45 | 0.88 (9H, s), 0.89 (3H, s), 1.32-1.42 (2H, m), 1.65-2.20 (4H, m), 2.30-2.45 (7H, m), 2.49 (2H, t, J=5.2Hz), 2.80-2.90 (1H, m), 3.36 (4H, br s), 3.60 (2H, t, J=4.9Hz), 4.25-4.40 (1H, m), 6.80-7.40 (6H, m), 7.84 (1H, s) |
| 46 | 0.87 (9H, s), 1.33-1.39 (2H, m), 2.12 (3H, s), 2.27-2.36 (2H, m), 2.36-2.43 (4H, m), 2.54 (3H, s), 2.82-2.91 (1H, m), 2.91-2.98 (1H, m), 3.20-3.27 (1H, m), 3.31-3.35 (4H, m), 4.27 (2H, t, J=4.0Hz), 4.54 (1H, t, J=4.9Hz), 5.11-5.16 (1H, m), 6.62-6.72 (2H, m), 6.88-6.98 (2H, m), 6.99 (1H, s), 7.15-7.23 (1H, m), 7.75-7.80 (1H, m) |
| 47 | 0.82 (9H, s), 1.31-1.37 (2H, m), 2.20 (3H, s), 2.25-2.42 (6H, m), 3.32-3.42 (4H, m), 3.55 (3H, s), 3.94 (1H, d, J=14.6Hz), 4.06-4.11 (2H, m), 5.53 (1H, m), 5.77 (1H, d, J=14.6Hz), 6.49-6.57 (2H, m), 6.64-6.73 (2H, m), 6.80-6.94, (3H, m), 7.16 (1H, s), 7.67 (1H, s) |
| 48 | 0.07-0.09 (2H, m), 0.46-0.54 (2H, m), 0.80-0.85, (1H, m), 2.15 (3H, s), 2.23-2.27 (2H, m), 2.45-2.49 (4H, m), 2.73 (3H, s), 3.10-3.18 (2H, m), 3.35-3.37 (4H, m), 3.48-3.68 (1H, m), 4.25-4.29 (2H, m), 4.48 (1H, m), 5.16 (1H, m), 6.67 (2H, m), 6.86 (1H, d, J=7.9Hz), 6.92-6.98 (1H, m), 7.12 (1H, s) 7.20 (1H, m) 8.36 (1H, d, J=7.9Hz) |
| 49 | 0.88 (9H, s), 1.34-1.40 (2H, m), 2.14 (3H, s), 2.29-2.49 (6H, m), 2.72 (3H, s), 3.03-3.18 (2H, m), 3.31-3.35 (4H, m), 3.47-3.67 (1H, m), 4.21-4.31 (2H, m), 4.51-4.54 (1H, m), 5.12-5.19 (1H, m), 6.67 (2H, m), 6.85 (1H, d, J=7.9Hz), 6.92-6.97 (1H, m), 7.06-7.22 (2H, m), 8.36 (1H, dd, J=1.2, 7.9Hz) |
| 50 | 0.85 (9H, s), 1.31-1.39 (2H, m), 1.99 (3H, s), 2.19 (3H, s), 2.24-2.40 (6H, m), 3.33-3.38 (4H, m), 3.53 (3H, s), 3.90 (1H, d, J=14.6Hz), 4.10-4.12 (2H, m), 5.56 (1H, m), 5.75 (1H, d, J=14.6Hz), 6.33-6.35 (1H, m), 6.52-6.56 (2H, m), 6.66-7.11 (3H, m), 7.14 (1H, s), 7.48 (1H, s) |
| 51 | 0.01-0.06 (2H, m), 0.42-0.49 (2H, m), 0.75-0.81 (1H, m), 1.98 (3H, s), 2.17-2.25 (5H, m), 2.38-2.48 (4H, m), 3.32-3.41 (4H, m), 3.50 (3H, s), 3.89 (1H, d, J=14.6Hz), 4.11 (2H, m), 5.54 (1H, m), 5.75 (1H, d, J=14.6Hz), 6.34 (1H, dd, J=1.0, 8.0Hz), 6.50-6.54 (2H, m), 6.66-7.07 (3H, m), 7.13 (1H, s) 7.37 (1H, s) |
| 52 | 0.88 (9H, s), 1.33-1.42 (2H, m), 2.24 (3H, s), 2.27-2.45 (6H, m), 3.35-3.43 (4H, m), 3.68 (3H, s), 3.94 (1H, d, J=14.6Hz), 4.20 (2H, d, J=5.4Hz), 5.01-5.07 (1H, m), 5.79 (1H, d, J=14.6Hz), 6.54 (1H, dd, J=2.2, 8.4Hz), 6.61-7.02 (5Hm), 7.05 (1H, d, J=1.0Hz), 7.53 (1H, d, J=4.7Hz) |
| 53 | 0.01-0.06 (2H, m), 0.42-0.47 (2H, m), 0.76-0.80 (1H, m), 2.15-2.23 (5H, m), 2.41-2.46 (4H, m), 3.35-3.48 (4H, m), 3.54 (3H, s), 3.90 (1H, d, J=14.6Hz), 4.11 (2H, m), 5.46 (1H, m), 5.72 (1H, d, J=14.6Hz), 6.46 (1H, dd, J=1.5, 8.5Hz), 6.54-6.61 (2H, m), 6.67-6.73 (1H, m), 6.81-7.09 (2H, m), 7.13 (1H, s), 7.99 (1H, s) |
| 54 | 0.84 (9H, s), 1.31-1.37 (2H, m), 1.99 (3H, s), 2.24-2.33 (6H, m), 3.34 (4H, m), 3.57 (3H, s), 3.90 (1H, d, J=14.6Hz), 4.08 (2H, m), 5.70 (1H, d, J=14.6Hz), 6.01 (1Hm), 6.34 (1H, m), 6.53-6.60 (2H, m), 6.70 (1H, s), 6.82-6.91 (2H, m), 7.10 (1H, s), 7.49 (1H, s) |
| 55 | 0.02-0.08 (2H, m), 0.44-0.51 (2H, m), 0.77-0.82 (1H, m), 2.02 (3H, s), 2.16-2.24 (2H, m), 2.42-2.44 (4H, m), 3.36-3.42 (4H, m), 3.57 (3H, s), 3.92 (1H, d, J=14.6Hz), 4.11 (2H, m), 5.72 (1H, d, J=14.6Hz), 5.86 (1H, m), 6.36 (1H, dd, J=1.0, 8.0Hz), 6.54-6.89 (5H, m), 7.12 (1H, s), 7.27 (1H, s) |
| 56 | 0.87 (9H, s), 1.33-1.40 (2H, m), 2.27-2.40 (6H, m), 3.34-3.42 (4H, m), 3.60 (3H, s), 3.94 (1H, d, J=14.6Hz), 4.16 (2H, d, J=5.2Hz), 5.72 (1H, d, J=14.6Hz), 5.81 (1H, t, J=5.4, 11.1 Hz), 6.50 (1H, dd, J=2.2, 8.4Hz), 6.60-6.73 (3H, m), 6.91-7.02 (3H, m), 8.00 (1H, s) |
| 57 | 0.02-0.08 (2H, m), 0.44-0.48 (2H, m), 0.77-0.81 (1H, m), 2.20-2.24, (2H, m), 2.43 (4H, m), 3.37-3.47 (4H, m), 3.57 (3H, s), 3.93 (1H, d, J=14.6Hz), 4.14 (2H, m), 5.70 (1H, d, J=14.6Hz), 5.82 (1H, m), 6.48 (1H, dd, J=2.0, 8.5Hz), 6.58-6.72 (3H, m), 6.89-7.00 (2H, m), 7.14 (1H, s), 7.92 (1H, s) |
| 58 | 0.89 (9H, s), 1.34-1.42 (2H, m), 2.29-2.46 (6H, m), 3.35-3.44 (4H, m), 3.66 (3H, s), 3.98 (1H, d, J=14.6Hz), 4.21 (2H, d, J=5.4Hz), 5.14-5.24 (1H, m), 5.76 (1H, d, J=14.6Hz), 6.58 (1H, dd, J=2.2, 8.4Hz), 6.74-7.00 (4H, m), 7.07 (1H, s), 7.22 (1H, s) |
| 59 | 0.02-0.07 (2H, m), 0.43-0.48 (2H, m), 0.77-0.81 (1H, m), 2.19-2.24 (2H, m), 2.42-2.47 (4H, m), 3.37-3.41 (4H, m), 3.56 (3H, s), 3.95 (1H, d, J=14.6Hz), 4.10 (2H, m), 5.68 (1H, d, J=14.6Hz), 5.89 (1H, m), 6.49 (1H, dd, J=2.0, 8.5Hz), 6.68-6.96 (5H, m), 7.14 (1H, s), 7.93 (1H, s) |
| 60 | 0.82 (9H, s), 1.33-1.41 (2H, m), 1.99 (3H, s), 2.28-2.38 (6H, m), 3.36-3.37 (4H, m), 3.60 (3H, s), 3.95 (1H, d, J=14.6Hz), 4.07 (2H, m), 5.69 (1H, d, J=14.6Hz), 6.06 (1H, m), 6.37 (1H, m), 6.61-6.82 (3H, m), 6.94 (2H, m), 7.14 (1H,s), 7.46 (1H, s) |
| 61 | 0.02-0.07 (2H, m), 0.43-0.50 (2H, m), 0.76-0.81 (1H, m), 1.97 (3H, s), 2.16-2.23 (2H, m), 2.41-2.45 (4H, m), 3.36-3.44, (4H, m), 3.54 (3H, s), 3.93 (1H, d, J=14.6Hz), 4.05 (2H, m), 5.68 (1H, d, J=14.6Hz), 6.00 (1H, m), 6.34 (1H, m), 6.58-6.79 (4H, m), 6.90 (1H, s), 7.11 (1H, s), 7.35(1H, s) |

### Example 62

### 6-Chloro-3-methyl-3,4,9,10-tetrahydro-2,3,4,9-tetraaza-benzo[f]azulene

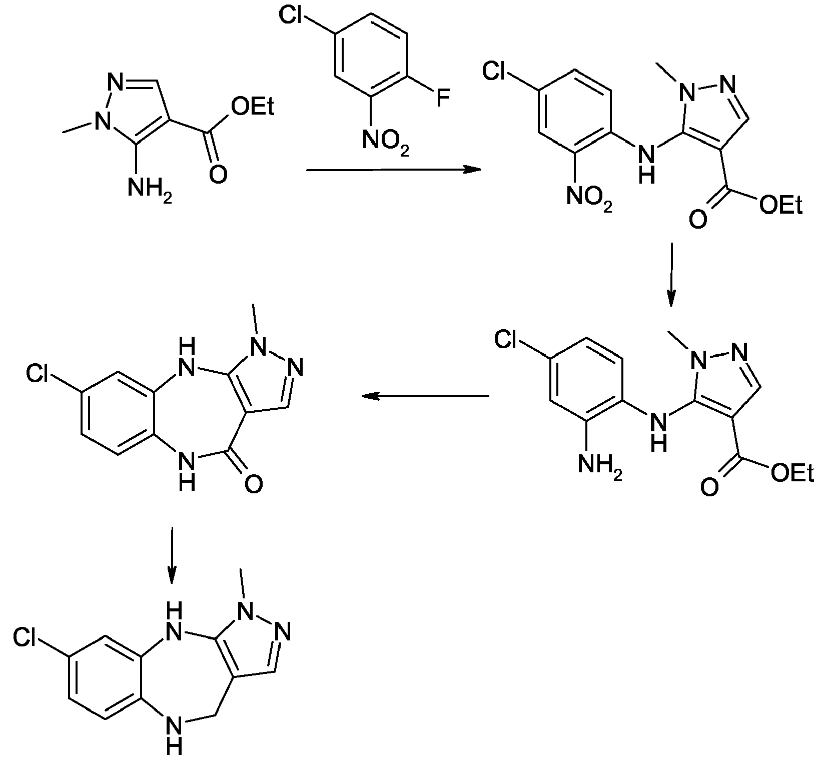

### Example 62.1

### 5-(4-Chloro-2-nitro-phenylamino)-1-methyl-1H-pyrazole-4-carboxylic acid ethyl ester

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h. A solution of 4-chloro-2-fluoronitrobenzene (22.6g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x 2), and the combined organic extracts were washed with brine and concentrated in vacuo. The residue was purified by flash chromatography on silica gel (eluant; 60% pet ether:40% EtOAc) to yield the title compound (27.5g, 62%) .

### Example 62.2

### 5-(2-Amino-4-chloro-phenylamino)-1-methyl-1H-pyrazole-4-carboxylic acid ethyl ester

Zinc powder (26.17g, 400mmol) was added to a suspension of 5-(4-chloro-2-nitro-phenylamino)-1-methyl-1H-pyrazole-4-carboxylic acid ethyl ester from Example 62.1 (26.0g, 80mmol) in methanol/acetic acid (10:1, 330ml) at room temperature. After the exothermic reaction which followed, the resulting suspension was stirred at room temperature for 18h before being filtered through Celite® filter agent. The filtrate was concentrated in vacuo. The residue was dissolved in EtOAc, and the solution was washed with saturated NaHCO₃ and brine, and concentrated in vacuo. The residue was purified by flash chromatography on silica gel (eluant; 65% EtOAc:35% pet ether to 80% EtOAc:20% pet ether) to yield the title compound (18.41g, 78.0%) .

### Example 62.3

### 6-Chloro-3-methyl-4,9-dihydro-3H-2,3,4,9-tetraaza-benzo[f]azulen-10-one

Sodium (methylsulphinyl)methanide (53.4mmol) was prepared from sodium hydride (60% dispersion in oil, 2.14g, 53.4mmol) and anhydrous dimethyl sulphoxide (35ml) by heating at 65°C until a solution was observed. To this was added a solution of 5-(2-amino-4-chloro-phenylamino)-1-methyl-1H-pyrazole-4-carboxylic acid ethyl ester from Example 62.2 (9.02g, 30.5mmol) in anhydrous dimethyl sulphoxide (20ml), and stirring continued at 65°C for 30min. The mixture was poured into ice (200ml), and the resulting solid collected and purified by recrystallisation from methanol/EtOAc to yield the title compound (5.56g, 73.3%).

### Example 62.4

### 6-Chloro-3-methyl-3,4,9,10-tetrahydro-2,3,4,9-tetraaza-benzo[f]azulene

To a suspension of 6-chloro-3-methyl-4,9-dihydro-3H-2,3,4,9-tetraaza-benzo[f]azulen-10-one from Example 62.3 (5.56g, 22.4mmol) in anhydrous THF (200ml) at 0°C was added lithium aluminium hydride (4.24g, 112mmol), and the resulting suspension was heated at reflux for 18h, then allowed to cool to room temperature. A further portion of lithium aluminium hydride (4.24g, 112mmol) was added, and the mixture was heated at reflux for 18h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in vacuo. The residue was purified by flash chromatography on silica gel (eluant; 0.5% ammonia (35%):5.0% methanol:94.5% dichloromethane) to yield the title compound (3.88g, 74%).

### Example 63

### 4-(tert-Butoxycarbonylamino-methyl)-cyclohexanecarboxylic acid

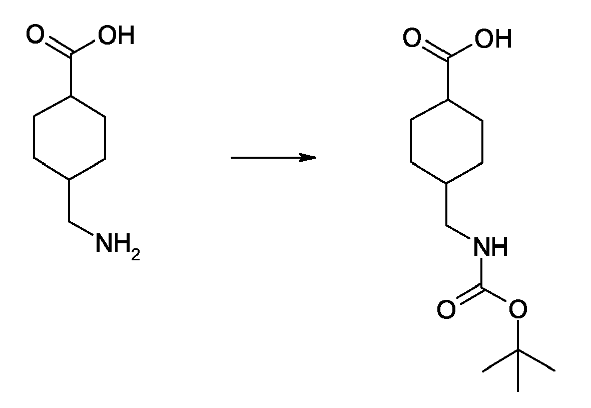

To a solution of 4-aminomethyl-cyclohexanecarboxylic acid (20.0g, 127.39mmol) in dioxan (400ml) was added 1N KHCO₃ (300ml, 300mmol) and di-tert-butyl dicarbonate (33.3g, 129.57mmol). The mixture was stirred for 18h and concentrated in vacuo. The aqueous residue was washed with ether, then acidified with 1N KHSO₄ and extracted with EtOAc (x 3). The combined organic extracts were washed with water and brine and concentrated in vacuo to give a white solid identified as the title compound (31.9g, 98%).

### Example 64

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

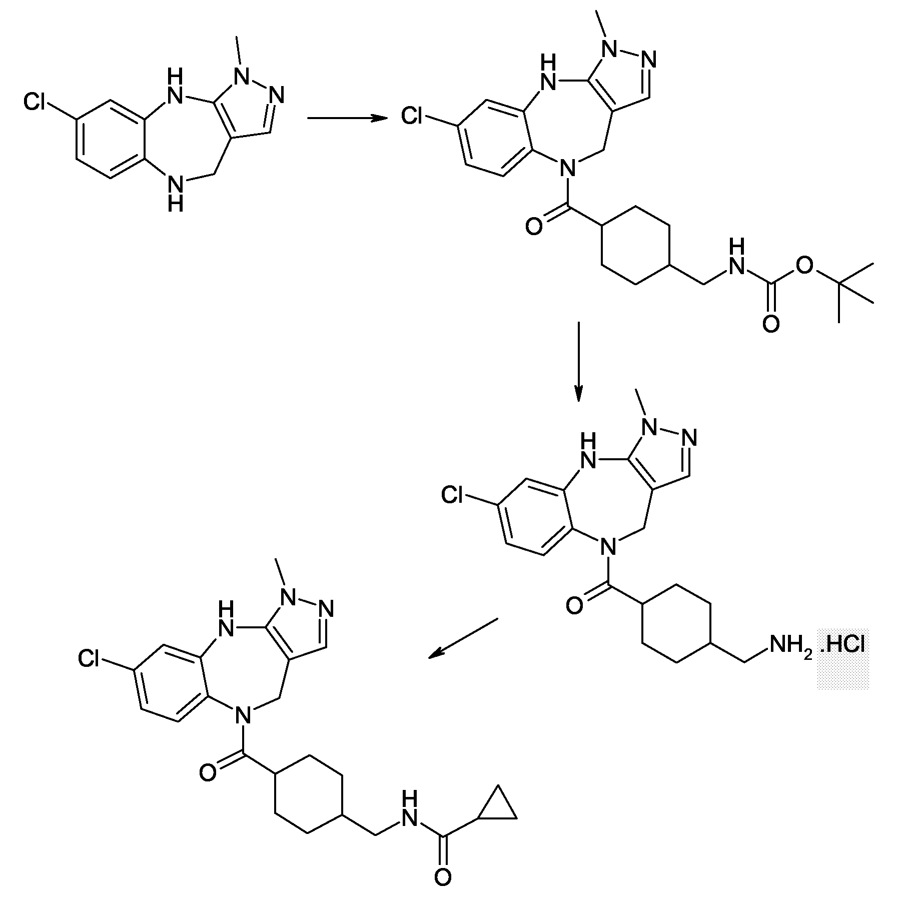

### Example 64.1

### [4-(6-Chloro-3-methyl-4,10-dihydro-3H-2,3,4,9-tetraaza-benzo[f]azulene-9-carbonyl)-cyclohexylmethyl]-carbamic acid tert-butyl ester

A solution of 4-(tert-butoxycarbonylamino-methyl)-cyclohexanecarboxylic acid from Example 63 (510mg, 2.0mmol) in dichloromethane (25 ml) at room temperature was treated with DIEA (0.70ml, 4.0mmol), PyBrop (2.40g, 5.1mmol), and 6-chloro-3-methyl-3,4,9,10-tetrahydro-2,3,4,9-tetraaza-benzo[f]azulene from Example 62 (422mg, 1.8mmol) and the resulting solution was heated at reflux for 20h and allowed to cool to room temperature. The solution was diluted with dichloromethane, washed with brine and concentrated in vacuo. The residue was purified by flash chromatography on silica gel (eluant EtOAc) to yield the title compound (775mg, 91%).

### Example 64.2

### (4-Aminomethyl-cyclohexyl)-(6-chloro-3-methyl-4,10-dihydro-3H-2,3,4,9-tetraaza-benzo[f]azulen-9-yl)-methanone hydrochloride

A solution of [4-(6-chloro-3-methyl-4,10-dihydro-3H-2,3,4,9-tetraaza-benzo[f]azulene-9-carbonyl)-cyclohexylmethyl]-carbamic acid tert-butyl ester from Example 64.1 (775mg, 1.63mmol) in methanol (20.0ml) at 0°C was treated with 4N HCl/dioxan (10.0ml) and the solution was allowed to warm to room temperature and stirred at room temperature for 2h and concentrated in vacuo. The residue was azeotroped with methanol, chloroform and dichloromethane to yield the title compound (655mg, 100%).

### Example 64.3

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

DIEA (0.50ml, 2.90mmol) and cyclopropanecarbonyl chloride (0.045ml, 0.50mmol) were added to a solution of (4-aminomethyl-cyclohexyl)-(6-chloro-3-methyl-4,10-dihydro-3H-2,3,4,9-tetraaza-benzo[f]azulen-9-yl)-methanone hydrochloride from Example 64.2 (220mg, 0.53mmol) in dichloromethane (5ml). The mixture was stirred for 2h then diluted with dichloromethane, washed with water and concentrated in vacuo. The residue was purified by preparative HPLC (eluant; 0.5% 35% ammonia/10% methanol/90% dichloromethane) to give a white solid identified as the title compound (132 mg, 60%).
¹H NMR (270MHz, CDCl₃): δ 0.42-0.90 (6H, m), 1.28 (6H, m), 1.78-1.80 (2H, m), 2.08 (1H, m), 2.95-3.01 (2H, m), 3.66 (3H, s), 3.76 (1H,d, J=14.6Hz), 5.55 (1H, d, J=14.6Hz), 5.95 (1H, m), 6.97 (1H, dd, J=8.4, 2.1Hz), 7.03 (1H, s), 7.07 (1H, d, J=8.4Hz), 7.09 (1H, d, J=2.1Hz), 7.14 (1H, s) .
MS: (ESI)⁺: [M+H]⁺=442.3/444.3

### Example 65

### 3-Methyl-3,4,9,10-tetrahydro-2,3,4,9-tetraaza-benzo[f]azulene

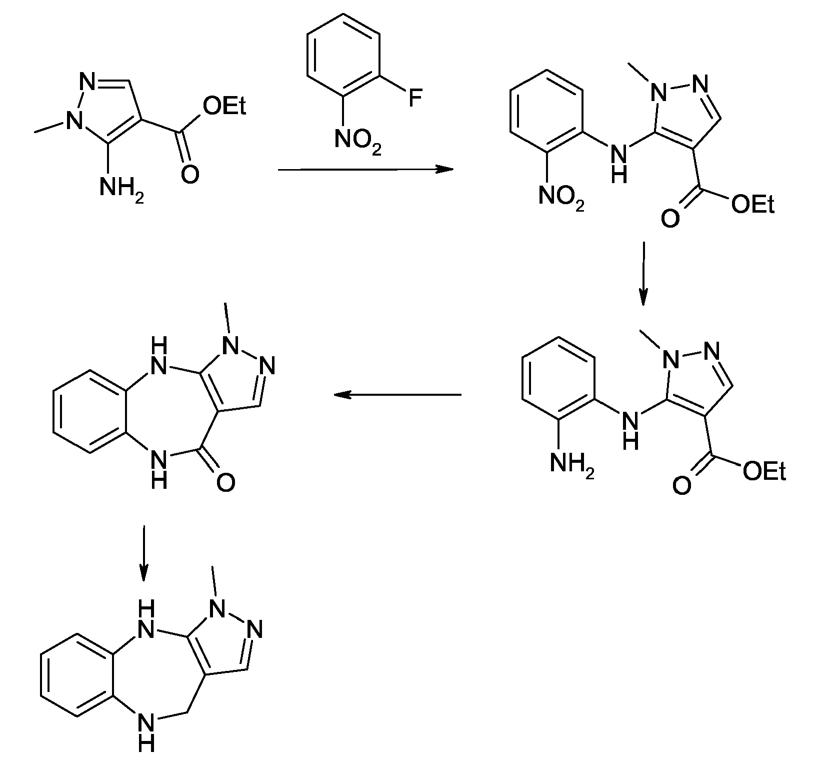

### Example 65.1

### 1-Methyl-5-(2-nitro-phenylamino)-1H-pyrazole-4-carboxylic acid ethyl ester

Sodium hydride (60% dispersion in oil, 7.0g, 170mmol) was added portionwise to a suspension of 5-amino-1-methyl-1H-pyrazole-4-carboxylic acid ethyl ester (21.1g, 125mmol) in anhydrous THF (300ml) at 0°C. The mixture was allowed to warm to room temperature and stirred for 0.75h then cooled to 0°C. 1-fluoro-2-nitrobenzene (17.6g, 125mmol) was added and the resultant suspension was stirred at room temperature for 18h. EtOAc and 0.3M KHSO4 were added and separated. The aqueous layer was extracted with EtOAc and the combined organic layers were washed with brine, dried and concentrated in vacuo. The residue was purified by flash chromatography on silica gel (eluant 50% hexanes/50% ethyl acetate) to yield the title compound (20.8g, 58%).

### Example 65.2

### 5-(2-Amino-phenylamino)-1-methyl-1H-pyrazole-4-carboxylic acid ethyl ester

1-Methyl-5-(2-nitro-phenylamino)-1H-pyrazole-4-carboxylic acid ethyl ester (20.8g, 72mmol) from Example 65.1 was dissolved in methanol (330ml) and hydrogenated over 10% Pd/C catalyst for 4h. The mixture was filtered through Celite® filter agent and the filtrate was concentrated in vacuo to give a white solid identified as the title compound (16.2g, 87%).

### Example 65.3

### 3-Methyl-4,9-dihydro-3H-2,3,4,9-tetraaza-benzo[f]azulen-10-one

Sodium (methylsulphinyl)methanide (29.7mmol) was prepared from sodium hydride (60% dispersion in oil, 1.19g, 29.7mmol) and anhydrous dimethyl sulphoxide (7ml) by heating at 65°C until a solution was observed. To this was added a solution of 5-(2-amino-phenylamino)-1-methyl-1H-pyrazole-4-carboxylic acid ethyl ester from Example 65.2 (3.63g, 16.9mmol) in anhydrous dimethyl sulphoxide (10ml), and stirring continued at 65°C for 2.5h. The mixture was poured into ice (100ml), and the resulting solid collected and purified by recrystallisation from methanol/EtOAc/60-80 pet ether to yield the title compound (1.46g, 40%).

### Example 65.4

### 3-Methyl-3,4,9,10-tetrahydro-2,3,4,9-tetraaza-benzo[f]azulene

To a suspension of 3-methyl-4,9-dihydro-3H-2,3,4,9-tetraaza-benzo[f]azulen-10-one from Example 65.3 (3.01g, 14.1 mmol) in anhydrous THF (100ml) at 0°C was added lithium aluminium hydride (2.13g, 56.2mmol), and the resulting suspension was heated at reflux for 18h, then allowed to cool to room temperature. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in vacuo. The residue was purified by flash chromatography on silica gel (eluant; 2% methanol:chloroform rising to 5% methanol:chloroform) to yield the title compound (1.60g, 57%).

### Example 66

### 3-Fluoro-4-hydroxy-benzoic acid ethyl ester

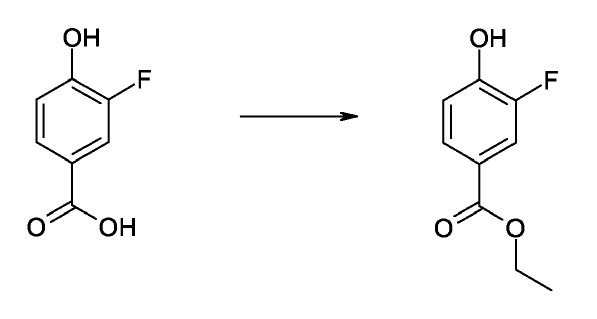

A solution of 3-fluoro-4-hydroxy-benzoic acid (5.16g, 33.1mmol) in ethanol (100ml) was treated with conc. sulphuric acid (5 ml), and the mixture heated under reflux for 4 days, then allowed to cool to room temperature. The volatiles were removed in vacuo, and the aqueous residue was basified with saturated NaHCO₃ and extracted twice with ether. The combined organic extracts were dried over MgSO₄ and concentrated in vacuo to yield the title compound (5.16g, 85%).

### Example 66

### 4-(3-Hydroxy-propyl)-piperazine-1-carboxylic acid tert-butyl ester

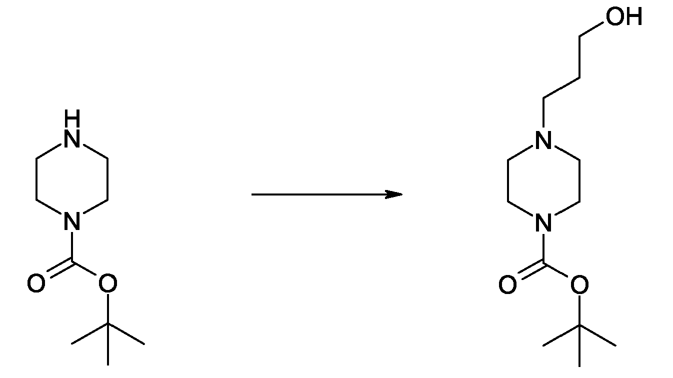

A solution of piperazine-1-carboxylic acid tert-butyl ester (26.5g, 142 mmol) in acetone (300ml) at room temperature was treated with 3-bromo-propan-1-ol (14.5 ml, 156.2 mmol), potassium carbonate (50g, 361.8 mmol) and potassium iodide (2.4g, 14.2 mmol), and the resulting mixture was heated at reflux for 18h then allowed to cool to room temperature. The suspension was filtered and the filtrate concentrated in vacuo. The residue was purified by flash chromatography on silica gel (eluant; 5% methanol:dichloromethane) to yield the title compound (27.7g, 80%).

### Example 68

### 4-[3-(4-Carboxy-2-fluoro-phenoxy)-propyl]-piperazine-1-carboxylic acid tert-butyl ester

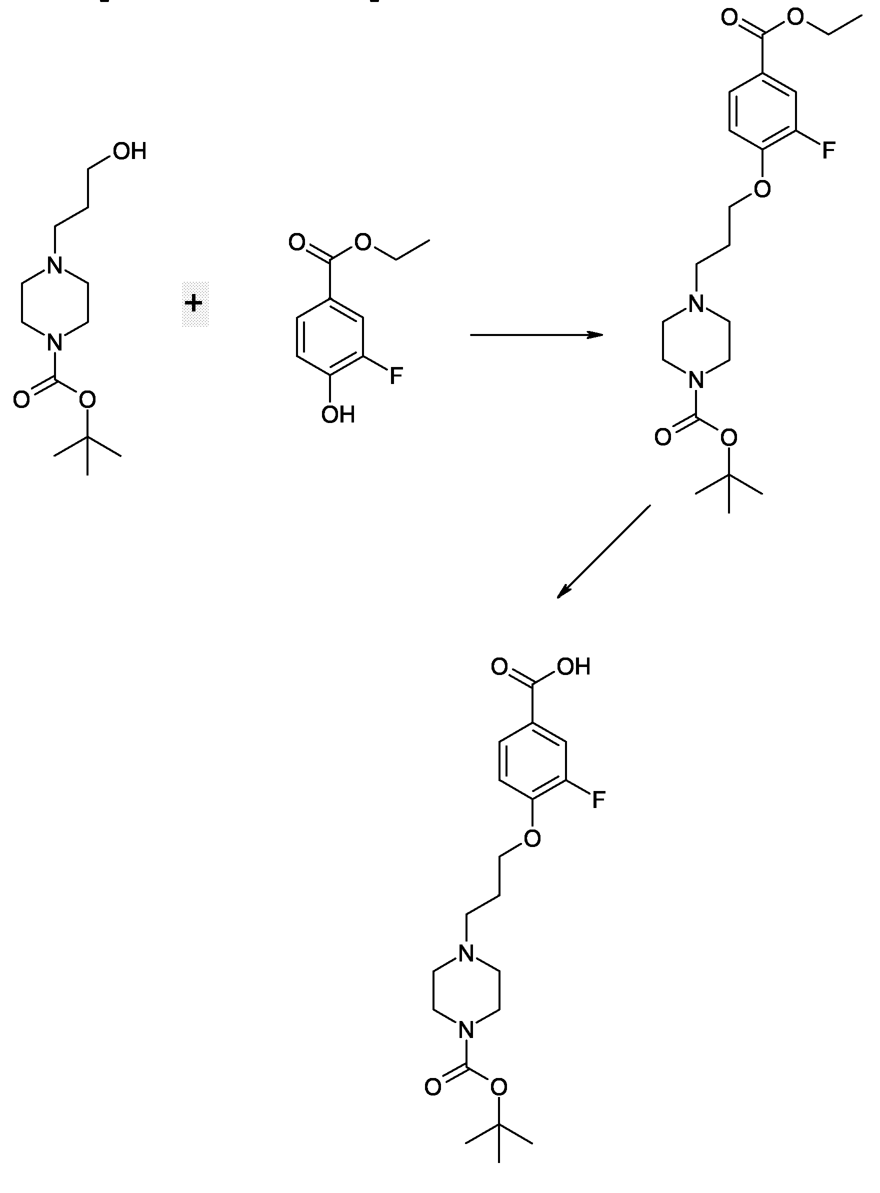

### Example 68.1

### 4-[3-(4-Ethoxycarbonyl-2-fluoro-phenoxy)-propyl]-piperazine-1-carboxylic acid tert-butyl ester

To a solution of 3-fluoro-4-hydroxy-benzoic acid ethyl ester from Example 66 (1.3g, 7.37 mmol) and 4- (3-hydroxypropyl)-piperazine-1-carboxylic acid tert-butyl ester from Example 67 (1.8g, 7.37mmol) in tetrahydrofuran (100ml) at 0°C were added triphenylphosphine polystyrene (loading 1.0 mmol/g, 11g, 11.0 mmol) and DEAD (diethyl azodicarboxylate) (2.45g, 11mmol), and the resulting suspension was allowed to warm to room temperature and stirred for 18h. The mixture was filtered and the filtrate concentrated in vacuo. The residue was purified by flash chromatography on silica gel (eluant; EtOAc) to yield the title compound (1.7g, 57%).

### Example 68.2

### 4-[3-(4-Carboxy-2-fluoro-phenoxy)-propyl]-piperazine-1-carboxylic acid tert-butyl ester

A solution of 4-[3-(4-ethoxycarbonyl-2-fluoro-phenoxy)-propyl]-piperazine-1-carboxylic acid tert-butyl ester from Example 68.1 (1.7g, 4.1 mmol) in dioxan (25ml) was treated with 2N NaOH (3ml) and the mixture stirred at 50°C for 18h. A further aliquot of 2N NaOH was added (2ml), and stirring continued at 50°C for 3h. The mixture was allowed to cool to room temperature, and the volatiles removed in vacuo. The residue was azeotroped with toluene, and purified by flash chromatography on silica gel (eluant; 1% AcOH:9% MeOH:90% CHCl₃) to yield the title compound (1.45g, 92%).

### Example 69

### (4-{3-[4-(3,3-Dimethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone

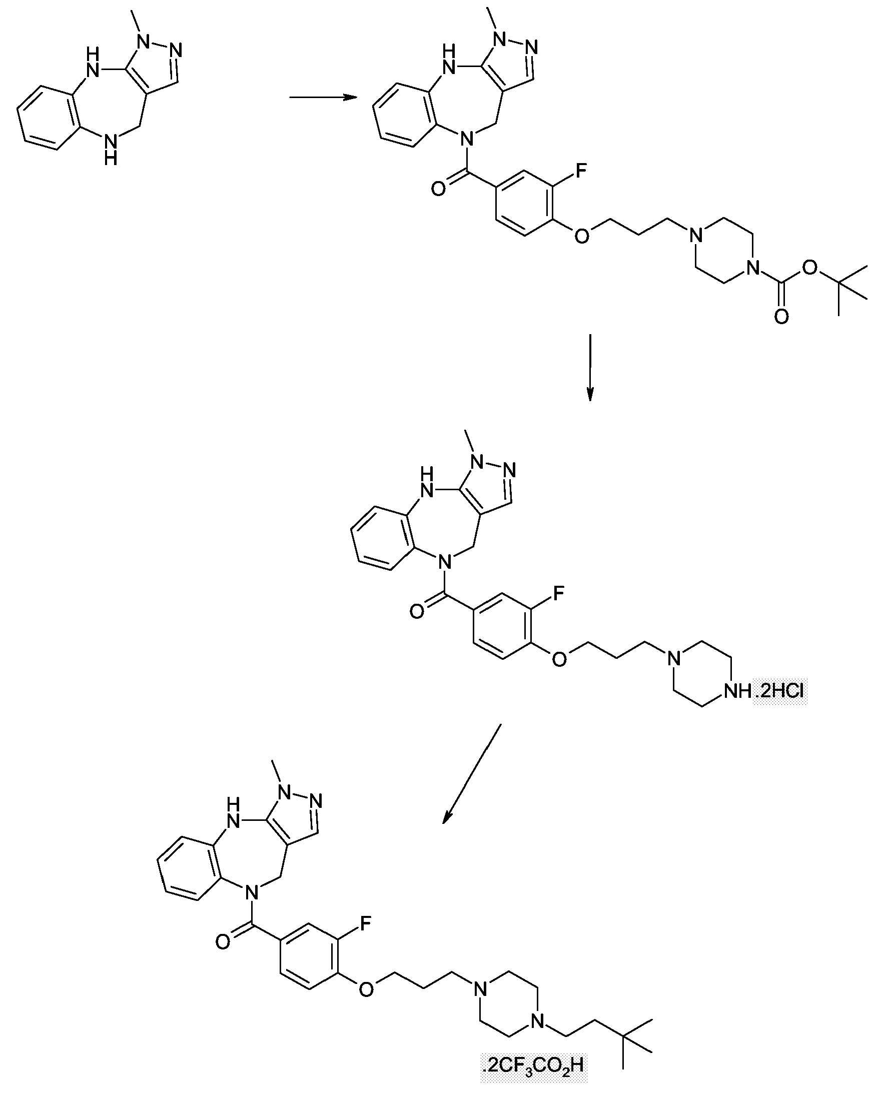

### Example 69.1

### 4-{3-[2-Fluoro-4-(3-methyl-4,10-dihydro-3H-2,3,4,9-tetraaza-benzo[f]azulene-9-carbonyl)-phenoxy]-propyl}-piperazine-1-carboxylic acid tert-butyl ester

A solution of 4-[3-(4-carboxy-2-fluoro-phenoxy)-propyl]-piperazine-1-carboxylic acid tert-butyl ester from Example 68.2 (865mg, 2.3mmol) in dichloromethane (50ml) was treated with triethylamine (to pH9), WSCD (865mg, 4.5mmol) and DMAP (276mg, 2,.3mmol), and the mixture stirred at room temperature for 10min. 3-Methyl-3,4,9,10-tetrahydro-2,3,4,9-tetraaza-benzo[f]azulene from Example 65.4 (541mg, 2.7mmol) was added, and the resulting mixture was heated at reflux for 18h, allowed to cool to room temperature and concentrated in vacuo. The residue was dissolved in EtOAc, washed with saturated NaHCO₃ and brine and concentrated in vacuo. The residue was purified by flash chromatography on silica gel (eluant; 10% MeOH:90% dichloromethane) to yield the title compound (615mg, 48%).

### Example 69.2

### [3-Fluoro-4-(3-piperazin-1-yl-propoxy)-phenyl]-(3-methyl-4,10-dihydro-3H-2,3,4,9-tetraaza-benzo[f]azulen-9-yl)-methanone dihydrochloride

A solution of 4-{3-[2-fluoro-4-(3-methyl-4,10-dihydro-3H-2,3,4,9-tetraaza-benzo[f]azulene-9-carbonyl)-phenoxy]-propyl}-piperazine-1-carboxylic acid tert-butyl ester from Example 69.1 (615 mg, 1.09 mmol) in methanol (2ml) at 0°C was treated with 4N HCl/dioxan (5ml) and the solution was allowed to warm to room temperature and stirred at room temperature for 1h and concentrated in vacuo to yield the title compound (585 mg, 100%).

### Example 69.3

### (4-{3-[4-(3,3-Dimethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone bis(trifluoroacetate)

To a solution of [3-fluoro-4-(3-piperazin-1-yl-propoxy)-phenyl]-(3-methyl-4,10-dihydro-3H-2,3,4,9-tetraaza-benzo[f]azulen-9-yl)-methanone dihydrochloride from Example 69.2 (585mg, 1.09mmol) in methanol/acetic acid (99:1, v/v, 20ml) were added triethylamine (to pH9) and 3,3-dimethylbutyraldehyde (120mg, 1.20mmol) and the resulting mixture was stirred at room temperature for 1h. Sodium cyanoborohydride (103mg, 1.30mmol) was added and the resulting mixture was stirred at room temperature for 18h then concentrated in vacuo. The residue was purified by flash chromatography on silica gel (eluant; 10% methanol: 90% dichloromethane) to yield (4-{3-[4-(3,3-dimethylbutyl)-piperazin-1-yl]-propoxy}-3-fluoro-phenyl)-(3-methyl-4,10-dihydro-3H-2,3,4,9-tetraaza-benzo[f]azulen-9-yl)-methanone, which was lyophilised from aqueous trifluoroacetic acid to yield the title compound (700mg, 83%).
¹H NMR (270MHz, d₆ DMSO): 0.89-0.95 (11H, m), 1.58-1.62 (2H, m), 2.14-2.17 (2H, m), 3.03-3.08 (8H, m), 3.49-3.52 (2H, m), 3.79 (1H, d, J=14.6Hz), 3.96 (3H, s), 4.06-4.09 (2H, m), 5.65 (1H, d, J=14.6Hz), 6.74-6.97 (5H, m), 7.15-7.21 (2H, m), 7.37-7.42 (1H, m) 8.83 (1H, s).
MS: (APCI)⁺:[M+H]⁺=549.4

### Example 70

### 3,6-Dimethyl-3,4,9,10-tetrahydro-2,3,4,9-tetraaza-benzo[f]azulene

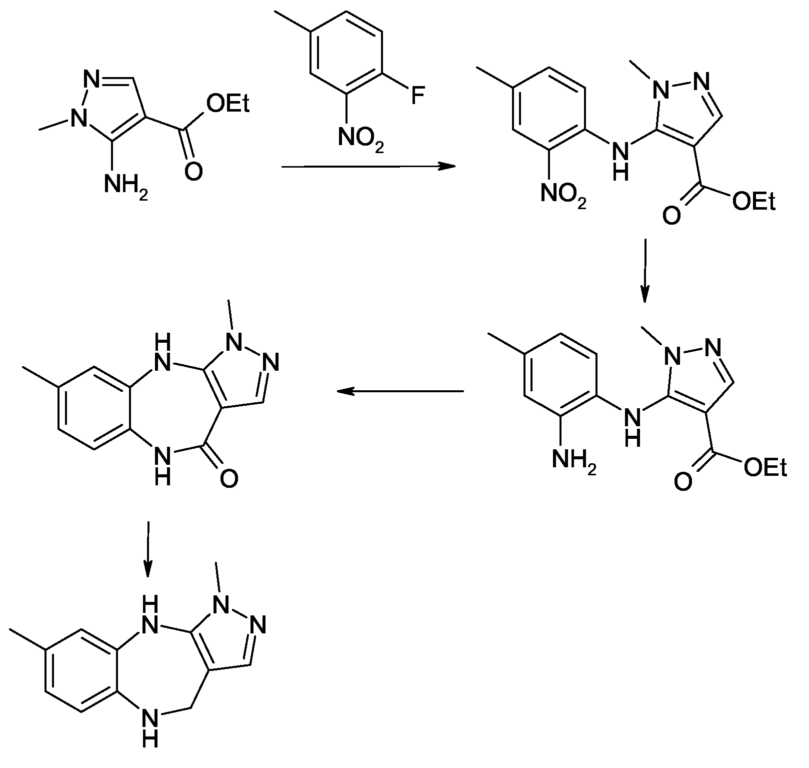

### Example 70.1

### 1-Methyl-5-(4-methyl-2-nitro-phenylamino)-1H-pyrazole-4-carboxylic acid ethyl ester

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h then a solution of 3-fluoro-4-nitrotoluene (20g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x2), and the combined organic extracts were washed with brine and concentrated in vacuo. The residue was purified by flash chromatography on silica gel (eluant; 60% pet ether:40% EtOAc) to yield the title compound (28.00g, 61%).

### Example 70.2

### 5-(2-Amino-4-methyl-phenylamino)-1-methyl-1H-pyrazole-4-carboxylic acid ethyl ester

Tin (II) chloride (86.65g, 457.0mmol) was added to a solution of 1-methyl-5-(4-methyl-2-nitro-phenylamino)-1H-pyrazole-4-carboxylic acid ethyl ester from Example 70.1 (28.00g, 91.4mmol) in methanol and the mixture heated at reflux for 3 days. The solvent was removed in vacuo and the residue taken up in EtOAc (400ml) and cooled to 0°C. 35% Ammonia solution was added to pH14, and the mixture was stirred for 15min before being filtered through Celite® filter agent. The filtrate was washed with 2M NH₃ and brine and concentrated in vacuo. The residue was purified by flash chromatography on silica gel (eluant; 6% methanol:94% dichloromethane rising to 10% methanol:90% dichloromethane) to yield the title compound (12.8g, 52%).

### Example 70.3

### 3,6-Dimethyl-4,9-dihydro-3H-2,3,4,9-tetraaza-benzo[f]azulen-10-one

Sodium (methylsulphinyl)methanide (22.7mmol) was prepared from sodium hydride (60% dispersion in oil, 912mg, 22.7mmol) and anhydrous dimethyl sulphoxide (5.5ml) by heating at 65°C until a solution was observed. To this was added a solution of 5-(2-amino-4-methyl-phenylamino)-1-methyl-1H-pyrazole-4-carboxylic acid ethyl ester from Example 70.2 (3.56g, 13.0mmol) in anhydrous dimethyl sulphoxide (10ml), and stirring continued at 65°C for 30min. The mixture was then poured into ice (200ml), the resulting solid collected and purified by flash chromatography on silica gel (eluant; 10% methanol:90% dichloromethane) to yield the title compound (1.12g, 38%).

### Example 70.4

### 3,6-Dimethyl-3,4,9,10-tetrahydro-2,3,4,9-tetraaza-benzo[f]azulene

To a suspension of 3,6-dimethyl-4,9-dihydro-3H-2,3,4,9-tetraaza-benzo[f]azulen-10-one from Example 70.3 (2.35g, 10.3mmol) in anhydrous THF (100ml) at 0°C was added lithium aluminium hydride (1.56g, 41.2mmol), and the resulting suspension was heated at reflux for 18h then allowed to cool to room temperature. A further portion of lithium aluminium hydride (781mg, 20.6mmol) was added, and the mixture was heated at reflux for 3h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in vacuo. The residue was purified by flash chromatography on silica gel (eluant; 10% methanol:90% dichloromethane) to yield the title compound (1.60g, 72%).

### Example 71

### 4-Cyano-3-methyl-benzoic acid

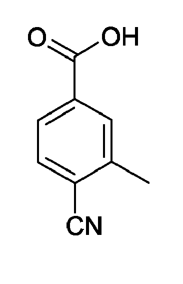

Example 71 is described in WO 03/01631 A1, pages 12-17. (Hudson, P. J.; Pitt, G. R. W.; Rooker, D. P.; Batt, A. R. Laurent, C. M. S.; Roe, M. B. "Diazacycloalkanes as Oxytocin Agonists"), pages 26-31.

### Example 72

### Cyclopropanecarboxylic acid 4-(3,6-dimethyl-4,10-dihydro-3H-2,3,4,9-tetraaza-benzo[f]azulene-9-carbonyl)-2-methyl-benzylamide

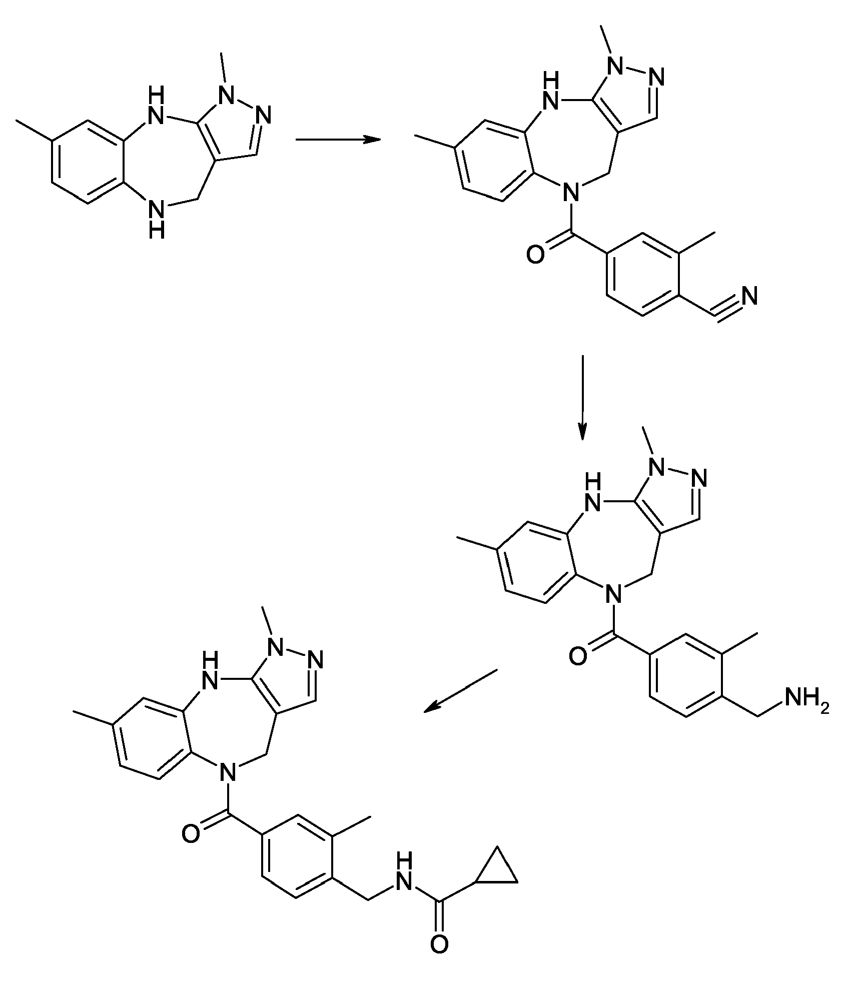

### Example 72.1

### 4-(3,6-Dimethyl-4,10-dihydro-3H-2,3,4,9-tetraaza-benzo[f]azulene-9-carbonyl)-2-methyl-benzonitrile

Thionyl chloride (1.02ml, 14.0mmol) was added to a stirred suspension of 4-cyano-3-methylbenzoic acid from Example 71 (753mg, 4.67mmol) in toluene (10ml). The mixture was heated at reflux for 2h, cooled to room temperature and concentrated in vacuo. The residue was azeotroped with toluene then dissolved in dichloromethane (45ml). To the resulting solution was added a stirred solution of 3,6-dimethyl-3,4,9,10-tetrahydro-2,3,4,9-tetraaza-benzo[f]azulene from Example 70.4 (1.00 g, 4.67 mmol) and triethylamine (1.30ml, 9.34mmol) in dichloromethane (5ml). The mixture was stirred at room temperature for 24h, washed with brine and concentrated in vacuo. The residue was purified by flash chromatography on silica gel (eluant EtOAc) to yield the title compound (1.43 g, 850) .

### Example 72.2

### (4-Aminomethyl-3-methyl-phenyl)-(3,6-dimethyl-4,10-dihydro-3H-2,3,4,9-tetraaza-benzo[f]azulen-9-yl)-methanone

Cobalt(II) chloride hexahydrate (1.90g, 8.00mmol) was added to a solution of 4-(3,6-dimethyl-4,10-dihydro-3H-2,3,4,9-tetraaza-benzo[f]azulene-9-carbonyl)-2-methylbenzonitrile from Example 72.1 (1.43g, 4.00mmol) in methanol (50ml) at room temperature. After stirring for 15min, sodium borohydride (1.51g, 40.0mmol) was added portion-wise and the mixture was stirred at room temperature for 2h. The mixture was filtered through Celite® and the filtrate concentrated in vacuo. The residue was partitioned between EtOAc/saturated ammonium chloride and the layers were separated. The organic layer was dried over MgSO₄ and concentrated in vacuo. The residue was purified by flash chromatography on silica gel (eluant 1% ammonia (35%)/9%methanol/90% dichloromethane) to yield the title compound (511mg, 35%).

### Example 72.3

### Cyclopropanecarboxylic acid 4-(3,6-dimethyl-4,10-dihydro-3H-2,3,4,9-tetraaza-benzo[f]azulene-9-carbonyl)-2-methyl-benzylamide

Triethylamine (0.15ml, 1.04mmol) and cyclopropanecarbonyl chloride (0.04ml, 0.46mmol) were added to a solution of (4-aminomethyl-3-methyl-phenyl)-(3,6-dimethyl-4,10-dihydro-3H-2,3,4,9-tetraaza-benzo[f]azulen-9-yl)-methanone from Example 72.2 (150 mg, 0.42 mmol) in dichloromethane (10ml). The mixture was stirred for 18h then concentrated in vacuo. The residue was purified by preparative HPLC (eluant; 0.5% ammonia (35%)/9.5% methanol/90% dichloromethane) to give a white solid identified as the title compound (91mg, 51%).
¹H NMR (270MHz, CDCl₃) : δ 0.62-0.71 (2H,m), 0.81-0.89 (2H, m),1.39-1.50 (1H, m), 2.04 (3H, s), 2.12 (3H, s), 3.66 (3H, s) 3.91(1H, d, J=14.60Hz) 4.22 (2H, d, J=1. 98Hz) 5.82 (1H, d, J=14. 60Hz) 6.46 (2H, q, J=7.92Hz), 6. 68-6. 87 (4H, m), 7.06 (2H, s)
MS: (APCI)⁺: [M+H]⁺=430.4

### Example A

### In vitro Testing

Compounds were assayed to determine their ability to inhibit the cellular consequences of AVP stimulation on intact cells. In the assay, the compounds of the invention cause significant inhibition of cellular activation at concentrations of 30 µM or less. Preferred compounds cause significant inhibition at concentrations of 300 nM.

### Example B

### Tablet for Oral Administration

Tablets containing 100 mg of the compound of Example 15 as the active agent may be prepared from the following:

| | |
|---|---|
| Compound of Example 15 | 200.0 g |
| Corn starch | 71.0 g |
| Hydroxypropylcellulose | 18.0 g |
| Carboxymethylcellulose calcium | 13.0 g |
| Magnesium stearate | 3.0 g |
| Lactose | 195.0 g |
| *Total* | *500.0 g* |

The materials are blended and then pressed to give 2000 tablets of 250 mg, each containing 100 mg of the compound of Example 15.

All the cited references are hereby incorporated in their entirety.

## Claims

1. A compound according to general formula **1,** or a compound which is a tautomer or a pharmaceutically acceptable salt thereof, 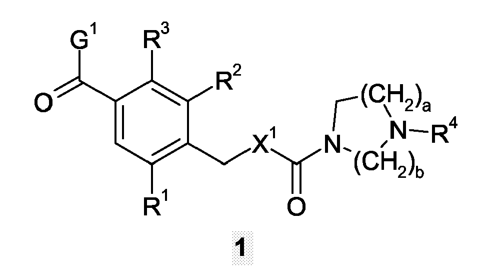
wherein:
G¹ is a bicyclic or tricyclic fused piperidine or azepine derivative selected among general formula **2, 3, 4** and **5** 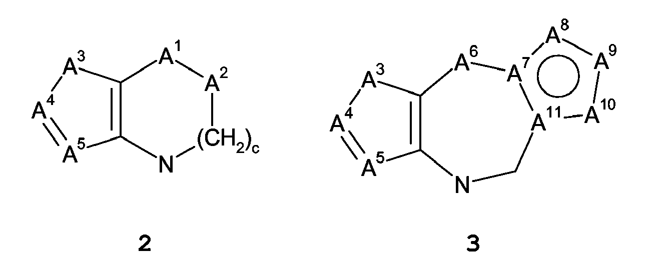 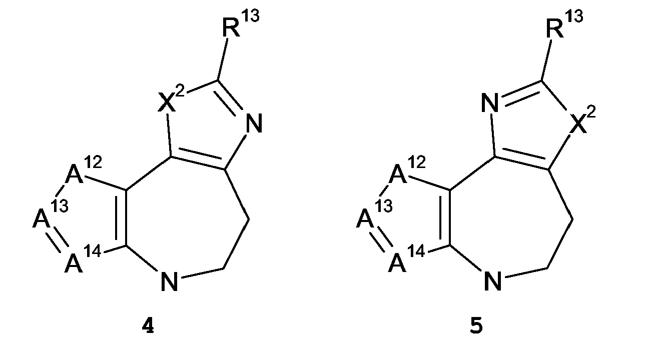
A¹ is selected among CH₂, CH(OH), NH, N-alkyl, O and S;
A² is selected among CH₂, CH(OH), C(=O) and NH;
A³ is selected among S, NH, N-alkyl, -C(R⁷)=CH-, -C(R⁷) =N-, -N=C(R⁷)- and -CH=C(R⁷)-;
A⁴ is selected among C(R⁸) and N;
A⁵ is selected among C(R⁹) and N;
A⁶ is selected among CH₂, NH, N-alkyl and O;
A⁷ and A¹¹ are independently selected among C and N;
A⁸ and A⁹ are independently selected among CH, N, NH, N(CH₂)_{d}R⁵ and S;
A¹⁰ is selected among -CH=CH-, CH, N, NH, N-(CH₂)_{d}-R⁵ and S;
A¹² is selected among S, NH, N-alkyl, -C(R¹⁰)=CH-, -C(R¹⁰)=N-, -N=C(R¹⁰)- and -CH=C(R¹⁰)-;
A¹³ is selected among C(R¹¹) and N;
A¹⁴ is selected among C (R¹²) and N;
X¹ is selected among O and NH;
X² is selected among O, S, NH and N-alkyl;
R¹, R² and R³ are independently selected among H, alkyl, O-alkyl, F, Cl and Br;
R⁴ is selected among H, alkyl, optionally substituted phenyl, optionally substituted pyridyl, optionally substituted thienyl, optionally substituted furyl,
-(CH₂)ₑ-R⁶ and Y-R¹⁵;
R¹³ and R¹⁵ are independently selected among H, alkyl, optionally substituted phenyl, optionally substituted pyridyl, optionally substituted thienyl, optionally substituted furyl and -(CH₂)_{f}-R¹⁴;
R⁵, R⁶ and R¹⁴ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON (alkyl) ₂, CN and CF₃;
R⁷, R⁸ and R⁹ are independently selected among H, alkyl, alkoxy, F, Cl, Br, CN, NH₂, NO₂, NH(alkyl) and N (alkyl) ₂;
R¹⁰, R¹¹ and R¹² are independently selected among H, alkyl, alkoxy, F, Cl, Br, CN, NH₂, NO₂, NH(alkyl), and N (alkyl) ₂;
Y is selected among C=O and S(=O)_{g};
a is selected among 1 and 2;
b is selected among 1, 2 and 3;
c is selected among 1 and 2;
d is selected among 1, 2 and 3;
e is selected among 1, 2 and 3;
f is selected among 1, 2 and 3;
g is selected among 1 and 2; and subject to the proviso that the ring containing A³, A⁴ and A⁵ according to general formulae **2** and **3** is substituted in at least one position by alkyl, alkoxy, F, Cl, Br, CN, NH₂, NO₂, NH(alkyl) or N(alkyl)₂;
and to the proviso that the ring constituted by A⁷, A⁸, A⁹, A¹⁰ and A¹¹ is aromatic.

2. The compound according to claim 1, wherein G¹ is selected among:

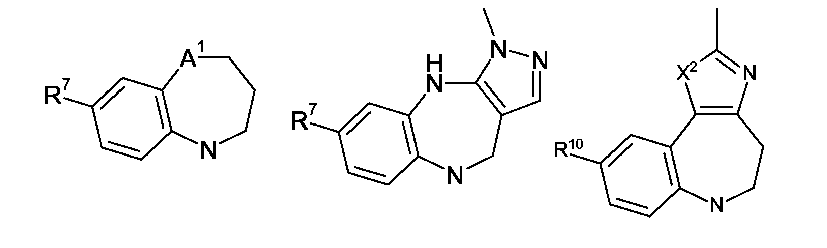

3. The compound according to claim 2, wherein G¹ is selected among general formula **6, 7, 8, 9, 10, 11, 12, 13, 14, 15, 16** and **17:**

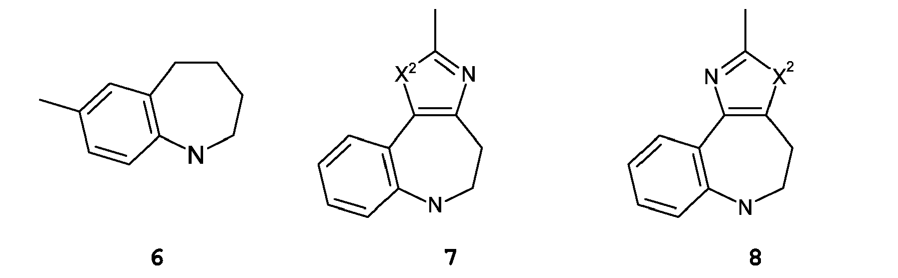

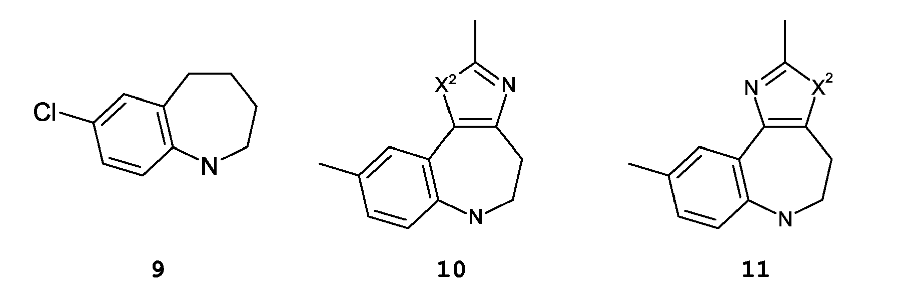

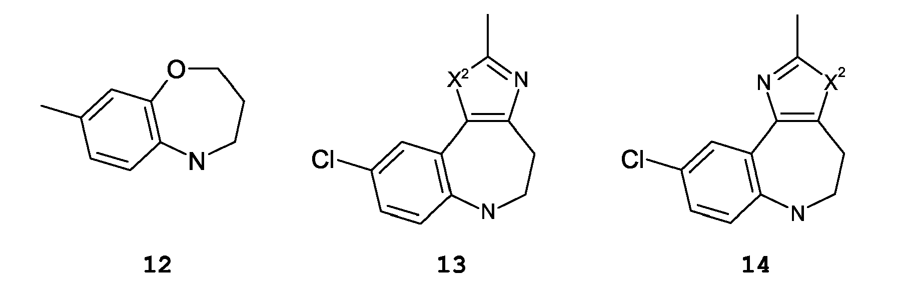

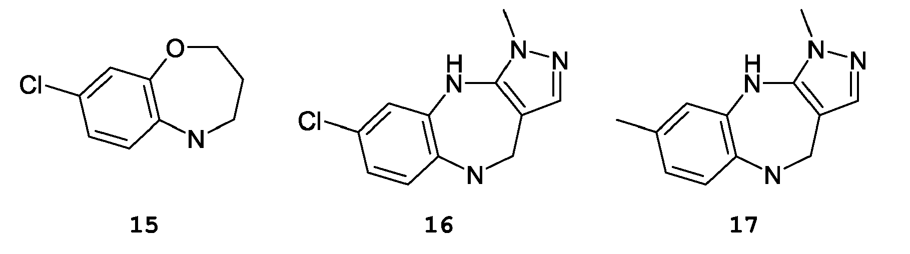

4. The compound according to any of the preceding claims, wherein at least one of R¹, R² and R³ is other than hydrogen.

5. The compound according to claim 4, wherein one of R¹, R² and R³ is selected among methyl, chlorine and fluorine, and the others are hydrogen.

6. The compound according to any of the preceding claims, wherein X¹ is NH.

7. The compound according to any of the preceding claims, wherein a is 1 and b is 2.

8. The compound according to any of the preceding claims, wherein R⁴ is alkyl.

9. The compound according to claim 1 having the general formula **18;** wherein R¹ is selected among methyl, chlorine and fluorine; and R⁴ is alkyl.

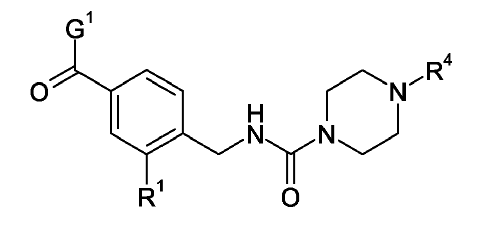

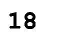

10. The compound according to claim 3 having the general formula **19;** wherein R¹ is selected among methyl, chlorine and fluorine:

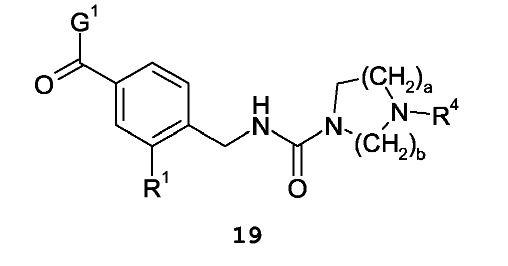

11. The compound according to claim 3 having the general formula **20;** wherein R⁴ is alkyl:

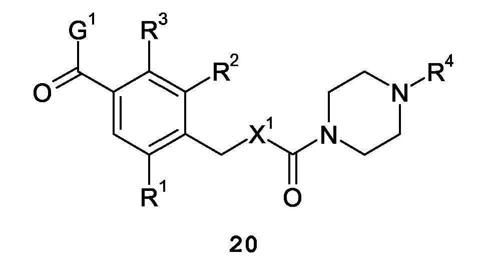

12. The compound according to claim 3 having the general formula **21;** wherein R¹ is selected among methyl, chlorine and fluorine; and R⁴ is alkyl:

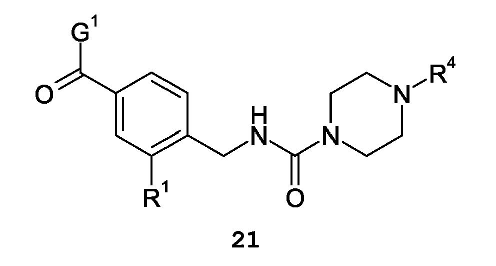

13. The compound according to claim 1 selected among
4-(2,2-Dimethyl-propyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(2,2-Dimethyl-propyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 2-chloro-4-(3,6-dimethyl-4,10-dihydro-3H-2,3,4,9-tetraaza-benzo[f]azulene-9-carbonyl)-benzylamide;
4-(3,3-Dimethyl-butyl)-piperazine-1-carboxylic acid 2-chloro-4- (6-chloro-3-methyl-4, 10-dihydro-3H-2,3,4,9-tetraaza-benzo[f]azulene-9-carbonyl)-benzylamide;
4-(3,3-Dimethyl-butyl)-piperazine-1-carboxylic acid 2-methyl-4-(2-methyl-4,5-dihydro-1H-1,3,6-triaza-benzo[e]azulene-6-carbonyl)-benzylamide;
4-(3,3-Dimethyl-butyl)-piperazine-1-carboxylic acid 2-methyl-4-(2-methyl-4,5-dihydro-1-oxa-3,6-diaza-benzo[e]azulene-6-carbonyl)-benzylamide;
4-(3,3-Dimethyl-butyl)-piperazine-1-carboxylic acid 2-methyl-4- (3-methyl-7, 8-dihydro-6H-5-oxa-9-aza-benzocycloheptene-9-carbonyl)-benzylamide;
4-(3,3-Dimethyl-butyl)-piperazine-1-carboxylic acid 2-methyl-4-(8-methyl-3,4-dihydro-2H-quinoline-1-carbonyl)-benzylamide;
4-(3,3-Dimethyl-butyl)-piperazine-1-carboxylic acid 3-chloro-4-(3,6-dimethyl-4,10-dihydro-3H-2,3,4,9-tetraaza-benzo[f]azulene-9-carbonyl)-benzylamide;
4-(3,3-Dimethyl-butyl)-piperazine-1-carboxylic acid 3-chloro-4-(6-chloro-3-methyl-4,10-dihydro-3H-2,3,4,9-tetraaza-benzo[f]azulene-9-carbonyl)-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-fluoro-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-3-fluoro-benzylamide;
4-(3,3-Dimethyl-butyl)-piperazine-1-carboxylic acid 4-(3,6-dimethyl-4,10-dihydro-3H-2,3,4,9-tetraaza-benzo[f]azulene-9-carbonyl)-3-methyl-benzylamide;
4-(3,3-Dimethyl-butyl)-piperazine-1-carboxylic acid 4-(3-chloro-7,8-dihydro-6H-5-oxa-9-aza-benzocycloheptene-9-carbonyl)-2-methyl-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-2-fluoro-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-3-fluoro-benzylamide;
4-(3,3-Dimethyl-butyl)-piperazine-1-carboxylic acid 4-(6-chloro-3-methyl-4,10-dihydro-3H-2,3,4,9-tetraaza-benzo[f]azulene-9-carbonyl)-3-methyl-benzylamide;
4-(3-Methyl-butyl)-piperazine-1-carboxylic acid 4-(3,6-dimethyl-4,10-dihydro-3H-2,3,4,9-tetraaza-benzo[f]azulene-9-carbonyl)-2-methyl-benzylamide;
4-(3-Methyl-butyl)-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopentylmethyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Cyclopentylmethyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 2-chloro-4-(3,6-dimethyl-4,10-dihydro-3H-2,3,4,9-tetraaza-benzo[f]azulene-9-carbonyl)-benzylamide;
4-Cyclopropylmethyl-piperazine-1-carboxylic acid 2-chloro-4-(6-chloro-3-methyl-4,10-dihydro-3H-2,3,4,9-tetraaza-benzo[f]azulene-9-carbonyl)-benzylamide;
4-Cyclopropylmethyl-piperazine-1-carboxylic acid 3-chloro-4-(3,6-dimethyl-4,10-dihydro-3H-2,3,4,9-tetraaza-benzo[f]azulene-9-carbonyl)-benzylamide;
4-Cyclopropylmethyl-piperazine-1-carboxylic acid 3-chloro-4-(6-chloro-3-methyl-4,10-dihydro-3H-2,3,4,9-tetraaza-benzo[f]azulene-9-carbonyl)-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-2-fluoro-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 4-(3,6-dimethyl-4,10-dihydro-3H-2,3,4,9-tetraaza-benzo[f]azulene-9-carbonyl)-3-fluoro-benzylamide;
4-Cyclopropylmethyl-piperazine-l-carboxylic acid 4-(3,6-dimethyl-4,10-dihydro-3H-2,3,4,9-tetraaza-benzo[f]azulene-9-carbonyl)-3-methyl-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-2-fluoro-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Cyclopropylmethyl-piperazine-1-carboxylic acid 4-(6-chloro-3-methyl-4,10-dihydro-3H-2,3,4,9-tetraaza-benzo[f]azulene-9-carbonyl)-3-fluoro-benzylamide;
4-Cyclopropylmethyl-piperazine-l-carboxylic acid 4-(6-chloro-3-methyl-4,10-dihydro-3H-2,3,4,9-tetraaza-benzo[f]azulene-9-carbonyl)-3-methyl-benzylamide;
4-Isobutyl-piperazine-1-carboxylic acid 4-(3,6-dimethyl-4,10-dihydro-3H-2,3,4,9-tetraaza-benzo[f]azulene-9-carbonyl)-2-methyl-benzylamide;
4-Isobutyl-piperazine-1-carboxylic acid 4-(6-chloro-3-methyl-4,10-dihydro-3H-2,3,4,9-tetraaza-benzo[f]azulene-9-carbonyl)-2-methyl-benzylamide;
4-Propyl-piperazine-l-carboxylic acid 4-(3,6-dimethyl-4,10-dihydro-3H-2,3,4,9-tetraaza-benzo[f]azulene-9-carbonyl)-2-methyl-benzylamide;
4-Propyl-piperazine-1-carboxylic acid 4-(6-chloro-3-methyl-4,10-dihydro-3H-2,3,4,9-tetraaza-benzo[f]azulene-9-carbonyl)-2-methyl-benzylamide; and
4-(3,3-Dimethyl-butyl)-piperazine-1-carboxylic acid 2-methyl-4-(2-methyl-4,5-dihydro-3-thia-1,6-diaza-benzo[e]azulene-6-carbonyl)-benzylamide.

14. A compound according to general formula **1b,** or a compound which is a tautomer or a pharmaceutically acceptable salt thereof, 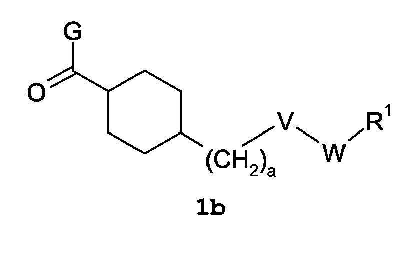
wherein G is a group selected among general formula **2, 3, 4** and **5**; 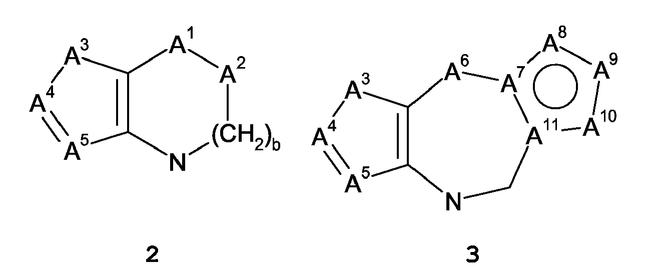 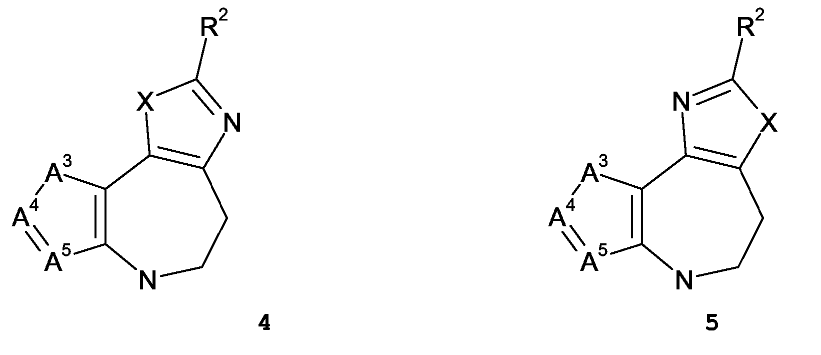
and wherein
A¹ is selected among CH₂, CH(OH), NH, N-alkyl, O and S;
A² is selected among CH₂, CH(OH), C(=O) and NH;
A³ is selected among S, NH, N-alkyl, -C(R³)=CH-, -C(R³)=N-, -N=C(R³)- and -CH=C(R³)-;
A⁴ is selected among C (R⁴) and N;
A⁵ is selected among C(R⁵) and N;
A⁶ is selected among CH₂, NH, N-alkyl and O;
A⁷ and A¹¹ are independently selected among C and N;
A⁸ and A⁹ are independently selected among CH, N, NH, N(CH₂)_{c}R⁶ and S;
A¹⁰ is selected among -CH=CH-, CH, N, NH, N-(CH₂)_{c}-R⁶ and S;
wherein the ring constituted by A⁷, A⁸, A⁹, A¹⁰ and A¹¹ is aromatic, and
R¹ is selected among H, alkyl, optionally substituted phenyl, optionally substituted pyridyl, optionally substituted thienyl, optionally substituted furyl, and -(CH₂) _{d}-R⁷, 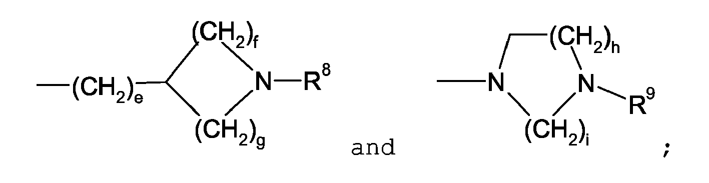
R² is selected among H, alkyl, optionally substituted phenyl, optionally substituted pyridyl, optionally substituted thienyl, optionally substituted furyl, and―(CH₂)ⱼ―R¹⁰;
R³, R⁴ and R⁵ are independently selected among H, alkyl, alkoxy, F, Cl, Br, CN, NH₂, NO₂, NH(alkyl), and N (alkyl)₂;
R⁶ and R⁷ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl CONH₂, CONH-alkyl, CON(alkyl)₂, CN and CF₃;
R⁸ and R⁹ are independently selected among H, alkyl, optionally substituted phenyl, optionally substituted pyridyl, optionally substituted thienyl, optionally substituted furyl, - (CH₂)ₖ-R¹¹ and Y-R¹²;
R¹⁰ and R¹¹ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H CO₂-alkyl CONH₂, CONH-alkyl, CON(alkyl)₂, CN and CF₃;
R¹² is selected among H, alkyl, optionally substituted phenyl, optionally substituted pyridyl, optionally substituted thienyl, optionally substituted furyl and
-(CH₂)₁-R¹³_{;}
R¹³ is selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
V is selected among O, CH₂ and NH;
W is selected among C(=O) and S(=O)ₘ;
X is selected among O, S, NH and N-alkyl;
Y is selected among -C(=O) and -S(=O)ₙ;
a is selected among 0, 1 and 2;
b is selected among 1 and 2;
c and d are independently selected among 1, 2 and 3; e is selected among 0, 1 and 2;
f, g, h and i are independently selected among 1 and 2;
j, k and l are independently selected among 1, 2 and 3;
m and n are independently selected among 1 and 2.

15. The compound according to claim 14, wherein G is selected among:

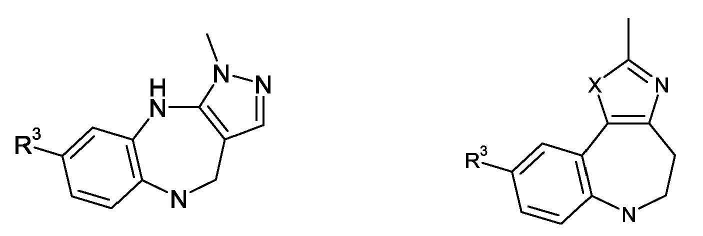

16. The compound according to claim 14, wherein G is selected among:

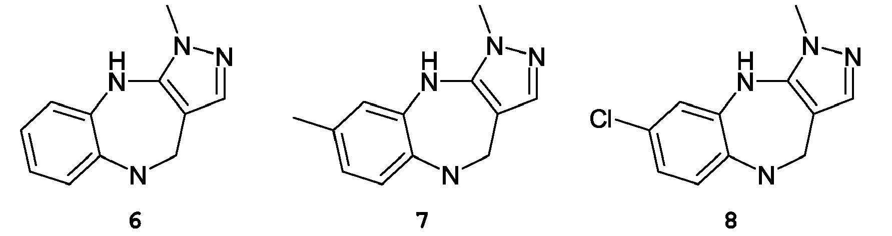

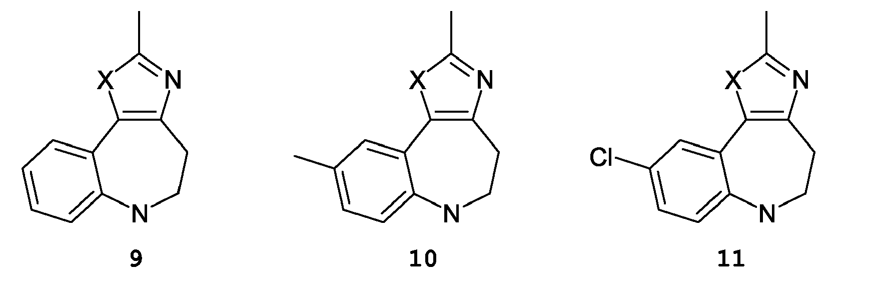

17. The compound according to claim 14, wherein R⁸ is alkyl.

18. The compound according to claim 14, wherein R⁹ is alkyl.

19. The compound according to claim 14, wherein R¹ is selected among:

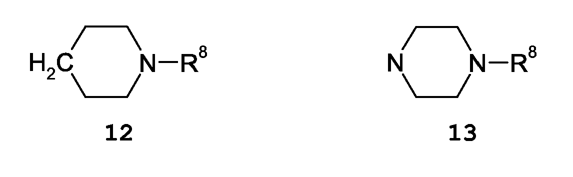

20. The compound according to claim 19, wherein R¹ is selected among:

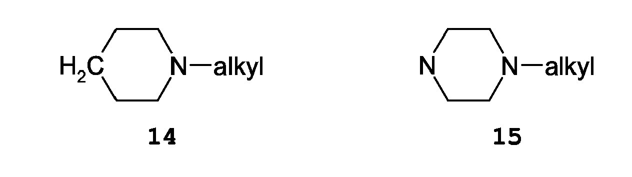

21. The compound according to claim 14, wherein V is NH.

22. The compound according to claim 14, wherein W is C(=O).

23. The compound according to claim 14, wherein a is 1.

24. The compound according to claim 14, wherein V is NH, W is C(=0), a is 1, and R¹ is selected among general formulae **12** to **15,** preferably R¹ is selected among **14** and **15**; as shown in formula **16**:

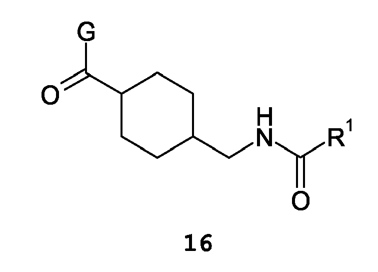

25. The compound according to claim 14, wherein V is NH, W is C(=O) and a is 1; as shown in formula **17**: 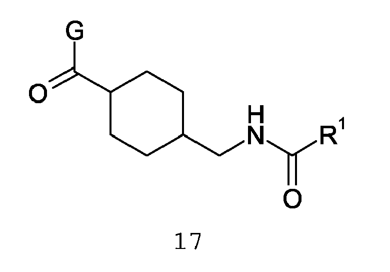 wherein G is selected from general formulae **6** to **11**.

26. The compound according to claim 14, as shown in formula **18**: 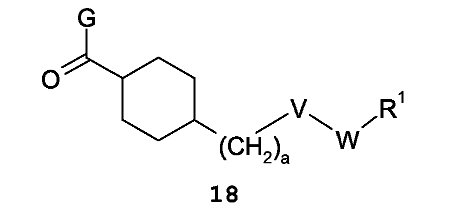
wherein G is selected among general formulae **6** to 11 and R¹ is selected among general formulae **12** to **15;** preferably R¹ is selected among **14** and **15.**

27. The compound according to claim 14, as shown in formula **19,** wherein V is NH, W is C(=O) and a is 1: 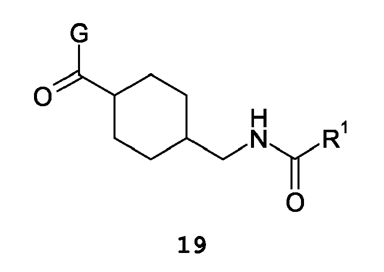 and wherein G is selected among general formulae **6** to **11** and R¹ is selected among general formulae **12** to **15;** preferably R¹ is selected among **14** and **15.**

28. The compound according to claim 14, selected among the group consisting of:
1-(3,3-Dimethyl-butyl)-piperidine-4-carboxylic acid [4-(3,6-dimethyl-4,10-dihydro-3H-2,3,4,9-tetraaza-benzo[f]azulene-9-carbonyl)-cyclohexylmethyl]-amide;
1-(3,3-Dimethyl-butyl)-piperidine-4-carboxylic acid [4-(3-methyl-4,10-dihydro-3H-2,3,4,9-tetraaza-benzo[f]azulene-9-carbonyl)-cyclohexylmethyl]-amide;
1-(3,3-Dimethyl-butyl)-piperidine-4-carboxylic acid [4-(6-chloro-3-methyl-4,10-dihydro-3H-2,3,4,9-tetraaza-benzo[f]azulene-9-carbonyl)-cyclohexylmethyl]-amide;
1-Cyclopropylmethyl-piperidine-4-carboxylic acid [4-(3,6-dimethyl-4,10-dihydro-3H-2,3,4,9-tetraaza-benzo[f]azulene-9-carbonyl)-cyclohexylmethyl]-amide;
1-Cyclopropylmethyl-piperidine-4-carboxylic acid [4-(3-methyl-4,10-dihydro-3H-2,3,4,9-tetraaza-benzo[f]azulene-9-carbonyl)-cyclohexylmethyl]-amide;
1-Cyclopropylmethyl-piperidine-4-carboxylic acid [4-(6-chloro-3-methyl-4,10-dihydro-3H-2,3,4,9-tetraaza-benzo[f]azulene-9-carbonyl)-cyclohexylmethyl]-amide;
4-(2,2-Dimethyl-propyl)-piperazine-1-carboxylic acid [4-(3,6-dimethyl-4,10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(3,6-dimethyl-4,10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(3-methyl-4,10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
4-(3-Methyl-butyl)-piperazine-1-carboxylic acid [4-(3,6-dimethyl-4,10-dihydro-3H-2,3,4,9-tetraaza-benzo[f]azulene-9-carbonyl)-cyclohexylmethyl]amide;
4-(3-Methyl-butyl)-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
4-Cyclopentylmethyl-piperazine-1-carboxylic acid [4-(3,6-dimethyl-4,10-dihydro-3H-2,3,4,9-tetraaza-benzo[f]azulene-9-carbonyl)-cyclohexylmethyl]-amide;
4-Cyclopentylmethyl-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
4-Cyclopropylmethyl-piperazine-1-carboxylic acid [4-(3,6-dimethyl-4,10-dihydro-3H-2,3,4,9-tetraaza-benzo[f]azulene-9-carbonyl)-cyclohexylmethyl]amide;
4-Cyclopropylmethyl-piperazine-1-carboxylic acid [4-(3-methyl-4,10-dihydro-3H-2,3,4,9-tetraaza-benzo[f]azulene-9-carbonyl)-cyclohexylmethyl]-amide;
4-Cyclopropylmethyl-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
4-Isobutyl-piperazine-1-carboxylic acid [4-(3,6-dimethyl-4,10-dihydro-3H-2,3,4,9-tetraaza-benzo[f]azulene-9-carbonyl)-cyclohexylmethyl]-amide;
4-Isobutyl-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
4-Propyl-piperazine-l-carboxylic acid [4-(3,6-dimethyl-4,10-dihydro-3H-2,3,4,9-tetraaza-benzo[f]azulene-9-carbonyl)-cyclohexylmethyl]-amide;
4-Propyl-piperazine-1-carboxylic acid [4-(6-chloro-3-methyl-4,10-dihydro-3H-2,3,4,9-tetraaza-benzo[f]azulene-9-carbonyl)-cyclohexylmethyl]-amide;
Cyclopropanecarboxylic acid [4-(3,6-dimethyl-4,10-dihydro-3H-2,3,4,9-tetraaza-benzo[f]azulene-9-carbonyl)-cyclohexylmethyl]-amide;
Cyclopropanecarboxylic acid [4-(3-methyl-4,10-dihydro-3H-2,3,4,9-tetraaza-benzo[f]azulene-9-carbonyl)-cyclohexylmethyl]-amide; and
Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide.

29. A compound according to general formula **1c**, or a compound which is a tautomer or a pharmaceutically acceptable salt thereof, 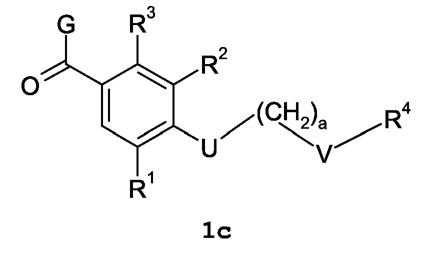 G is a group selected among general formula **2, 3, 4, 5,** and **6.** 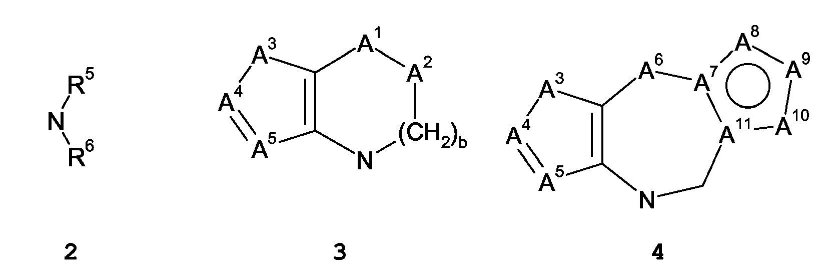 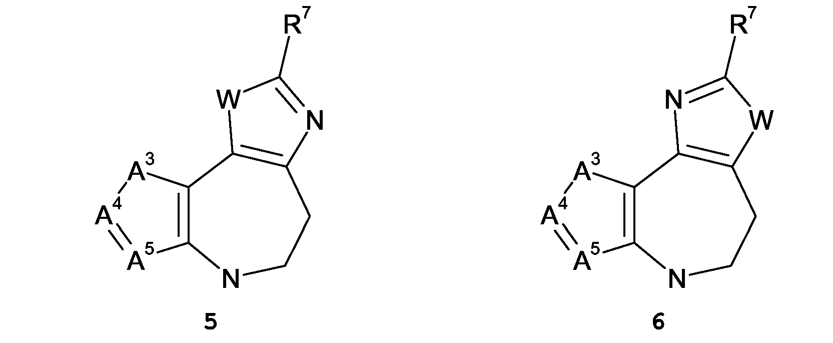
wherein:
A¹ is selected among CH₂, CH(OH), NH, N-alkyl, O and S;
A² is selected among CH₂, CH(OH), C(=O) and NH;
A³ is selected among S, NH, N-alkyl, -C(R⁸) =CH-, -C(R⁸)=N-, -N=C(R⁸)- and -CH=C(R⁸)-;
A⁴ is selected among C(R⁹) and N;
A⁵ is selected among C(R¹⁰) and N;
A⁶ is selected among CH₂, NH, N-alkyl and 0;
A⁷ and A¹¹ are independently selected among C and N;
A⁸ and A⁹ are independently selected among CH, N, NH, N (CH₂)_{c}R¹¹ and S;
A¹⁰ is selected among -CH=CH-, CH, N, NH, N-(CH₂)_{c}-R¹¹ and S;
wherein the ring constituted by A⁷, A⁸, A⁹, A¹⁰ and A¹¹ is aromatic,
R¹, R² and R³ are independently selected among H, alkyl, O-alkyl, NO₂, F, Cl and Br;
R⁴ is selected among 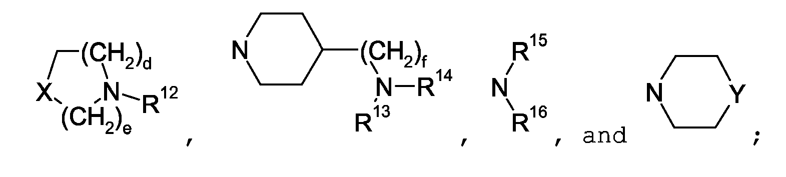
R⁵ and R⁶ are independently selected among alkyl, Ar and -(CH₂)g-Ar, wherein Ar is selected among optionally substituted phenyl and optionally substituted thienyl;
R⁷ is selected among H, alkyl, optionally substituted phenyl, optionally substituted pyridyl, optionally substituted thienyl, optionally substituted furyl, and -(CH₂)ₕ-R¹⁷;
R⁸, R⁹ and R¹⁰ are independently selected among H, alkyl, alkoxy, F, Cl, Br, CN, NH₂, NO₂, NH(alkyl), and N(alkyl)₂;
R¹¹ is selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R¹² is selected among H, alkyl, optionally substituted phenyl, optionally substituted pyridyl, optionally substituted thienyl, optionally substituted furyl, -(CH₂)ᵢ-R¹⁸, and Z-R¹⁹;
R¹³ and R¹⁴ are independently selected among H, alkyl, optionally substituted phenyl, optionally substituted pyridyl, optionally substituted thienyl, optionally substituted furyl, and -(CH₂)ⱼ-R²⁰;
R¹⁵ and R¹⁶ are independently selected among H, alkyl, optionally substituted phenyl, optionally substituted pyridyl, optionally substituted thienyl, optionally substituted furyl, and -(CH₂)ₖ-R²¹;
R¹⁷ and R¹⁸ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R¹⁹ is selected among H, alkyl, optionally substituted phenyl, optionally substituted pyridyl, optionally substituted thienyl, optionally substituted furyl, and -(CH₂)ₗ-R²²;
R²⁰, R²¹ and R²² are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
U is selected among O and CH₂;
V is selected among CH₂, C(=O) and S(=O)ₘ;
W is selected among O, S, NH and N-alkyl ;
X is selected among N and CH;
Y is selected among O and S;
Z is selected among C=O and S(=O)ₙ;
a is selected among 0, 1, 2, and 3;
b is selected among 1 and 2;
c is selected among 1, 2 and 3;
d and e are independently selected among 1 and 2;
f is selected among 0 and 1;
g is selected among 1 and 2;
h, i, j, k and l are independently selected among 1, 2 and 3;
m and n are independently selected among 1 and 2.

30. The compound according to claim 29, wherein G is selected among:

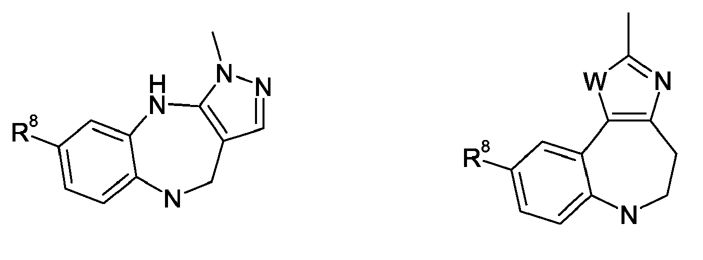

31. The compound according to claim 29 selected among:

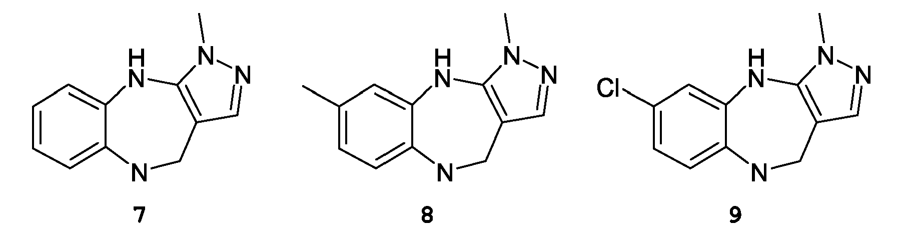

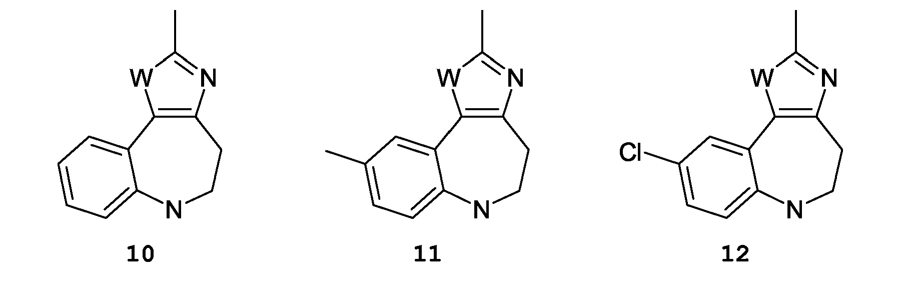

32. The compound according to claim 29, wherein at least one of R¹, R² and R³ is other than hydrogen; preferably one of R¹, R² and R³ is methyl, chlorine or fluorine and the others are hydrogen.

33. The compound according to claim 29, wherein R¹² is alkyl.

34. The compound according to claim 29, wherein R⁴ is selected among:

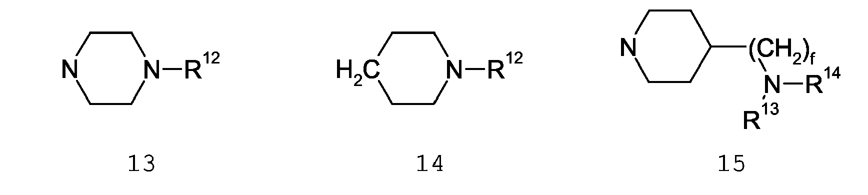

35. The compound according to claim 29, wherein R⁴ is selected among:

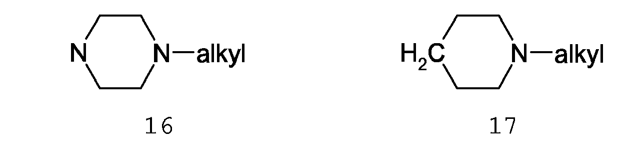

36. The compound according to claim 29, wherein U is O, V is C(=O), and a is 1.

37. The compound according to claim 29, wherein U is CH2, V is C(=O), and a is selected among 1 and 2.

38. The compound according to claim 29, wherein U is O, V is CH2, and a is selected among 1 and 2.

39. The compound according to claim 29, wherein R² and R³ are both H, U is O, V is C(=0) and a is 1, as shown in formula **18**: 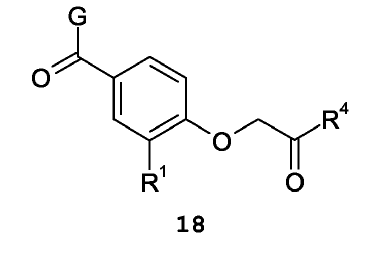
wherein R¹ is selected among methyl, chlorine and fluorine, and R⁴ is selected among general formulae **13, 15** and **16;** preferably R⁴ is **16.**

40. The compound according to claim 29, wherein R² and R³ are both H, U is CH₂, V is C(=O) and a is 1, as shown in formula **19**: 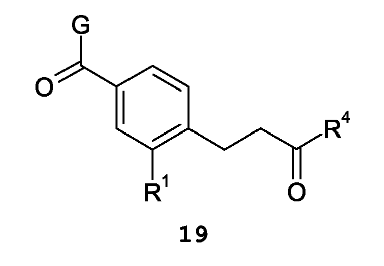
wherein R¹ is selected among methyl, chlorine and fluorine; preferably R¹ is methyl; R⁴ is selected among general formulae **13, 15** and **16;** preferably R⁴ is **16**.

41. The compound according to claim 29, wherein R² and R³ are both H, U is O and V is CH₂; as shown in formula **20**: 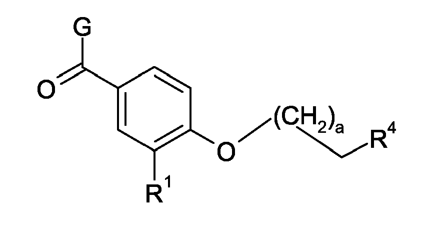 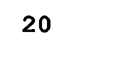
wherein R¹ is selected among methyl, chlorine and fluorine, and R⁴ is selected among general formulae **13** to **17;** preferably R⁴ is selected among general formulae **16** and **17**.

42. The compound according to claim 29, wherein R² and R³ are both H, U is O, V is CH₂ and a is 2, as shown in formula **21**: 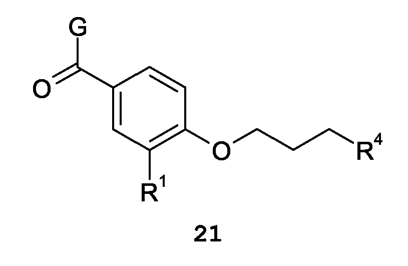
wherein R¹ is selected among methyl, chlorine and fluorine, and R⁴ is selected among general formulae **13** to **17;** preferably R⁴ is selected among **16** and **17.**

43. The compound according to claim 29, wherein U is O and V is CH₂, as shown in formula **22:** 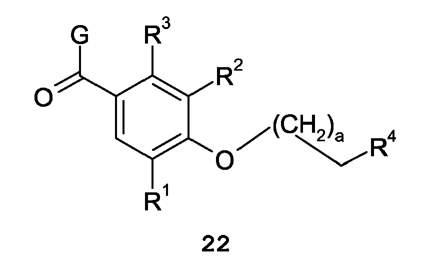
wherein G is selected among general formulae 7 to **12;** preferably R⁴ is selected among general formulae **13** to **17,** more preferably R⁴ is selected among **16** and **17**.

44. The compound according to claim 29, wherein U is O, V is CH₂ and a is 2, as shown in formula **23:** 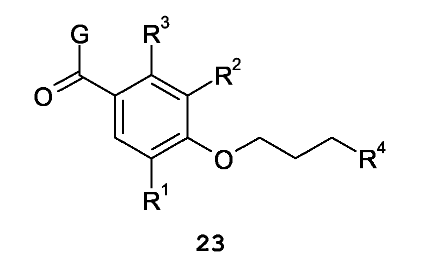
wherein G is selected among general formulae **7** to **12;** preferably R⁴ is selected among general formulae **13** to **17,** more preferably R⁴ is selected among **16** to **17**.

45. The compound according to claim 29, wherein U is CH₂, V is C(=O) and a is 1, as shown in formula **24:** 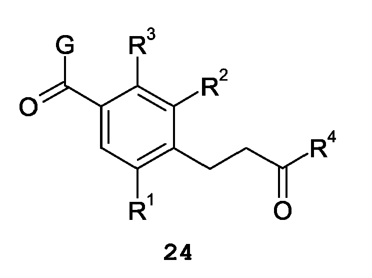
wherein G is selected among general formulae 7 to **12;** preferably R⁴ is selected among general formulae **13, 15** and **16;** more preferably R⁴ is **16.**

46. The compound according to claim 29, wherein U is O, V is C(=O) and a is 1, as shown in formula **25:** 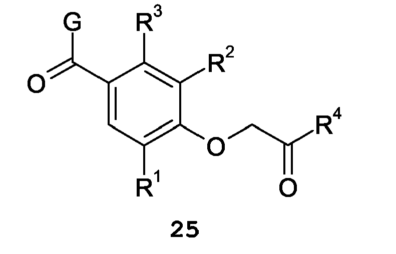
wherein G is selected among general formulae **7** to **12;** preferably R⁴ is selected among general formulae **13, 15** and **16;** more preferably R⁴ is **16.**

47. The compound according to claim 29, wherein R² and R³ are both H, as shown in formula **26:** 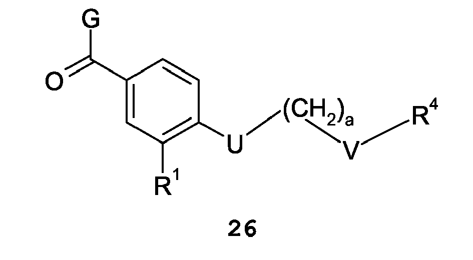
wherein R¹ is selected among methyl, chlorine and fluorine, and G is selected among general formulae **7** to **12.**

48. The compound according to claim 29, wherein R² and R³ are both H, U is O and V is CH₂, as shown in formula **27:** 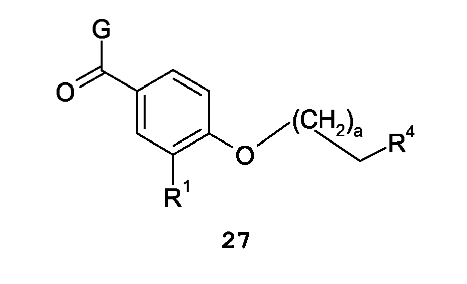
wherein R¹ is selected among methyl, chlorine and fluorine, and G is selected among general formulae **7** to **12;** preferably R⁴ is selected among general formulae **13** to **17;** more preferably R⁴ is selected among **16** and **17.**

49. The compound according to claim 29, wherein R² and R³ are both H, U is O, V is CH₂ and a is 2, as shown in formula **28:** 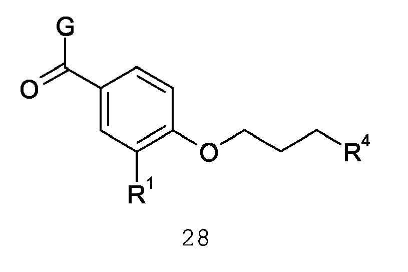
wherein R¹ is selected among methyl, chlorine and fluorine, and G is selected among general formulae **7** to **12;** preferably R⁴ is selected among general formulae **13** to **17,** more preferably R⁴ is selected among **16** and **17.**

50. The compound according to claim 29, wherein R² and R³ are both H, U is CH₂, V is C (=O) and a is 1, as shown in formula **29:** 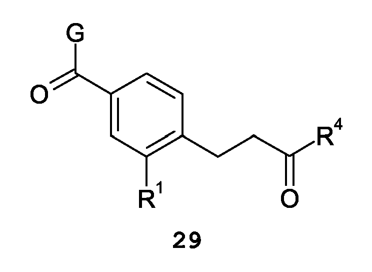
wherein R¹ is selected among methyl, chlorine and fluorine; preferably R¹ is methyl; G is selected among general formulae **7** to **12;** preferably R⁴ is selected among general formulae **13, 15** and **16;** more preferably R⁴ is **16.**

51. The compound according to claim 29, wherein R² and R³ are both H, U is O, V is C(=O) and a is 1, as shown in formula **30:** 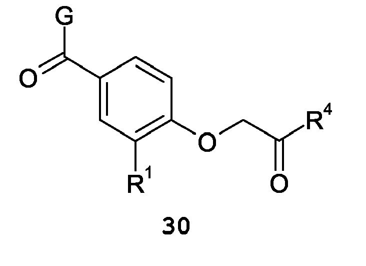
wherein R¹ is selected among methyl, chlorine and fluorine, and G is selected among general formulae **7** to **12;** preferably R⁴ is selected among general formulae **13, 15** and **16;** more preferred R⁴ is **16.**

52. The compound according to claim 29, selected among the group consisting of:
(2-Chloro-4-{3-[4-(3,3-dimethyl-butyl)-piperazin-1-yl]-propoxy}-phenyl)-(3-methyl-4,10-dihydro-3H-2,3,4,9-tetraaza-benzo[f]azulen-9-yl)-methanone;
(2-Chloro-4-{3-[4-(3,3-dimethyl-butyl)-piperazin-1-yl]-propoxy}-phenyl)-(3,6-dimethyl-4,10-dihydro-3H-2,3,4,9-tetraaza-benzo[f]azulen-9-yl)-methanone;
(2-Chloro-4-{3-[4-(3,3-dimethyl-butyl)-piperazin-1-yl]-propoxy}-phenyl)-(3,6-dimethyl-4,10-dihydro-3H-2,3,4,9-tetraaza-benzo[f]azulen-9-yl)-methanone;
(2-Chloro-4-{3-[4-(3,3-dimethyl-butyl)-piperazin-1-yl]-propoxy}-phenyl)-(6-chloro-3-methyl-4,10-dihydro-3H-2,3,4,9-tetraaza-benzo[f]azulen-9-yl)-methanone;
(3,6-Dimethyl-4,10-dihydro-3H-2,3,4,9-tetraaza-benzo[f]azulen-9-yl)-{3-methyl-4-[3-(4-propyl-piperazin-1-yl)-propoxy]-phenyl}-methanone;
(3,6-Dimethyl-4,10-dihydro-3H-2,3,4,9-tetraaza-benzo[f]azulen-9-yl)-{4-[3-(4-isobutyl-piperazin-1-yl)-propoxy]-3-methyl-phenyl}-methanone;
(3,6-Dimethyl-4,10-dihydro-3H-2,3,4,9-tetraaza-benzo[f]azulen-9-yl)-(4-{3-[4-(2,2-dimethyl-propyl)-piperazin-1-yl]-propoxy}-3-methyl-phenyl)-methanone;
(3,6-Dimethyl-4,10-dihydro-3H-2,3,4,9-tetraaza-benzo[f]azulen-9-yl)-(3-methyl-4-{3-[4-(3-methyl-butyl)-piperazin-1-yl]-propoxy}-phenyl)-methanone;
(3-Chloro-4-{3-[4-(3,3-dimethyl-butyl)-piperazin-1-yl]-propoxy}-phenyl)-(3-methyl-4,10-dihydro-3H-2,3,4,9-tetraaza-benzo[f]azulen-9-yl)-methanone;
(3-Chloro-4-{3-[4-(3,3-dimethyl-butyl)-piperazin-1-yl]-propoxy}-phenyl)-(3,6-dimethyl-4,10-dihydro-3H-2,3,4,9-tetraaza-benzo[f]azulen-9-yl)-methanone;
(3-Chloro-4-{3-[4-(3,3-dimethyl-butyl)-piperazin-1-yl]-propoxy}-phenyl)-(6-chloro-3-methyl-4,10-dihydro-3H-2,3,4,9-tetraaza-benzo[f]azulen-9-yl)-methanone;
(3-Fluoro-4-{3-[4-(3-methyl-butyl)-piperazin-1-yl]-propoxy}-phenyl)-(3-methyl-4,10-dihydro-3H-2,3,4,9-tetraaza-benzo[f]azulen-9-yl)-methanone;
(4-{2-[4-(3,3-Dimethyl-butyl)-piperazin-1-yl]-ethoxy}-3-methyl-phenyl)-(3-methyl-4,10-dihydro-3H-2,3,4,9-tetraaza-benzo[f]azulen-9-yl)-methanone;
(4-{3-[4-(2,2-Dimethyl-propyl)-piperazin-1-yl]-propoxy}-3-fluoro-phenyl)-(3-methyl-4,10-dihydro-3H-2,3,4,9-tetraaza-benzo[f]azulen-9-yl)-methanone;
(4-{3-[4-(2-Ethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone;
(4-{3-[4-(3,3-Dimethyl-butyl)-piperazin-1-yl]-propoxy}-2-fluoro-phenyl)-(3-methyl-4,10-dihydro-3H-2,3,4,9-tetraaza-benzo[f]azulen-9-yl)-methanone;
(4-{3-[4-(3,3-Dimethyl-butyl)-piperazin-1-yl]-propoxy}-3-fluoro-phenyl)-(3-methyl-4,10-dihydro-3H-2,3,4,9-tetraaza-benzo[f]azulen-9-yl)-methanone;
(4-{3-[4-(3,3-Dimethyl-butyl)-piperazin-1-yl]-propoxy}-3-fluoro-phenyl)-(3,6-dimethyl-4,10-dihydro-3H-2,3,4,9-tetraaza-benzo[f]azulen-9-yl)-methanone;
(4-{3-[4-(3,3-Dimethyl-butyl)-piperazin-1-yl]-propoxy}-3-methyl-phenyl)-(3-methyl-4,10-dihydro-3H-2,3,4,9-tetraaza-benzo[f]azulen-9-yl)-methanone;
(4-{3-[4-(3,3-Dimethyl-butyl)-piperazin-1-yl]-propoxy}-3-methyl-phenyl)-(3,6-dimethyl-4,10-dihydro-3H-2,3,4,9-tetraaza-benzo[f]azulen-9-yl)-methanone;
(6-Chloro-3-methyl-4,10-dihydro-3H-2,3,4,9-tetraaza-benzo[f]azulen-9-yl)-(4-{3-[4-(3,3-dimethyl-butyl)-piperazin-1-yl]-propoxy}-3-methyl-phenyl)-methanone;
(6-Chloro-3-methyl-4,10-dihydro-3H-2,3,4,9-tetraaza-benzo[f]azulen-9-yl)-(4-{3-[4-(3,3-dimethyl-butyl)-piperazin-1-yl]-propoxy}-3-fluoro-phenyl)-methanone;
(6-Chloro-3-methyl-4,10-dihydro-3H-2,3,4,9-tetraaza-benzo[f]azulen-9-yl)-(4-{3-[4-(3,3-dimethyl-butyl)-piperazin-1-yl]-propoxy}-2-fluoro-phenyl)-methanone;
{2-Chloro-4-[3-(4-isobutyl-piperazin-1-yl)-propoxy]-phenyl}-(3-methyl-4,10-dihydro-3H-2,3,4,9-tetraaza-benzo[f]azulen-9-yl)-methanone;
{2-Chloro-4-[3-(4-propyl-piperazin-1-yl)-propoxy]-phenyl}-(3-methyl-4,10-dihydro-3H-2,3,4,9-tetraaza-benzo[f]azulen-9-yl)-methanone;
{3-Fluoro-4-[3-(4-isobutyl-piperazin-1-yl)-propoxy]-phenyl}-(3-methyl-4,10-dihydro-3H-2,3,4,9-tetraaza-benzo[f]azulen-9-yl)-methanone;
{3-Fluoro-4-[3-(4-propyl-piperazin-1-yl)-propoxy]-phenyl}-(3-methyl-4,10-dihydro-3H-2,3,4,9-tetraaza-benzo[f]azulen-9-yl)-methanone;
{4-[3-(4-Cyclopentylmethyl-piperazin-1-yl)-propoxy]-3-fluoro-phenyl}-(3-methyl-4,10-dihydro-3H-2,3,4,9-tetraaza-benzo[f]azulen-9-yl)-methanone;
{4-[3-(4-Cyclopropylmethyl-piperazin-1-yl)-propoxy]-3-fluoro-phenyl}-(3-methyl-4,10-dihydro-3H-2,3,4,9-tetraaza-benzo[f]azulen-9-yl)-methanone;
{4-[3-(4-Cyclopropylmethyl-piperazin-1-yl)-propoxy]-3-methyl-phenyl}-(3-methyl-4,10-dihydro-3H-2,3,4,9-tetraaza-benzo[f]azulen-9-yl)-methanone;
{4-[3-(4-Cyclopropyl-piperazin-1-yl)-propoxy]-3-fluorophenyl}-(3-methyl-4,10-dihydro-3H-2,3,4,9-tetraaza-benzo[f]azulen-9-yl)-methanone;
1-(4-Benzylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclobutylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclobutylmethyl-piperazin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclobutylmethyl-piperazin-1-yl)-4-[2-methyl-4-(3-methyl-4,10-dihydro-3H-2,3,4,9-tetraaza-benzo[f]azulene-9-carbonyl)-phenyl]-butan-1-one;
1-(4-Cyclohexylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclopentylaminomethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclopentylaminomethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclopentylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclopropylaminomethyl-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Cyclopropylmethyl-piperazin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Hexylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-(4-Isobutylamino-piperidin-1-yl)-3-[2-methyl-4-(3-methyl-4,10-dihydro-3H-2,3,4,9-tetraaza-benzo[f]azulene-9-carbonyl)-phenyl]-propan-1-one;
1-[4-(1-Benzyl-piperidin-4-ylamino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(2-Hydroxy-ethylamino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(3,3-Dimethyl-butyl)-piperazin-1-yl]-2-[2-methyl-4-(3-methyl-4,10-dihydro-3H-2,3,4,9-tetraaza-benzo[f]azulene-9-carbonyl)-phenoxy]-ethanone;
1-[4-(3,3-Dimethyl-butyl)-piperazin-1-yl]-2-[4-(3-methyl-4,10-dihydro-3H-2,3,4,9-tetraaza-benzo[f]azulene-9-carbonyl)-phenoxy]-ethanone;
1-[4-(3,3-Dimethyl-butyl)-piperazin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(3,3-Dimethyl-butyl)-piperazin-1-yl]-4-[2-methyl-4-(3-methyl-4,10-dihydro-3H-2,3,4,9-tetraaza-benzo[f]azulene-9-carbonyl)-phenyl]-butan-1-one;
1-[4-(3,3-Dimethyl-butyl)-piperazin-1-yl]-5-[2-methyl-4-(3-methyl-4,10-dihydro-3H-2,3,4,9-tetraaza-benzo[f]azulene-9-carbonyl)-phenyl]-pentan-1-one;
1-[4-(3,4-Dihydro-1H-isoquinolin-2-ylmethyl)-piperidin-1-yl]-3-[2-methyl-4-(3-methyl-4,10-dihydro-3H-2,3,4,9-tetraaza-benzo[f]azulene-9-carbonyl)-phenyl]-propan-l-one;
1-[4-(3-Hydroxy-propylamino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(3-Imidazol-1-yl-propylamino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(Benzyl-butyl-amino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(Cyclohexylmethyl-amino)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-[4-(Isobutylamino-methyl)-piperidin-1-yl]-3-[2-methyl-4-(3-methyl-4,10-dihydro-3H-2,3,4,9-tetraaza-benzo[f]azulene-9-carbonyl)-phenyl]-propan-1-one;
1-{4-[(1-Benzyl-piperidin-4-ylamino)-methyl]-piperidin-1-yl}-3-[2-methyl-4-(3-methyl-4,10-dihydro-3H-2,3,4,9-tetraaza-benzo[f]azulene-9-carbonyl)-phenyl]-propan-1-one;
1-{4-[(Butyl-methyl-amino)-methyl]-piperidin-1-yl}-3-[2-methyl-4-(3-methyl-4,10-dihydro-3H-2,3,4,9-tetraaza-benzo[f]azulene-9-carbonyl)-phenyl]-propan-1-one;
1-{4-[Benzyl-(2-dimethylamino-ethyl)-amino]-piperidin-1-yl}-3-[2-methyl-4-(3-methyl-4,10-dihydro-3H-2,3,4,9-tetraaza-benzo[f]azulene-9-carbonyl)-phenyl]-propan-1-one;
3-[2-Methyl-4-(3-methyl-4,10-dihydro-3H-2,3,4,9-tetraaza-benzo[f]azulene-9-carbonyl)-phenyl]-1-[4-(2-oxo-2-p-tolyl-ethyl)-piperazin-1-yl]-propan-1-one;
3-[2-Methyl-4-(3-methyl-4,10-dihydro-3H-2,3,4,9-tetraaza-benzo[f]azulene-9-carbonyl)-phenyl]-1-[4-(2-pyrrolidin-1-yl-ethyl)-piperazin-1-yl]-propan-1-one;
3-[2-Methyl-4-(3-methyl-4,10-dihydro-3H-2,3,4,9-tetraaza-benzo[f]azulene-9-carbonyl)-phenyl]-1-[4-(2-morpholin-4-yl-ethylamino)-piperidin-1-yl]-propan-1-one;
3-[2-Methyl-4-(3-methyl-4,10-dihydro-3H-2,3,4,9-tetraaza-benzo[f]azulene-9-carbonyl)-phenyl]-1-(4-phenethylamino-piperidin-1-yl)-propan-1-one;
3-[2-Methyl-4-(3-methyl-4,10-dihydro-3H-2,3,4,9-tetraaza-benzo[f]azulene-9-carbonyl)-phenyl]-1-{4-[2-(1-methylpyrrolidin-2-yl)-ethylamino]-piperidin-1-yl}-propan-1-one;
3-[2-Methyl-4-(3-methyl-4,10-dihydro-3H-2,3,4,9-tetraaza-benzo[f]azulene-9-carbonyl)-phenyl]-1-[4-(2-pyrrolidin-1-yl-ethylamino)-piperidin-1-yl]-propan-1-one;
3-[2-Methyl-4-(3-methyl-4,10-dihydro-3H-2,3,4,9-tetraaza-benzo[f]azulene-9-carbonyl)-phenyl]-1-[4-(phenethylaminomethyl)-piperidin-1-yl]-propan-1-one; and
3-[2-Methyl-4-(3-methyl-4,10-dihydro-3H-2,3,4,9-tetraaza-benzo[f]azulene-9-carbonyl)-phenyl]-1-(4-thiomorpholin-4-ylmethyl-piperidin-1-yl)-propan-1-one.

53. A compound according to general formula **1d,** or a compound which is a tautomer or a pharmaceutically acceptable salt thereof, 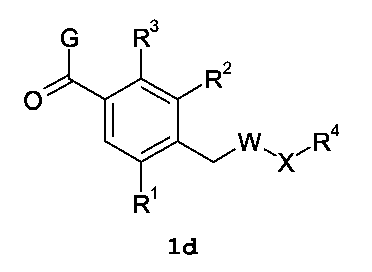
wherein G is a group selected among general formula **2, 3, 4, 5,** and **6**. 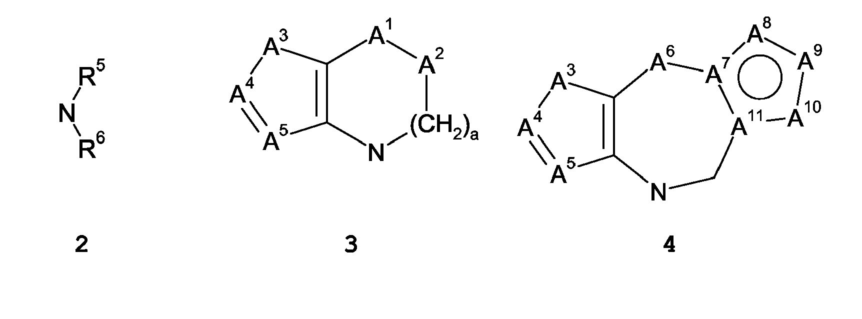 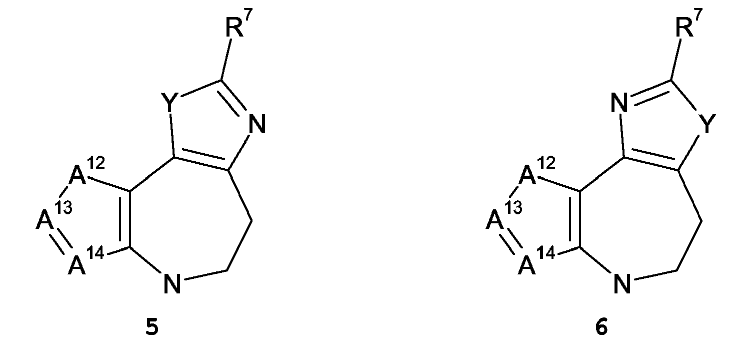
wherein:
A¹ is selected among CH₂, CH(OH), NH, N-alkyl, O and S;
A² is selected among CH₂, CH(OH), C(=O) and NH;
A³ and A¹² are independently selected among S, NH, N-alkyl, -C(R⁸)=CH-, -C(R⁸)=N-, -N=C(R⁸)- and -CH=C(R⁸)- ;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S;
A¹⁰ is selected among -CH=CH-, CH, N, NH, N-(CH₂)_{b}-R¹¹ and S;
wherein the ring constituted by A⁷, A⁸, A⁹, A¹⁰ and A¹¹ is aromatic;
R¹, R² and R³ are independently selected among H, alkyl, O-alkyl, NO₂, F, Cl and Br;
R⁴ is selected among H, alkyl, optionally substituted phenyl, optionally substituted pyridyl, optionally substituted thienyl, optionally substituted furyl, -(CH₂)_{c}-R¹², and 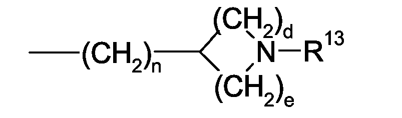
R⁵ and R⁶ are independently selected among alkyl, Ar and -(CH₂)_{f}-Ar, wherein Ar is selected among optionally substituted phenyl and optionally substituted thienyl;
R⁷ is selected among H, alkyl, optionally substituted phenyl, optionally substituted pyridyl, optionally substituted thienyl, optionally substituted furyl, and -(CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the proviso that when R⁴ is H, alkyl, optionally substituted phenyl, optionally substituted pyridyl, optionally substituted thienyl, optionally substituted furyl or -(CH₂)_{c}-R¹² then the ring containing A³, A⁴ and A⁵ according to general formulae **3** and **4** is substituted in at least one position by alkyl, alkoxy, F, Cl, Br, CN, NH₂, NO₂, NH(alkyl) or N(alkyl)₂;
R¹¹ and R¹² are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R¹³ is selected among H, alkyl, optionally substituted phenyl, optionally substituted pyridyl, optionally substituted thienyl, optionally substituted furyl,
-(CH₂)ₕ-R¹⁵ and Z-R¹⁶;
R¹⁴ and R¹⁵ are independently selected among H, alkyl, optionally substituted phenyl, optionally substituted pyridyl, optionally substituted thienyl, optionally substituted furyl, F, OH, O-alkyl, S-alkyl, O-acyl, NH₂, NH-alkyl, N(alkyl)₂, NH-acyl, N(alkyl)-acyl, CO₂H CO₂-alkyl, CONH₂, CONH-alkyl, CON(alkyl)₂, CN and CF₃;
R¹⁶ is selected among H, alkyl, optionally substituted phenyl, optionally substituted pyridyl, optionally substituted thienyl, optionally substituted furyl and
-(CH₂)ᵢ-R¹⁷;
R¹⁷ is selected among H, alkyl, optionally substituted phenyl, optionally substituted pyridyl, optionally substituted thienyl or optionally substituted furyl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
W is selected among O and NH;
X is selected among C(=O) and S(=O)ⱼ
Y is selected among O, S, NH and N-alkyl;
Z is selected among -C(=O) and -S(=O)ₖ;
a is selected among 1 and 2;
b and c are independently selected among 1, 2 and 3;
d, e and f are independently selected among 1 and 2;
g, h and i are independently selected among 1, 2 and 3;
j and k are independently selected among 1 and 2; and
n is selected among 0, 1 and 2.

54. The compound according to claim 53, wherein G is selected among:

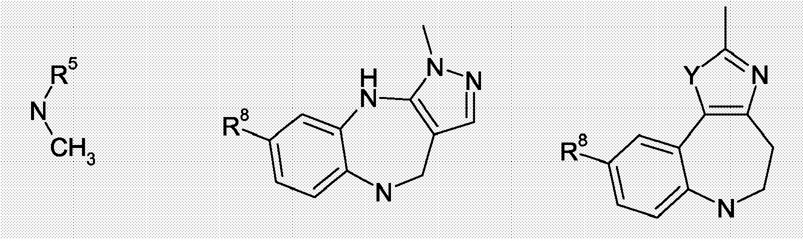

55. The compound according to claim 53, wherein G is selected among:

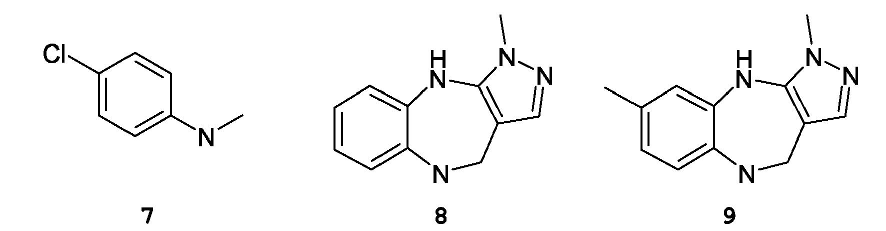

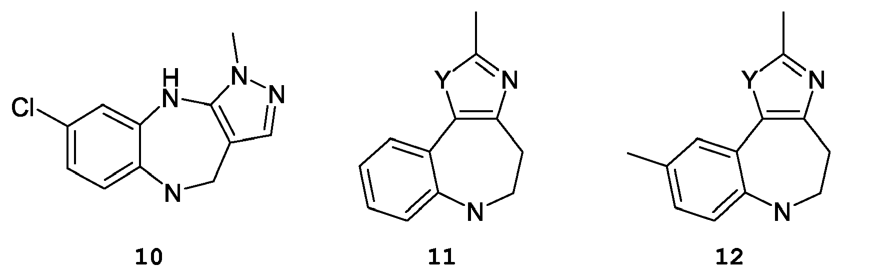

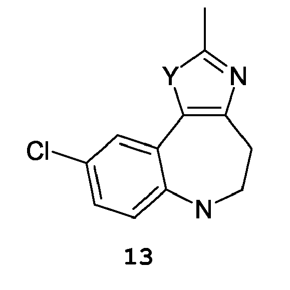

56. The compound according to claim 53, wherein at least one of R¹, R² and R³ is other than hydrogen; preferably one of R¹, R² and R³ is selected among methyl, chlorine and fluorine, and the others are hydrogen.

57. The compound according to claim 53, wherein W is NH.

58. The compound according to claim 53, wherein R⁴ is alkyl.

59. The compound according to claim 53, wherein d is 2 and e is 2.

60. The compound according to claim 53, wherein R¹³ is alkyl.

61. The compound according to claim 53, wherein n is 0.

62. The compound according to claim 53, wherein R² is H, R⁴ is a pipridine where d is 2 and e is 2, W is NH, X is C(=O) and n is 0, as shown in formula **14**: 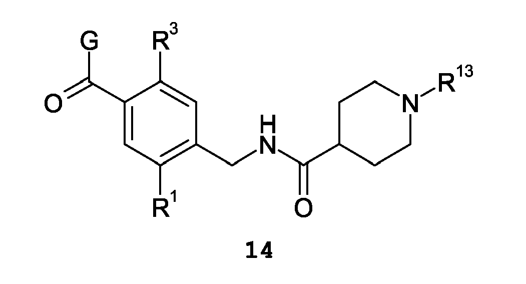
wherein R¹ is selected among methyl, chlorine and fluorine, and R³ is H; or R¹ is H, and R³ is selected among methyl, chlorine and fluorine, and R¹³ is alkyl.

63. The compound according to claim 53, wherein X is C(=O), n is 0 and R⁴ is a pipridine where d is 2 and e is 2, as shown in formula **15:** 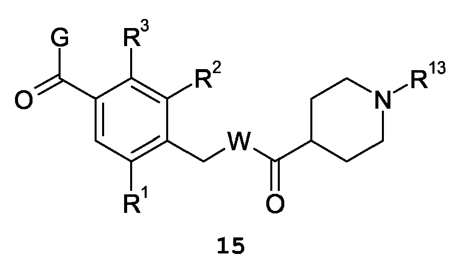
wherein G is selected among general formulae **7** to **13,** and R¹³ is alkyl.

64. The compound according to claim 53, wherein R² is H and W is NH as shown in formula **16:** 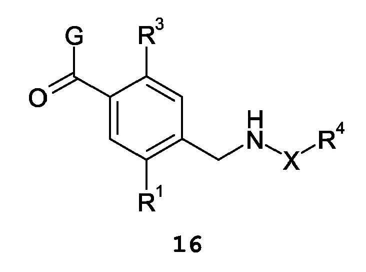
wherein either R¹ is selected among methyl, chlorine and fluorine, and R³ is H; or R¹ is H, and R³ is selected among methyl, chlorine and fluorine; and G is selected from general formulae **7** to **13**.

65. The compound according to claim 53, wherein R² is H, W is NH and X is C(=O) as shown in formula **17**: 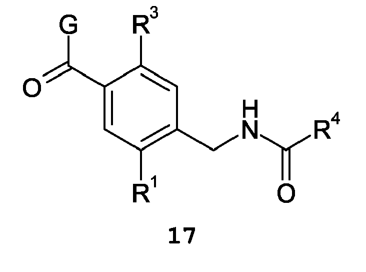
wherein R¹ is selected among methyl, chlorine and fluorine, and R³ is H; or R¹ is H, and R³ is selected among methyl, chlorine and fluorine; and R⁴ is alkyl.

66. The compound according to claim 53, wherein X is C(=O) as shown in formula **18:** 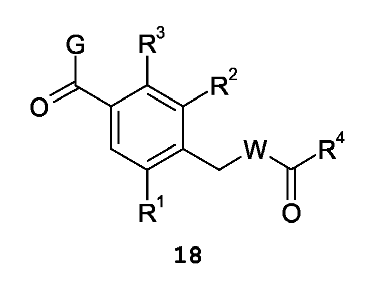
wherein G is selected among general formulae **7** to **13,** and R⁴ is alkyl.

67. The compound according to claim 53, wherein R² and R³ are both H, W is NH and X is C(=O) as shown in formula **19:** 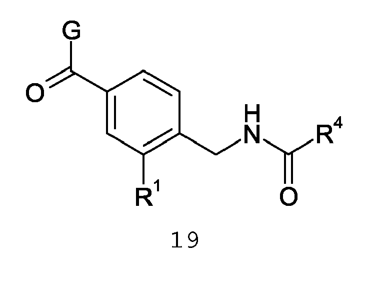
wherein G is selected among general formulae **9** and **10,** R¹ is selected among methyl, chlorine and fluorine, and R⁴ is alkyl.

68. The compound according to claim 53, wherein R² and R³ are both H, R⁴ is a pipridine where d is 2 and e is 2, W is NH, X is C(=O) and n is 0 as shown in formula **20:** 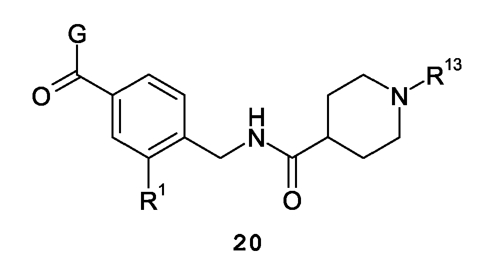
wherein G is selected among general formulae **9** and **10,** R¹ is selected among methyl, chlorine and fluorine, and R¹³ is alkyl.

69. The compound according to claim 53, selected among the group consisting of:
1-(3,3-Dimethyl-butyl)-piperidine-4-carboxylic acid 2-chloro-4-(3,6-dimethyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-chloro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-chloro-4-(6-chloro-3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 2-fluoro-4-(3-methyl-4,10-dihydro-3H-2,3,4,9-tetraaza-benzo[f]azulene-9-carbonyl)-benzylamide
1-(3,3-Dimethyl-butyl)-piperidine-4-carboxylic acid 2-methyl-4-(2-methyl-4,5-dihydro-1H-1,3,6-triaza-benzo[e]azulene-6-carbonyl)-benzylamide;
1-(3,3-Dimethyl-butyl)-piperidine-4-carboxylic acid 2-methyl-4-(2-methyl-4,5-dihydro-1-oxa-3,6-diaza-benzo[e]azulene-6-carbonyl)-benzylamide;
1-(3,3-Dimethyl-butyl)-piperidine-4-carboxylic acid 2-methyl-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4-(3,6-dimethyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-chloro-4-(6-chloro-3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-fluoro-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 3-methyl-4-(3-methyl-4,10-dihydro-3H-2,3,4,9-tetraaza-benzo[f]azulene-9-carbonyl)-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-2-methyl-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-3-methyl-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-2-fluoro-benzylamide;
1-(3,3-Dimethyl-butyl)-piperidine-4-carboxylic acid 4-(3,6-dimethyl-4,10-dihydro-3H-2,3,4,9-tetraaza-benzo[f]azulene-9-carbonyl)-3-fluoro-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-2-methyl-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-2-fluoro-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-3-methyl-benzylamide;
1-(3,3-Dimethyl-butyl)-piperidine-4-carboxylic acid 4-(6-chloro-3-methyl-4,10-dihydro-3H-2,3,4,9-tetraaza-benzo[f]azulene-9-carbonyl)-3-fluoro-benzylamide;
1-(3,3-Dimethyl-butyl)-piperidine-4-carboxylic acid 4-[(4-chloro-phenyl)-methyl-carbamoyl]-2-methyl-benzylamide;
1-Cyclopropylmethyl-piperidine-4-carboxylic acid 2-chloro-4-(3,6-dimethyl-4,10-dihydro-3H-2,3,4,9-tetraaza-benzo[f]azulene-9-carbonyl)-benzylamide;
1-Cyclopropylmethyl-piperidine-4-carboxylic acid 2-chloro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 2-chloro-4-(6-chloro-3-methyl-4,10-dihydro-3H-2,3,4,9-tetraaza-benzo[f]azulene-9-carbonyl)-benzylamide;
1-Cyclopropylmethyl-piperidine-4-carboxylic acid 2-fluoro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 2-methyl-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(3,6-dimethyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-chloro-4-(6-chloro-3-methyl-4,10-dihydro-3H-2,3,4,9-tetraaza-benzo[f]azulene-9-carbonyl)-benzylamide;
1-Cyclopropylmethyl-piperidine-4-carboxylic acid 3-fluoro-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 3-methyl-4-(3-methyl-4,10-dihydro-3H-2,3,4,9-tetraaza-benzo[f]azulene-9-carbonyl)-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-2-methyl-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-3-methyl-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-2-fluoro-benzylamide;
1-Cyclopropylmethyl-piperidine-4-carboxylic acid 4-(3,6-dimethyl-4,10-dihydro-3H-2,3,4,9-tetraaza-benzo[f]azulene-9-carbonyl)-3-fluoro-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-2-fluoro-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-2-methyl-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-3-methyl-benzylamide;
1-Cyclopropylmethyl-piperidine-4-carboxylic acid 4-(6-chloro-3-methyl-4,10-dihydro-3H-2,3,4,9-tetraaza-benzo[f]azulene-9-carbonyl)-3-fluoro-benzylamide;
1-Cyclopropylmethyl-piperidine-4-carboxylic acid 4-[(4-chloro-phenyl)-methyl-carbamoyl]-2-methyl-benzylamide;
Cyclobutanecarboxylic acid 2-chloro-4-(6-chloro-3-methyl-4,10-dihydro-3H-2,3,4,9-tetraaza-benzo[f]azulene-9-carbonyl)-benzylamide;
Cyclobutanecarboxylic acid 4-(3,6-dimethyl-4,10-dihydro-3H-2,3,4,9-tetraaza-benzo[f]azulene-9-carbonyl)-2-fluoro-benzylamide;
Cyclobutanecarboxylic acid 4-(6-chloro-3-methyl-4,10-dihydro-3H-2,3,4,9-tetraaza-benzo[f]azulene-9-carbonyl)-2-fluoro-benzylamide;
Cyclopentanecarboxylic acid 2-chloro-4-(6-chloro-3-methyl-4,10-dihydro-3H-2,3,4,9-tetraaza-benzo[f]azulene-9-carbonyl)-benzylamide;
Cyclopentanecarboxylic acid 4-(3,6-dimethyl-4,10-dihydro-3H-2,3,4,9-tetraaza-benzo[f]azulene-9-carbonyl)-2-fluoro-benzylamide;
Cyclopentanecarboxylic acid 4-(6-chloro-3-methyl-4,10-dihydro-3H-2,3,4,9-tetraaza-benzo[f]azulene-9-carbonyl)-2-fluoro-benzylamide;
Cyclopropanecarboxylic acid 2-chloro-4-(3,6-dimethyl-4,10-dihydro-3H-2,3,4,9-tetraaza-benzo[f]azulene-9-carbonyl)-benzylamide;
Cyclopropanecarboxylic acid 2-chloro-4-(6-chloro-3-methyl-4,10-dihydro-3H-2,3,4,9-tetraaza-benzo[f]azulene-9-carbonyl)-benzylamide;
Cyclopropanecarboxylic acid 3-chloro-4-(3,6-dimethyl-4,10-dihydro-3H-2,3,4,9-tetraaza-benzo[f]azulene-9-carbonyl)-benzylamide;
Cyclopropanecarboxylic acid 3-chloro-4-(6-chloro-3-methyl-4,10-dihydro-3H-2,3,4,9-tetraaza-benzo[f]azulene-9-carbonyl)-benzylamide;
Cyclopropanecarboxylic acid 4-(3,6-dimethyl-4,10-dihydro-3H-2,3,4,9-tetraaza-benzo[f]azulene-9-carbonyl)-2-methyl-benzylamide;
Cyclopropanecarboxylic acid 4-(3,6-dimethyl-4,10-dihydro-3H-2,3,4,9-tetraaza-benzo[f]azulene-9-carbonyl)-2-fluoro-benzylamide;
Cyclopropanecarboxylic acid 4- (3, 6-dimethyl-4, 10-dihydro-3H-2,3,4,9-tetraaza-benzo[f]azulene-9-carbonyl)-3-fluoro-benzylamide;
Cyclopropanecarboxylic acid 4-(3,6-dimethyl-4,10-dihydro-3H-2,3,4,9-tetraaza-benzo[f]azulene-9-carbonyl)-3-methyl-benzylamide;
Cyclopropanecarboxylic acid 4-(6-chloro-3-methyl-4,10-dihydro-3H-2,3,4,9-tetraaza-benzo[f]azulene-9-carbonyl)-2-methyl-benzylamide;
Cyclopropanecarboxylic acid 4-(6-chloro-3-methyl-4,10-dihydro-3H-2,3,4,9-tetraaza-benzo[f]azulene-9-carbonyl)-3-fluoro-benzylamide;
Cyclopropanecarboxylic acid 4-(6-chloro-3-methyl-4,10-dihydro-3H-2,3,4,9-tetraazabenzo[f]azulene-9-carbonyl)-2-fluoro-benzylamide;
Cyclopropanecarboxylic acid 4-(6-chloro-3-methyl-4,10-dihydro-3H-2,3,4,9-tetraaza-benzo[f]azulene-9-carbonyl)-3-methyl-benzylamide;
N-[2-Chloro-4-(6-chloro-3-methyl-4,10-dihydro-3H-2,3,4,9-tetraaza-benzo[f]azulene-9-carbonyl)-benzyl]-propionamide N-[2-Chloro-4-(6-chloro-3-methyl-4,10-dihydro-3H-2,3,4,9-tetraaza-benzo[f]azulene-9-carbonyl)-benzyl]-2,2-dimethyl-propionamide;
N-[2-Chloro-4-(6-chloro-3-methyl-4,10-dihydro-3H-2,3,4,9-tetraaza-benzo[f]azulene-9-carbonyl)-benzyl]-isobutyramide;
N-[2-Chloro-4-(6-chloro-3-methyl-4,10-dihydro-3H-2,3,4,9-tetraaza-benzo[f]azulene-9-carbonyl)-benzyl]-butyramide;
N-[4-(3,6-Dimethyl-4,10-dihydro-3H-2,3,4,9-tetraaza-benzo[f]azulene-9-carbonyl)-2-fluoro-benzyl]-propionamide;
N-[4-(3,6-Dimethyl-4,10-dihydro-3H-2,3,4,9-tetraaza-benzo[f]azulene-9-carbonyl)-2-fluoro-benzyl]-2,2-dimethyl-propionamide;
N-[4-(3,6-Dimethyl-4,10-dihydro-3H-2,3,4,9-tetraaza-benzo[f]azulene-9-carbonyl)-2-fluoro-benzyl]-isobutyramide;
N-[4-(3,6-Dimethyl-4,10-dihydro-3H-2,3,4,9-tetraaza-benzo[f]azulene-9-carbonyl)-2-fluoro-benzyl]-butyramide;
N-[4-(6-Chloro-3-methyl-4,10-dihydro-3H-2,3,4,9-tetraaza-benzo[f]azulene-9-carbonyl)-2-fluoro-benzyl]-propionamide;
N-[4-(6-Chloro-3-methyl-4,10-dihydro-3H-2,3,4,9-tetraaza-benzo[f]azulene-9-carbonyl)-2-fluoro-benzyl]-2,2-dimethyl-propionamide;
N-[4-(6-Chloro-3-methyl-4,10-dihydro-3H-2,3,4,9-tetraaza-benzo[f]azulene-9-carbonyl)-2-fluoro-benzyl]-isobutyramide;
N-[4-(6-Chloro-3-methyl-4,10-dihydro-3H-2,3,4,9-tetraaza-benzo[f]azulene-9-carbonyl)-2-fluoro-benzyl]-acetamide;
N-[4-(6-Chloro-3-methyl-4,10-dihydro-3H-2,3,4,9-tetraaza-benzo[f]azulene-9-carbonyl)-2-fluoro-benzyl]-butyramide; and
N-[4-(6-Chloro-3-methyl-4,10-dihydro-3H-2,3,4,9-tetraaza-benzo[f]azulene-9-carbonyl)-2-fluoro-benzyl]-formamide.

70. A pharmaceutical composition comprising a compound according to any of the preceding claims.

71. The pharmaceutical composition according to claim 70, formulated for oral administration, preferably as a tablet, a capsule or a sachet.

72. The pharmaceutical composition according to claim 70 or 71, for the treatment of a condition selected among primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

73. Use of a compound according to any of the claims 1 to 69 for the manufacture of a medicament for the treatment of a condition selected among primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

74. A method of treatment of a disorder selected among primary dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure, comprising the administration to a person in need of such treatment of an effective amount of a compound according to any of the claims 1 to 69.
